# EUROPEAN PATENT SPECIFICATION

(11) **EP 3 283 529 B1**
(45) Date of publication and mention of the grant of the patent: **07.06.2023**
(21) Application number: 16780837.7
(22) Date of filing: 15.04.2016
(51) Int. Cl.: C07K 16/28, C07K 14/525, C07K 14/475, A61K 38/18, A61K 39/395

(54) **AGENTS, SYSTEMS AND METHODS FOR TREATING CANCER**
WIRKSTOFFE, SYSTEME UND VERFAHREN ZUR BEHANDLUNG VON KREBS
AGENTS, SYSTÈMES ET MÉTHODES DE TRAITEMENT DU CANCER

(30) Priority: 17.04.2015 US 201562148795 P
(43) Date of publication of application: 21.02.2018
(73) Proprietor: The General Hospital Corporation, Boston, MA 02114 (US)
(72) Inventor: SHAH, Khalid, Andover, MA 01810 (US)
(74) Representative: Barker Brettell LLP
(86) International application number: PCT/US2016/027760
(87) International publication number: WO 2016/168601

(56) References cited:
- WO-A1-2014/035474
- WO-A2-2012/106281
- JORDI MARTINEZ-QUINTANILLA ET AL: "Therapeutic Efficacy and Fate of Bimodal Engineered Stem Cells in Malignant Brain Tumors : Tumor Efficacy and Fate of Therapeutic Stem Cells", STEM CELLS., vol. 31, no. 8, 1 August 2013 (2013-08-01) , pages 1706-1714, XP055459331, US ISSN: 1066-5099, DOI: 10.1002/stem.1355
- SASPORTAS LAURA S ET AL: "Assessment of therapeutic efficacy and fate of engineered human mesenchymal stem cells for cancer therapy", PROCEEDINGS OF THE NATIONAL ACADEMY OF SCIENCES OF THE UNITED STATES OF AMERICA, vol. 106, no. 12, March 2009 (2009-03), pages 4822-4827, XP002784880, ISSN: 0027-8424, DOI: 10.1073/PNAS.0806647106
- CARNEY B J ET AL: "Migration and fate of therapeutic stem cells in different brain disease models", NEUROSCIENCE, NEW YORK, NY, US, vol. 197, 28 August 2011 (2011-08-28), pages 37-47, XP028101736, ISSN: 0306-4522, DOI: 10.1016/J.NEUROSCIENCE.2011.08.063 [retrieved on 2011-09-14]
- BEXELL DANIEL ET AL: "Stem cell-based therapy for malignant glioma", CANCER TREATMENT REVIEWS, ELSEVIER, AMSTERDAM, NL, vol. 39, no. 4, 13 July 2012 (2012-07-13), pages 358-365, XP029004169, ISSN: 0305-7372, DOI: 10.1016/J.CTRV.2012.06.006
- SHAH, K.: 'Mesenchymal Stem Cells engineered for Cancer Therapy.' ADV DRUG DELIV REV. vol. 64, no. 8, 01 June 2012, pages 739 - 748, XP028916800
- BAGCI-ONDER, T ET AL.: 'Targeting breast to brain metastatic tumours with death receptor ligand expressing therapeutic stem cells.' BRAIN. vol. 138, no. 6, 24 April 2015, pages 1710 - 1721, XP055323097

## Description

### CROSS-REFERENCE TO RELATED APPLICATIONS

This application claims benefit under 35 U.S.C. §119(e) of U.S. Provisional Application Serial No.: 62/148,795 filed April 17, 2015.

### GOVERNMENT SUPPORT

This invention was made with government support under RO 1 CA138922 and RO 1 NS071197 awarded by the National Institutes of Health (NIH). The government has certain rights in the invention.

### TECHNICAL FIELD

The field of the invention relates to therapies for the treatment of metastatic brain tumors.

### BACKGROUND

Metastatic brain tumors are the most commonly observed intracranial tumors, frequently occurring in patients with metastatic cancers of the lung, breast, and skin (melanoma) (Eichler et al., 2011). The incidence of brain metastasis has been estimated to be 35% of metastatic breast cancer patients (Lockman et al., 2010), which is often manifested with neurological symptoms and diagnosed with clinical imaging modalities (Steeg et al., 2011). Most patients have multiple metastatic lesions at the time of diagnosis, making surgery an inadequate therapeutic option on its own. Parallel therapeutic approaches include stereotactic radiosurgery, whole brain radiotherapy and chemotherapy (Kocher et al., 2011). However, the blood-brain barrier, which prevents the brain permeability of many systemic therapies, and the negative side effects of radiotherapy pose challenges for the success of existing therapies and cause failure to improve overall patient survival (Lockman et al., 2010). Jordi Martinez-Quintanilla et al, in STEM CELLS., US, (20130801), vol. 31, no. 8, doi:10.1002/stem. 1355, ISSN 1066-5099, pages 1706 - 1714, describes the therapeutic efficacy and fate of bimodal engineered stem cells in malignant brain tumors. Asportas Laura S et al, in PNAS vol. 106, no. 12, doi:10.1073/PNAS.0806647106, ISSN 0027-8424, pages 4822 - 4827, describe an assessment of the therapeutic efficacy and fate of engineered human mesenchymal stem cells for cancer therapy.

### SUMMARY

The compositions and methods comprising soluble tumor necrosis factor-related apoptosis inducing ligand (sTRAIL) polypeptides described herein provide novel therapeutic approaches for treatment of metastatic brain cancers. While primary brain tumors mostly arise at a single site and progress from there, metastases occur in several distinct spots giving rise to multiple metastatic foci of different sizes (Modha et al., 2005). Accordingly, as described herein tumor-seeking stem-cell-based therapies were evaluated for treating multiple metastatic tumor lesions in the brain. The nature of the metastatic breast cancer model was first assessed in detail. Using non-invasive bioluminescent and fluorescent imaging, the temporal and spatial distribution of the metastases in the brain were then assessed. The metastatropic properties of engineered NSCs and the therapeutic potential of TRAIL-secreting engineered neural stem cells (NSC-S-TRAIL) in this model were further explored. The studies described herein establish that NSCs engineered to express metastatic tumor-specific biomolecules can target brain metastases and deliver effective treatment.

Accordingly, provided herein, in some aspects, are methods of treating multifocal metastases in the brain comprising administering to an individual in need thereof stem cells engineered to express a binding agent specific for a tumor-associated cell-surface protein, whereby the growth and/or survival of multifocal metastases in the brain is/are inhibited. The invention is defined by the claims.

One aspect of the invention comprises stem cells engineered to express a binding agent specific for a tumor-associated cell-surface protein for use in the treatment of multifocal metastases in the brain, wherein the stem cells comprise mesenchymal stem cells (MSCs) or neuronal stem cells (NSCs) and the binding agent comprises a soluble TRAIL (sTRAIL) polypeptide , and wherein the treatment comprises administering the engineered stem cells to an individual in need thereof, via intracarotid artery injection, whereby the growth and/or survival of multifocal metastases in the brain is/are inhibited.

According to the present invention, the tumor-associated cell-surface protein may be death receptor 4 (DR4) or death receptor 5 (DR5).

According to the present invention, the sTRAIL polypeptide may comprise a fusion with a second tumor-associated cell-surface protein binding agent.

According to the present invention, the second tumor-associated cell-surface protein may be a growth factor receptor.

According to the present invention, the growth factor receptor may be selected from EGFR, IGF-R and NGF-R.

According to the present invention, the second tumor-associated cell surface marker binding agent may be an antibody or antigen-binding fragment thereof, or a nanobody.

According to the present invention, the neuronal stem cells may be derived from induced pluripotent stem (iPS) cells.

According to the present invention, the stem cells may be further engineered to express a HSV thymidine kinase.

According to the present invention, the treatment may further comprise, after treatment for multifocal brain metastases has been effected, administering gancyclovir, thereby eradicating the stem cells.

### Definitions

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims. Definitions of common terms in immunology, and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737).

As used herein, the terms "soluble TRAIL polypeptide agent" or "sTRAIL polypeptide" or "soluble TRAIL polypeptide" refer to a molecule comprising an extracellular domain of human TRAIL of SEQ ID NO: 1, or a variant or derivative thereof that maintains TRAIL functional activity but is not inserted or anchored in the cell membrane. Such sTRAIL polypeptides include fusion polypeptides comprising, in addition to the extracellular TRAIL domain, one or more heterologous sequences, including, for example, spacer region sequences, secretion signal sequences, linker sequences, and/or sequences encoding another therapeutic, biologically active protein or fragment thereof, including tumor-associated cell-surface proteins or fragments thereof

As used herein, the terms "metastatic brain cancer" or "secondary brain cancer" or "metastatic brain tumor" or "secondary brain tumor" or "multifocal brain metastases" refer to a tumor occurring in the brain that is caused by cancer cells spreading to the brain from a primary cancer elsewhere in the body, forming a tumor or tumor foci in the brain. Metastatic brain tumors are often multifocal in nature; such tumors are referred to herein as "multifocal brain metastases."

As used herein, the term "antibody agent" refers to a polypeptide comprising a monoclonal antibody or antigen-binding domain of a monoclonal antibody that includes at least one immunoglobulin variable domain or immunoglobulin variable domain sequence and which specifically binds a given antigen. The term "antibody agent" encompasses antigen-binding fragments of antibodies *(e.g*., single chain antibodies, Fab and sFab fragments, F(ab')₂, Fd fragments, Fv fragments, scFv, single domain antibodies (dAb) (de Wildt et al., Eur J. Immunol. 1996; 26(3):629-39)), nanobodies, as well as complete antibodies.

The terms "antigen-binding fragment" and "antigen-binding domain" are used herein to refer to one or more fragments of a full length antibody that retain the ability to specifically bind to a target of interest. Examples of binding fragments encompassed within the term "antigen-binding fragment" of a full length antibody include (i) a Fab fragment, a monovalent fragment consisting of the VL, VH, CL and CH1 domains; (ii) a F(ab')₂ fragment, a bivalent fragment including two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment (Ward et al., (1989) Nature 341:544-546), which consists of a VH or VL domain; and (vi) an isolated complementarity determining region (CDR) that retains specific antigen-binding functionality. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See *e.g.,* U.S. Pat. Nos. 5,260,203, 4,946,778, and 4,881,175; Bird et al. (1988) Science 242:423-426; and Huston et al. (1988) Proc. Natl. Acad. Sci. USA 85:5879-5883.

As used herein, the term "nanobody" refers to an antibody comprising the small single variable domain (VHH) of antibodies obtained from camelids and dromedaries. Antibody proteins obtained from members of the camel and dromedary (Camelus baclrianus and Calelus dromaderius) family including new world members such as llama species (Lama paccos, Lama glama and Lama vicugna) have been characterized with respect to size, structural complexity and antigenicity for human subjects. Certain IgG antibodies from this family of mammals as found in nature lack light chains, and are thus structurally distinct from the typical four chain quaternary structure having two heavy and two light chains, for antibodies from other animals. See PCT/EP93/ 02214 (WO 94/04678 published 3 Mar. 1994).

As used herein, a "prodrug" is a masked form of an active drug that has been designed to be activated after an enzymatic or chemical reaction once it has been administered into the body.

As used herein, a "prodrug modifying gene" or "prodrug modifying element" or gene encoding a "prodrug converting enzyme," or variations thereof refer to a gene that encodes a polypeptide that converts a prodrug to an active, e.g., cytotoxic compound.

As used herein, "neural stem cells" or "neuronal stem cells" or "NSCs" refer to a subset of multipotent cells which have partially differentiated along a neural cell pathway and express some neural markers including, for example, nestin. Neuronal stem cells, the markers they express, and their differentiation from human ESCs and iPS cells are described by Yuan et al. (PLOS One, 6(3): el7540 (2011). In some embodiments of the methods described herein, NSCs are marked by the cell-surface expression profile of CD184+/ CD271-/CD44-/CD24+. Neural stem cells can differentiate into neurons or glial cells (e.g., astrocytes and oligodendrocytes). Thus, "neural stem cells derived or differentiated from iPS cells" refers to cells that are multipotent but have partially differentiated along a neural cell pathway (i.e., express some neural cell markers), and themselves are the result of *in vitro* or *in vivo* differentiation iPS cells.

The phrases "parenteral administration" and "administered parenterally" as used herein, refer to modes of administration other than enteral and topical administration, usually by injection. The phrases "systemic administration," "administered systemically", "peripheral administration" and "administered peripherally" as used herein refer to the administration of soluble TRAIL polypeptides or cells engineered to express soluble TRAIL polypeptides other than directly into a target site, tissue, or organ, such as a metastatic tumor site, such that it enters the subject's circulatory system. As used herein, "injection" includes, without limitation, intravenous, intraarterial, intrathecal, intraventricular, subarachnoid, intraspinal, and intracerebrospinal injection and infusion.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder associated with a malignant condition or cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation or at least slowing of progress or worsening of symptoms that would be expected in absence of treatment. Beneficial or desired clinical results include, but are not limited to, alleviation of one or more symptom(s) of a malignant disease, diminishment of extent of a malignant disease, stabilized (i.e., not worsening) state of a malignant disease, delay or slowing of progression of a malignant disease, amelioration or palliation of the malignant disease state, and remission (whether partial or total), whether detectable or undetectable. The term "treatment" of a disease also includes providing relief from the symptoms or side-effects of the disease (including palliative treatment).

The terms "decrease", "reduced", "reduction" , "decrease" or "inhibit" are all used herein generally to mean a decrease by a statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level or non-detectable level as compared to a reference sample), or any decrease between 10-100% as compared to a reference level.

The terms "increased" ,"increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "statistically significant" or "significantly" refers to statistical significance and generally means a two standard deviation (2SD) below normal, or lower, concentration of the marker. The term refers to statistical evidence that there is a difference. It is defined as the probability of making a decision to reject the null hypothesis when the null hypothesis is actually true. The decision is often made using the p-value.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of refers to those elements required for a given embodiment. The term permits the presence of elements that do not materially affect the basic and novel or functional characteristic(s) of that embodiment of the invention.

The term "consisting of refers to compositions, methods, and respective components thereof as described herein, which are exclusive of any element not recited in that description of the embodiment.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth.

### BRIEF DESCRIPTION OF THE FIGURES

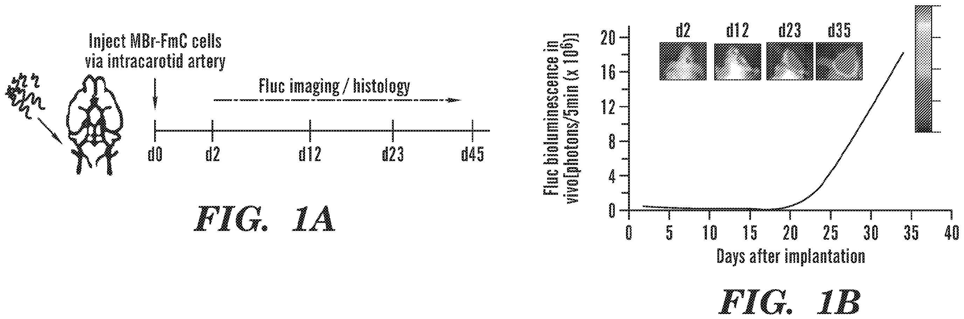

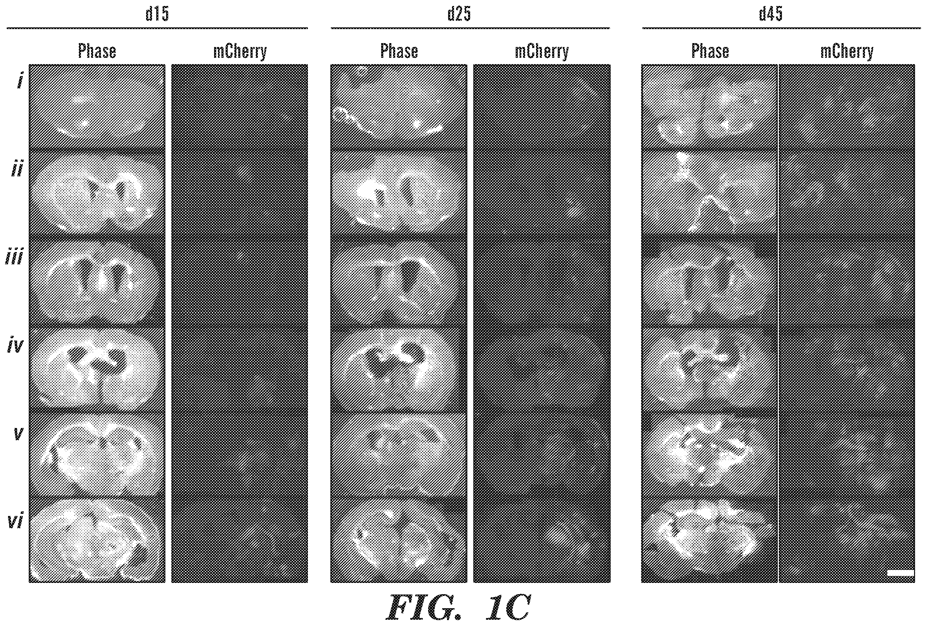

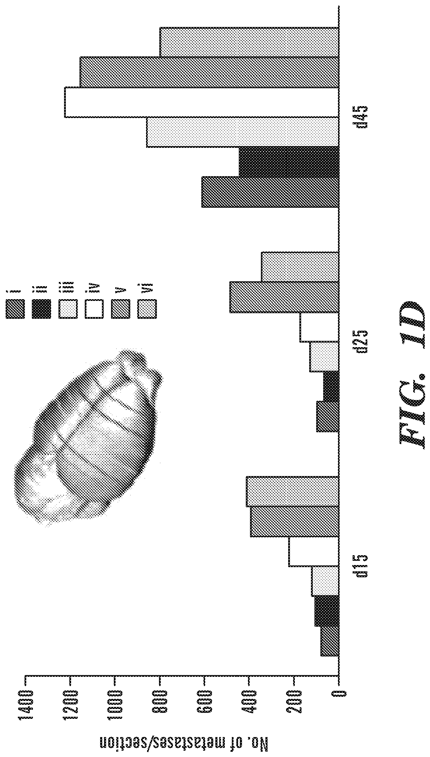

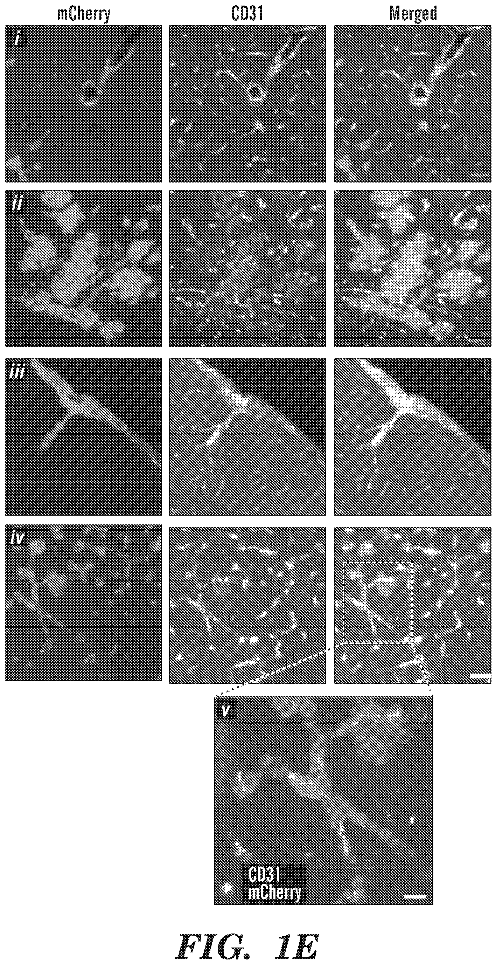

**FIGS. 1A-1E** demonstrate that imaging of brain metastases established with intracarotid artery injection reveals the tumor progression and blood vessel association in vivo. **(****FIG. 1A****)** Outline of the experiment. **(****FIG. 1B****)** Representative bioluminescent images of MBr-FmC tumors formed by intracarotid artery injection and plot showing the tumor growth of MBr-FmC over time. **(****FIG. 1C****)** Composite phase and fluorescent images (x 2) of coronal brain sections generated from intracarotid injected tumors from different planes along the anterior-posterior axis (Scale bar = 2 mm, depicted with schematic in 1D, inset). **(****FIG. 1D****)** Quantification of metastatic tumor growth along different brain regions over time. **(****FIG. 1E****)** Representative fluorescent images from CD31 immunostaining on brain sections showing distinct hallmarks of brain metastasis. (i) vascular mimicry; (ii) neovascularization; (iii) subpial growth; (iv) vascular co-option (Scale bar = 100 mm); (v) close association of CD31 positive blood vessels and mCherry labelled tumor cells (Scale bar = 50 mm).

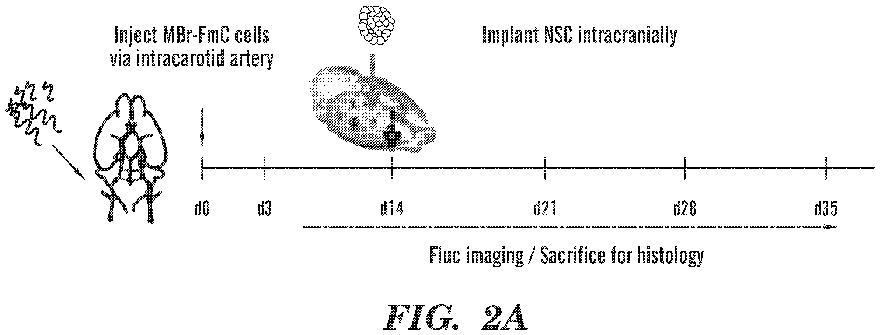

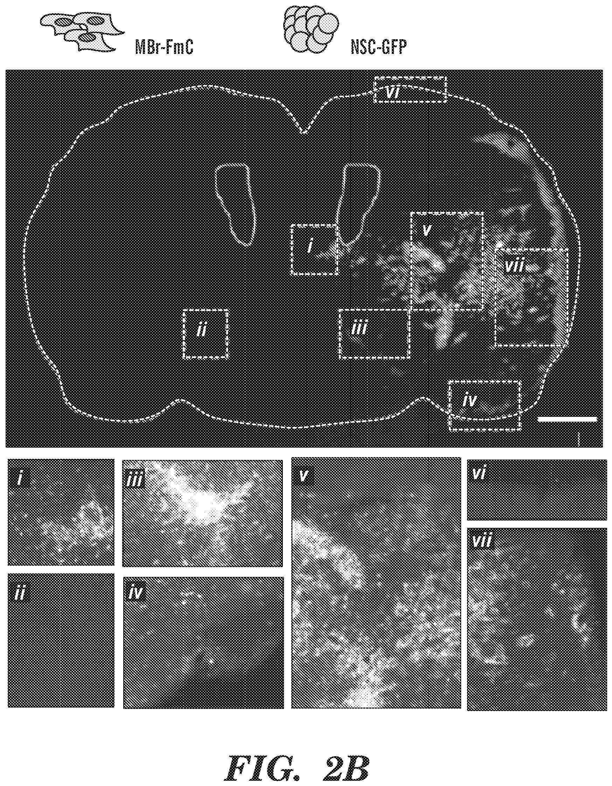

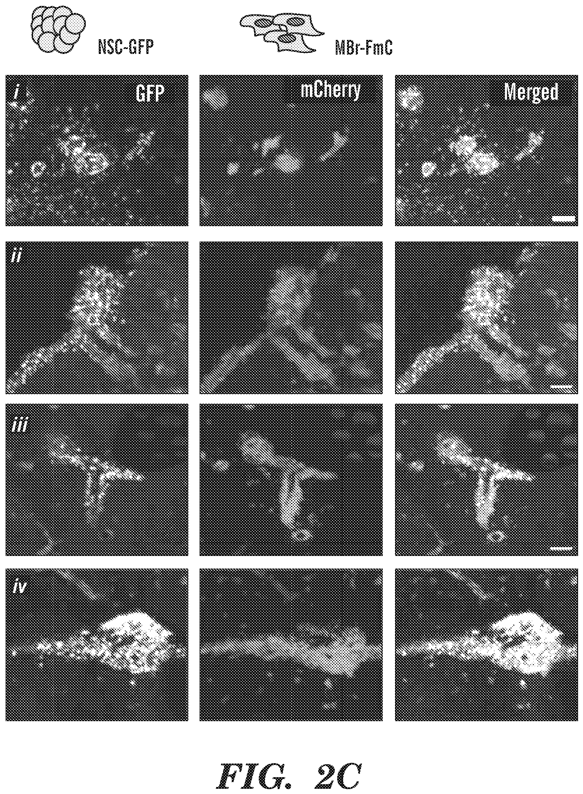

**FIGS. 2A-2C** demonstrate the interaction of neuronal stem cell (NSC) and metastatic tumor foci. **(****FIG. 2A****)** Outline of the experiment to study the association of engrafted NSCs with brain metastases after intracranial implantation of NSCs. **(****FIG. 2B****)** Representative composite fluorescent images of brain sections obtained at 2 weeks after NSC injection to an established brain metastasis model (Scale bar = 1 mm). mCherry depicting tumor cells. Insets i-vii show the GFP labelled NSCs. **(FIG. 2C)** Representative fluorescent images showing NSC homing to established metastases. NSC-GFP; MBr-FmC tumor cells. (i) Small metastatic deposits surrounded by NSC; (ii) leptomeningeal metastasis (subpial) infiltrated by NSCs; (iii) perivascular metastasis infiltrated by NSCs; (iv) cerebellar metastasis accessed by NSCs (Scale bar = 50 mm).

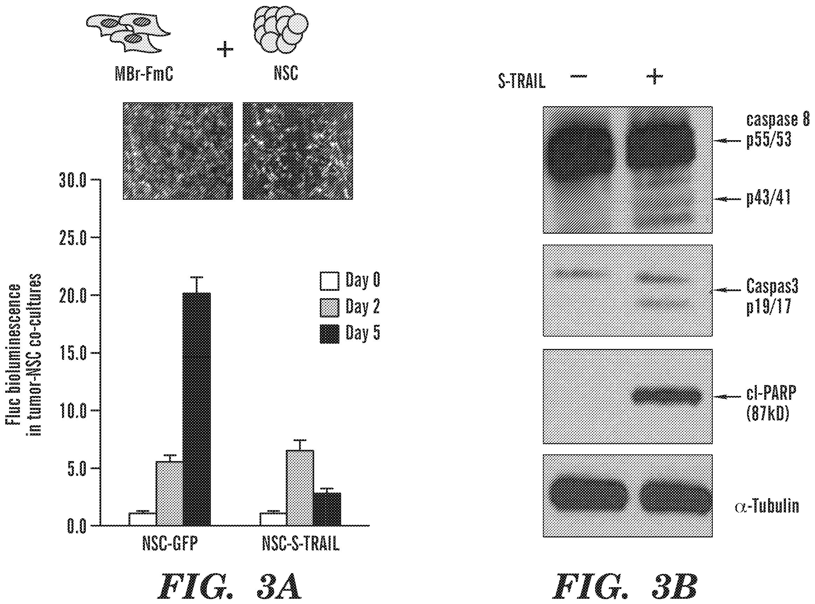

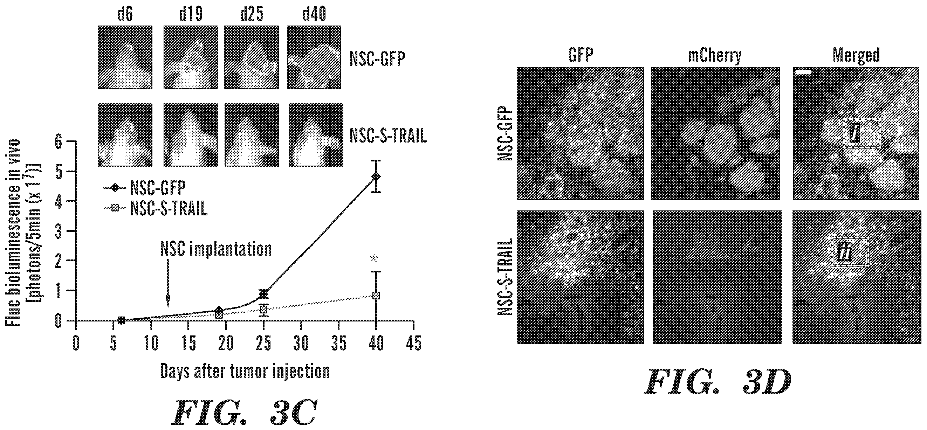

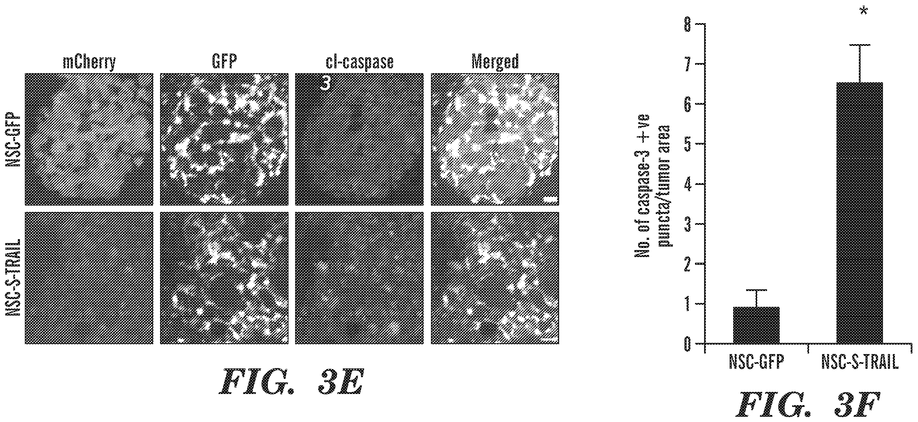

**FIGS. 3A-3F** demonstrate that NSCs secreting TRAIL home to metastatic foci and eradicate brain metastases in vivo. **(****FIG. 3A****)** Viability of MBr-FmC cells in tumor-NSC co-cultures as measured by Flue activity in vitro. The effects of NSCs engineered with GFP or secreting TRAIL are shown. **(****FIG. 3B****)** Western blot analysis of caspase 8, caspase 3, cleaved PARP1 and tubulin in MBr-FmC lysates collected upon treatment with conditioned medium from NSC-GFP (-, without secreted TRAIL in the medium) or NSC-S-TRAIL ( + , with secreted TRAIL in the medium). **(****FIG. 3C****)** Top: Representative bioluminescent images of metastatic MBr-FmC tumors established with intracarotid injection and administered with NSC-GFP or NSC-S-TRAIL on Days 6, 19, 25 and 40 after tumor cell injection. Bottom: Plot showing Flue activity of MBr-FmC tumors treated with NSC-GFP (n = 3 mice) or NSC-S-TRAIL (n = 5 mice). Arrow points to the time of intracranial NSC injection (14 days after tumor cells injection). **(****FIG. 3D****)** Representative fluorescent images of coronal brain sections obtained from tumor bearing mice treated with NSC-GFP or NSC-S-TRAIL. MBr-FmC cells; NSC-GFP or NSC-S-TRAIL (Scale bar = 100 mm). **(****FIG. 3E****)** Representative confocal images of cleaved caspase 3 immunohistochemistry of metastatic tumors surrounded by NSC-GFP (top) or NSC-S-TRAIL (bottom) obtained at Day 40. MBr-FmC cells; NSC-GFP or NSC-S-TRAIL; cleaved caspase 3 (Scale bar = 25 mm). **(****FIG. 3F****)** The number of activated caspase 3 positive cells in E was calculated. (n = 5 regions/group counted for puncta and area).

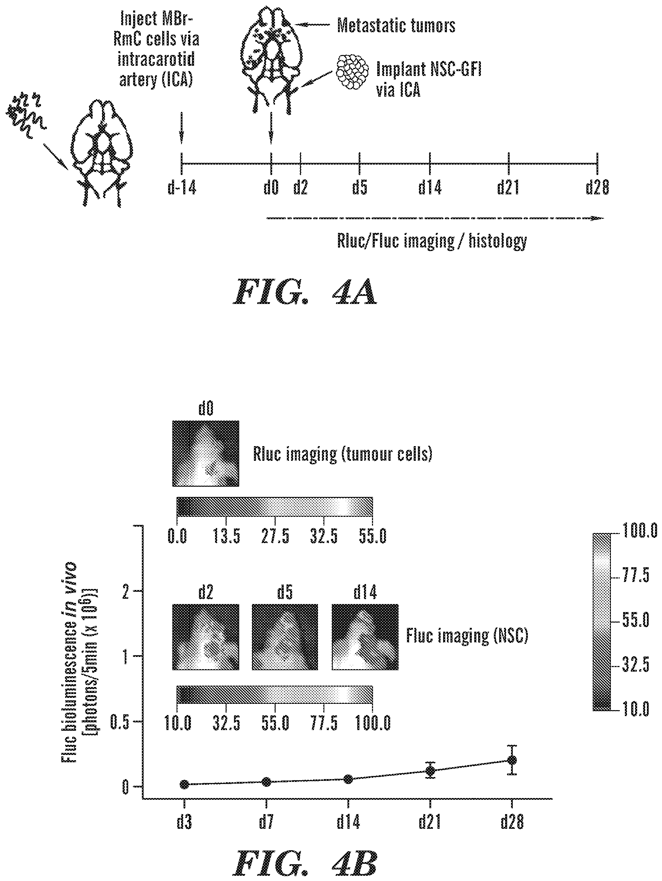

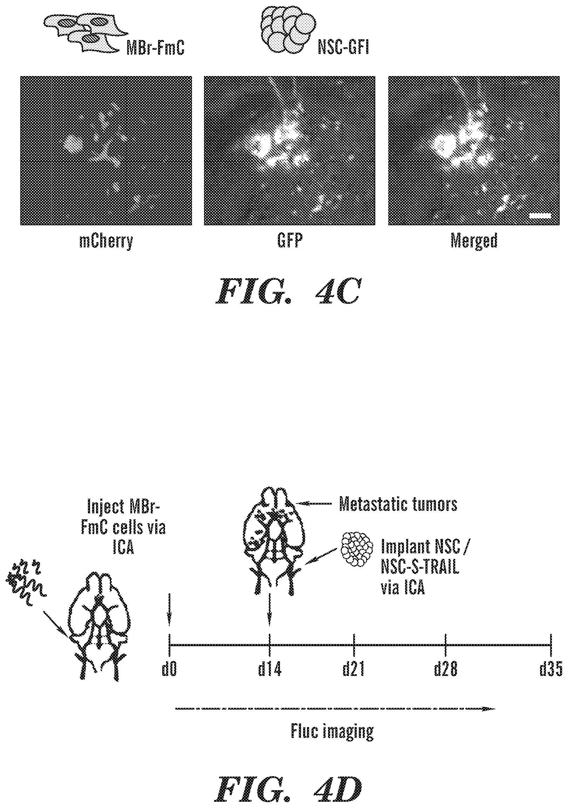

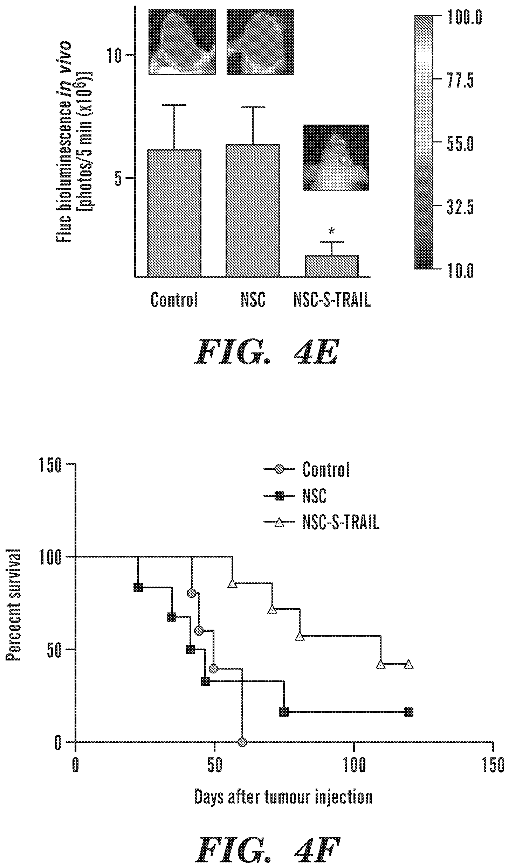

**FIGS. 4A-4F** demonstrate that intravascular administration of NSC-S-TRAIL in metastatic tumor bearing mice have anti-tumor effects. **(****FIG. 4A****)** Outline of the experiments to investigate the survival and distribution of NSC in the brain of metastatic tumor bearing mice. **(FIG.**
4B) Representative dual bioluminescent images of both MBr-RmC and NSC-GF1 cells administered via intracarotid injection. Rluc imaging depicting tumor cells [Day 0 (d0) representing 14 days after tumor cell injection] and Flue imaging depicting NSC (2, 5 and 14 days after NSC injection). Plot showing the Flue bioluminescence of NSC-GF1 cells over time (n = 3 mice). (FIG. 4C) Representative fluorescent images showing NSC surrounded or located within metastatic tumor foci 7 days after intracarotid administration (Scale bar = 50 mm). (FIG. 4D) Outline of experiments to study the efficacy of NSC-S-TRAIL administered intracarotidly in metastatic tumor-bearing mice. (FIG. 4E) Plot showing the Flue bioluminescence of MBr-FmC tumors without treatment (control, n = 5 mice) or treated with NSC (n = 11 mice) or NSC-S-TRAIL (n = 12 mice). Inset: representative bioluminescent images of metastatic MBr-FmC tumors established with intracarotid injection and treated with NSC or NSC-S-TRAIL on Day 35. (FIG. 4F) Kaplan-Meier survival curves of metastatic breast tumor bearing mice without treatment as control (n = 7 mice) or treated with NSC (n = 6 mice) or NSC-S-TRAIL (n = 7 mice, P = 0.021 in NSC and NSC-S-TRAIL comparison, log-rank test).

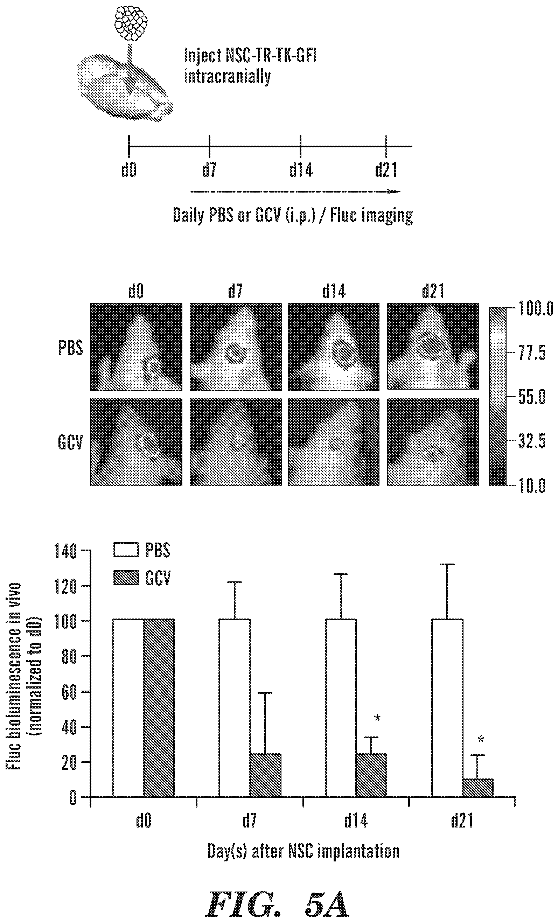

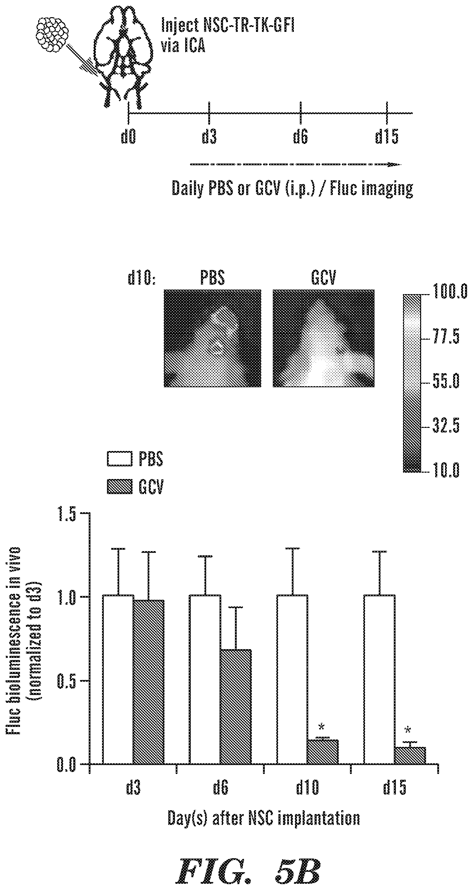

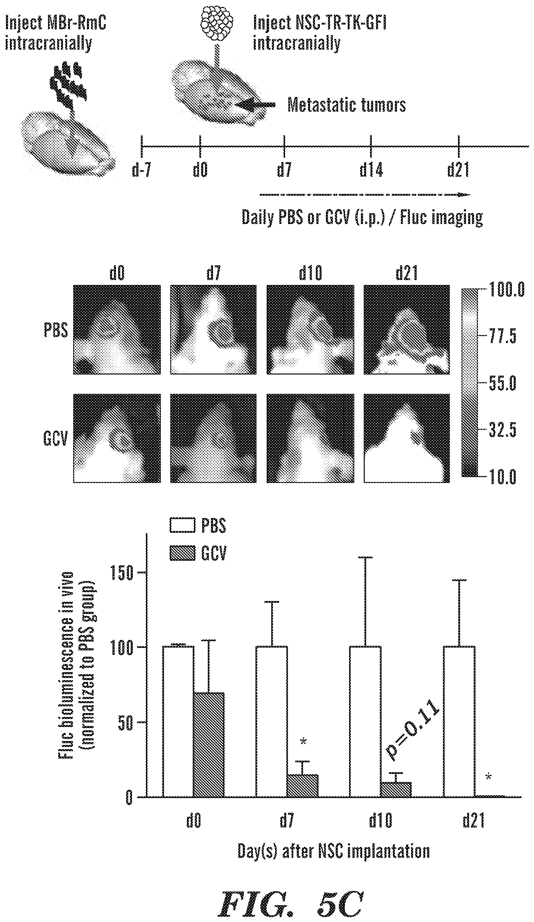

FIGS. 5A-5C demonstrate that therapeutic NSC can be eradicated post-tumor treatment in vivo. (FIG. 5A) Top: Outline of the experiment to test the eradication of intracranially injected NSC by ganciclovir (GCV) administration. Representative bioluminescent images of mice intracranially administered with NSC-TR-TK-GF1 cells with or without ganciclovir treatment. Plot showing the Flue activity of NSC-TR-TK-GF1 cells in vivo (n = 3 mice per group). (FIG. 5B) Top: Outline of the experiment to test the eradication of intracarotidly injected NSCs by ganciclovir administration. Representative bioluminescent images of mice intracarotidly administered with NSC-TR-TK-GF1 cells and treated with PBS or ganciclovir. Plot showing the Flue activity of NSC-TR-TK-GF1 cells in indicated groups in vivo (n = 5 mice per group). (FIG. 5C, top) Outline of the experiment to test the eradication of NSC post-tumor treatment. Representative bioluminescent images of mice intracranially administered with NSC-TR-TK-GF1 with or without ganciclovir treatment. Plot showing the Flue activity of NSC-TR-TK-GF1 cells in vivo (n = 4 mice per group).

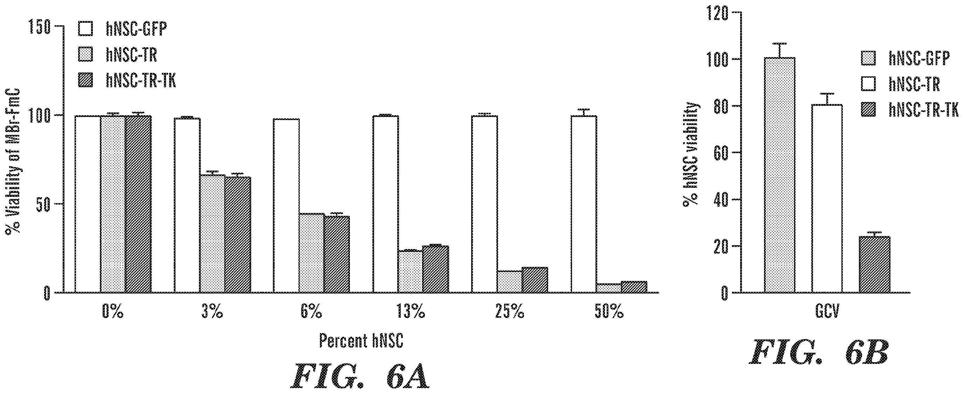

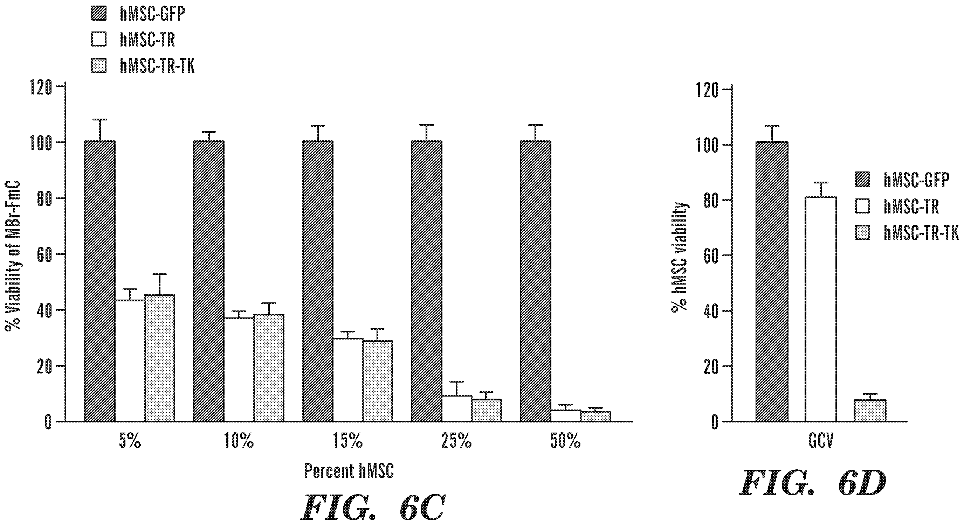

FIGS. 6A-6D demonstrate that human NSC and human MSC secreting TRAIL/HSV-TK kill metastatic tumor cells in vitro. (FIG. 6A) Plot showing the viability of MBr-FmC cells co-cultured with engineered human NSCs expressing TRAIL (TR), both TRAIL and HSV-TK (TR-TK) or GFP as measured by Flue activity in vitro. (FIG. 6B) Plot showing the viability of modified human NSCs treated with ganciclovir (10 mg/ml). (FIG. 6C) Plot showing the viability of MBr-FmC cells co-cultured with engineered human MSC expressing TRAIL (TR), both TRAIL and HSV-TK (TR-TK) or GFP. (FIG. 6D) Plot showing the viability of modified human MSCs treated with ganciclovir (10 mg/ml).

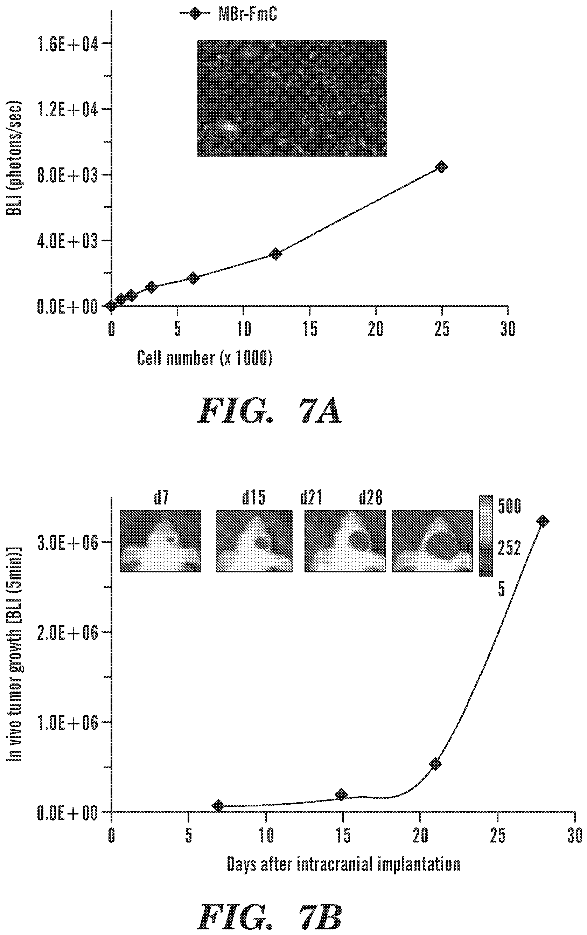

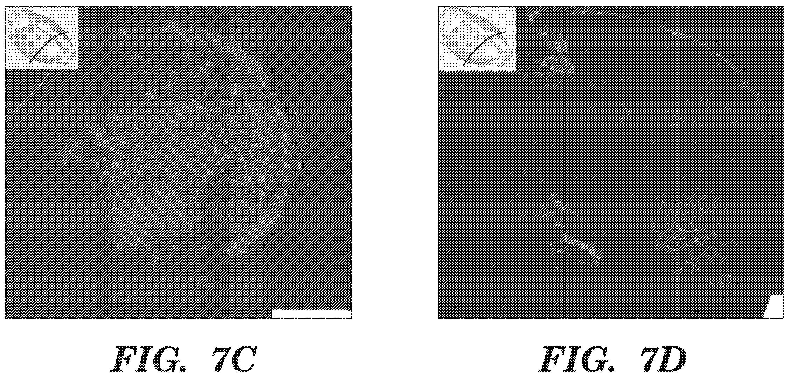

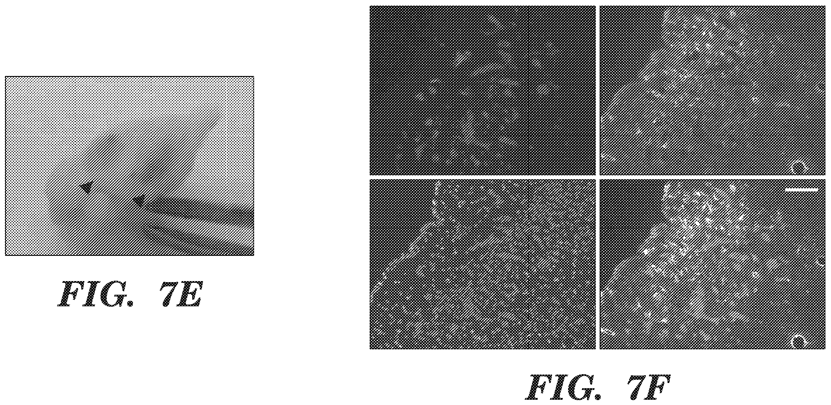

FIGS. 7A-7F show engineering a metastatic breast cancer cell line and characteristics of breast-to-brain metastatic tumors. (FIG. 7A) Representative fluorescent image of MBr-FmC cells transduced with lentiviral vector encoding Flue and mCherry. Plot showing the Flue activity of tumor cells with different cell numbers. **(****FIG. 7B****)** Representative bioluminescent images of MBr-FmC tumors formed by intracranial injection and plot showing the tumor growth over time. **(****FIG. 7C****)** Representative composite fluorescent images of coronal brain sections of MBr-FmC tumors on d28 after intracranially injected. **(****FIG. 7D****)** Composite fluorescent images of a representative brain section after intracarotid injection of MBr-FmC tumor cells. mCherry labeled tumor cells. **(****FIG.** 7E) Arrow head points out the hemorrhage spots in the brain 35 days after intracarotid injection of MBr-FmC cells. **(****FIG. 7F****)** Representative fluorescent images of mCherry-labeled tumor cells and GFAP-labeled reactive astrocytes, **DAPI** stained the nucleus (scale bar = 100 [tm).

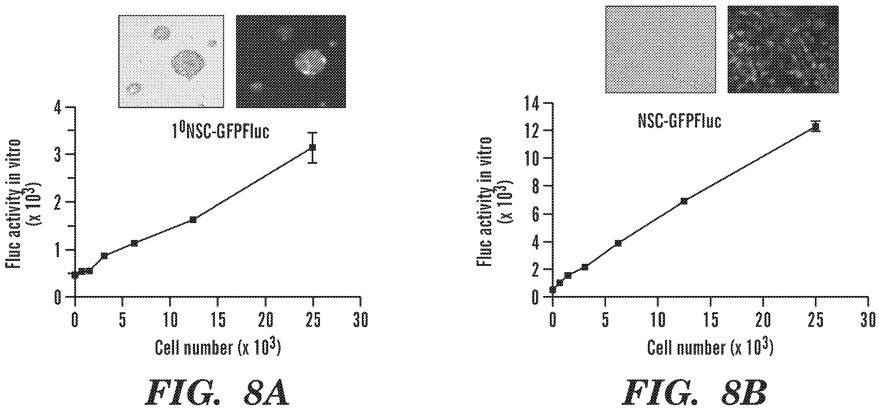

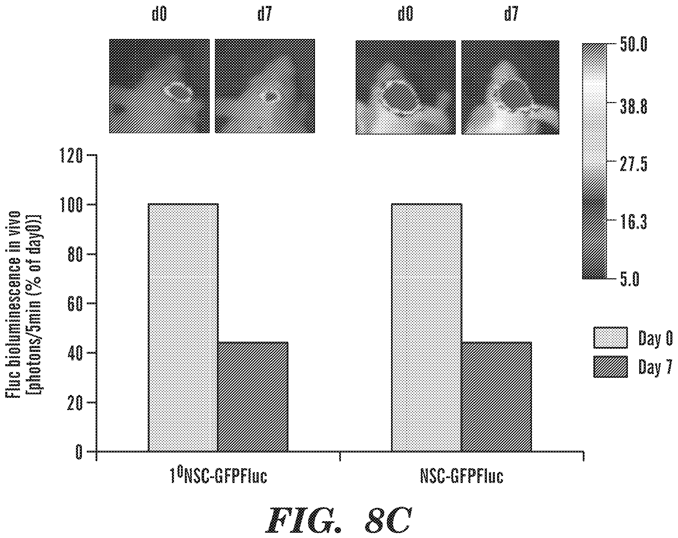

**FIGS. 8A-8C** show an assessment of two NSC lines for their survival ability. **(****FIG. 8A****)** Top: Representative fluorescent image of loNSC transduced with LV-GFP-Fluc. Bottom: Plot showing the Flue activity of loNSC with different cell numbers. **(****FIG. 8B****)** Top: Representative fluorescent image of NSC transduced with LV-GFP-Fluc. Bottom: Plot showing the Flue activity of NSC with different cell numbers. **(****FIG. 8C****)** Survival of NSC in vivo. Top: Representative bioluminescent images of loNSC-GF1 and NSC-GF1 taken on day 0 and 7 after intracranial implantation. Bottom: Plot showing the Flue activity of loNSC-GF1 or NSC-GF1 in vivo.

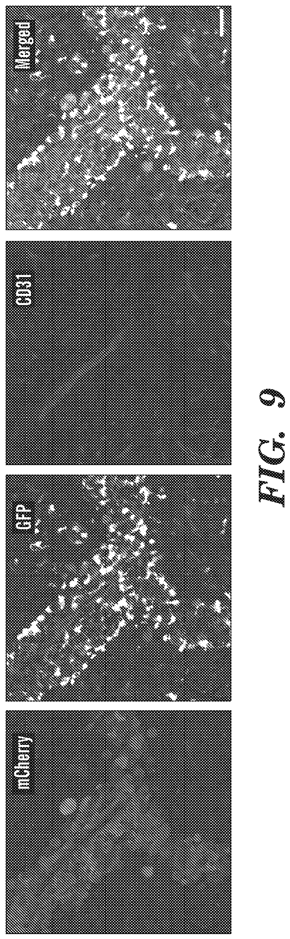

**FIG. 9** shows interaction of NSC, metastatic tumor cells and brain endothelial cells after intraparenchymal injection of NSC. Representative fluorescent images of NSC-GFP and mCherry-labeled tumor cells, and brain endothelium labeled with CD31 (scale bar = 100 [tm).

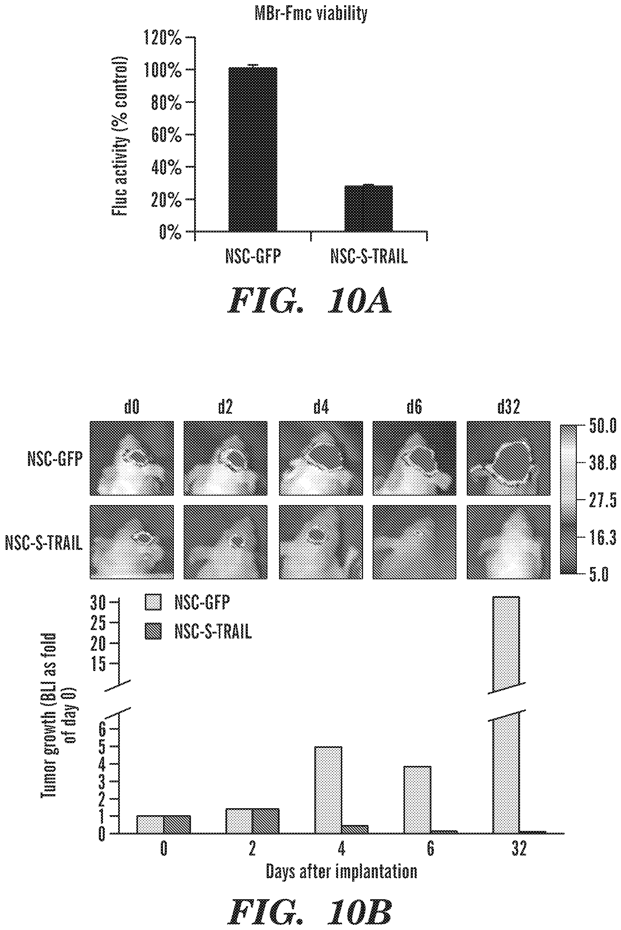

**FIGS. 10A-10B** demonstrate generation and functional characterization of NSC expressing S-TRAIL. **(****FIG. 10A****)** Viability of MBr-FmC cells in the presence of conditioned medium collected from NSC or NSC-S-TRAIL cells. **(****FIG. 10B****)** Top: Representative bioluminescent images of MBr-FmC tumor growth in the presence of NSC-GFP or NSC-S-TRAIL taken on days 0, 2, 4, 6 and 32 after intracranial implantation. Bottom: Plot showing the Flue activity of MBr-FmC tumors admixed with NSC-GFP or NSC-S-TRAIL at the time of implantation.

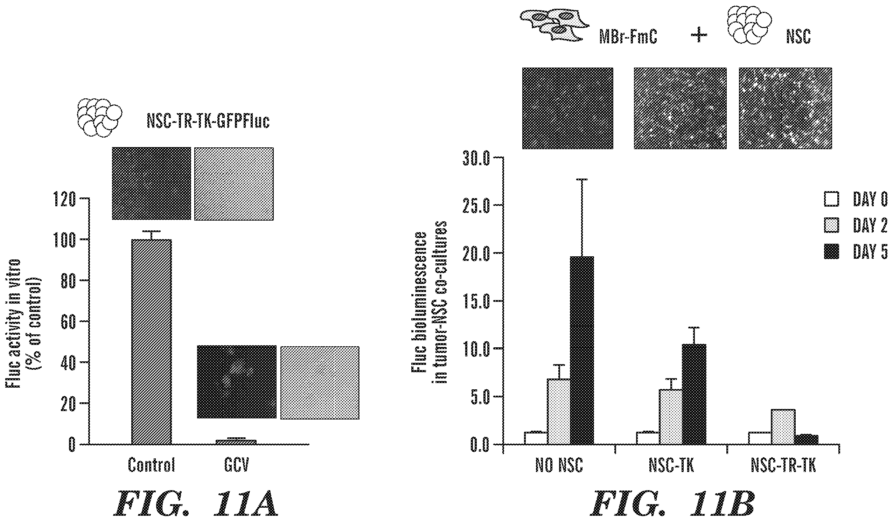

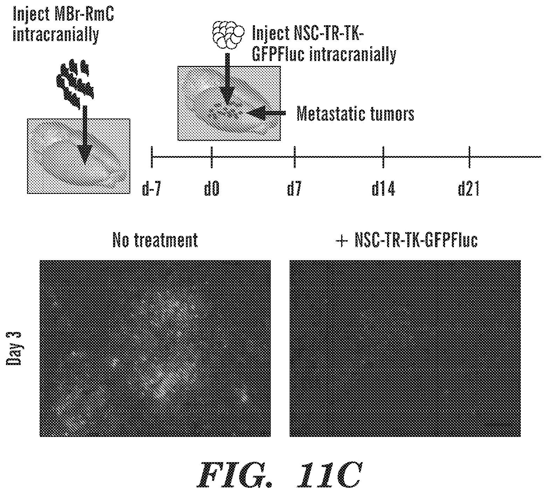

**(****FIG. 11A****)** Representative phase and fluorescent images of NSC-TR-TK-GF1 in the presence or absence of 10 mg/ml GCV. Plot showing the Flue activity of transduced cells in vitro. **(****FIG. 11B****)** Viability of MBr-FmC cells co-cultured with modified NSC at 1:1 ratio as measured by Flue activity in vitro. The effects of NSC engineered with TK or TK-TR are shown. **(****FIG. 11C****)** Upper, outline of the experiment. Representative fluorescent images of metastatic MBr-RmC tumors in the mouse brain with or without NSC-TR-TK-GF1 administration to show post-tumor treatment (scale bar = 100 [tm).

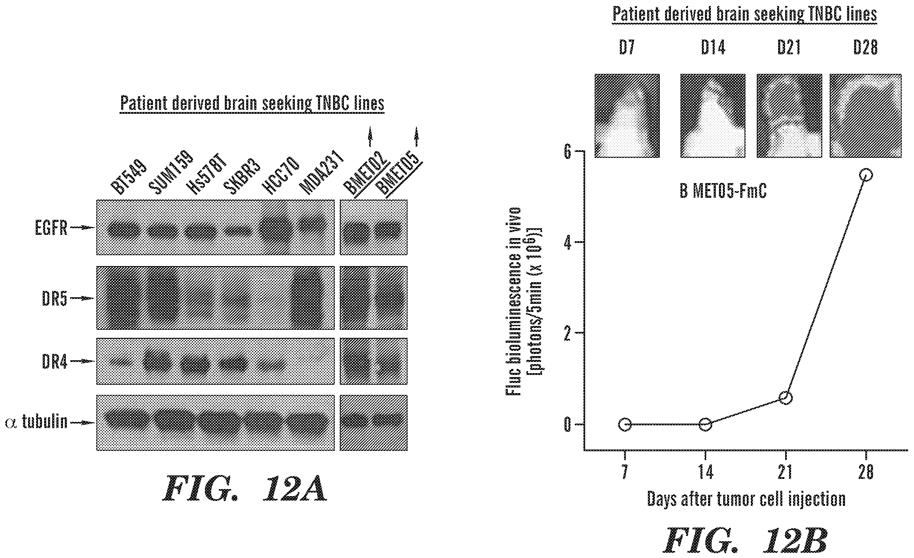

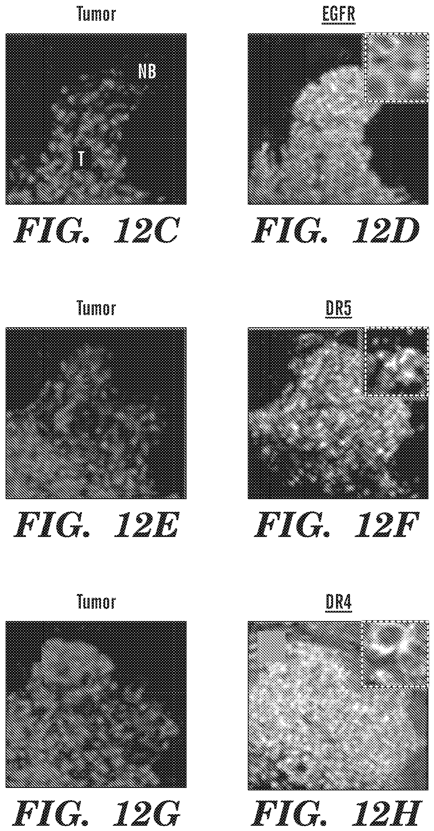

**FIGS. 12A-12H** demonstrate that brain-seeking patient-derived breast cancer cells form tumors in the brain and express EGFR and DRS. **(****FIG. 12A****)** Western blot analysis showing the expression of EGFR, DR4 and DRS in different triple negative breast cancer (TNBC) lines. **(****FIG. 12B****)** Plot and representative **BLI** images showing the tumor growth of patient-derived, brain-seeking breast metastatic BMET05-FmC post-ICA injection. **(****FIGS. 12C-12F****)** Representative fluorescent images showing mCherry tumors **(****FIG. 12C, 12E** and **12G****)** and EGFR **(****FIG. 12D****), DRS (****FIG. 12F****)** and DR4 **(****FIG. 12H****)** immunostaining on brain sections from BMET05 tumor bearing mice. Tumor (T); Normal brain (NB). Mag 10X; insets 40X.

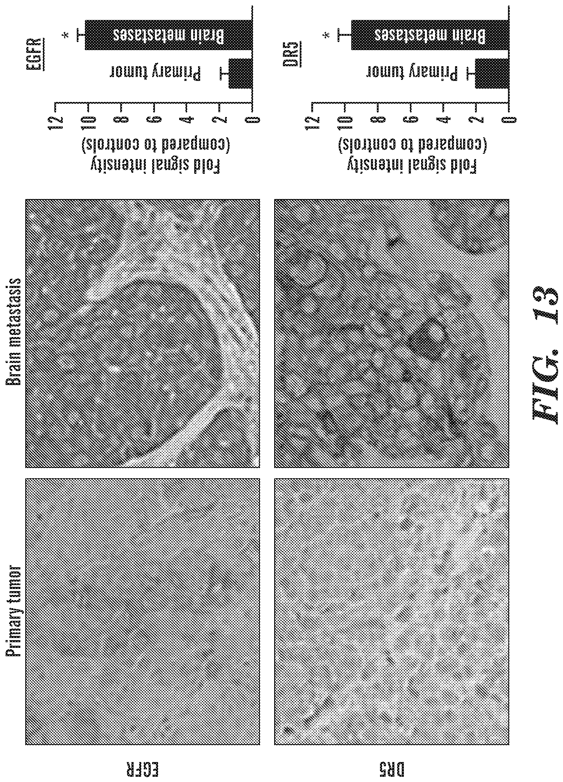

**FIG. 13** demonstrates that metastatic TNBC tumors express higher levels of EGFR and DRS compared to their matched primary breast tumors. Immunohistochemistry (IHC) analysis showing the expression of EGFR and DRS in tumor sections obtained from patients with primary breast tumor and their brain metastases. Plots showing quantification of EGFR and DRS expression.

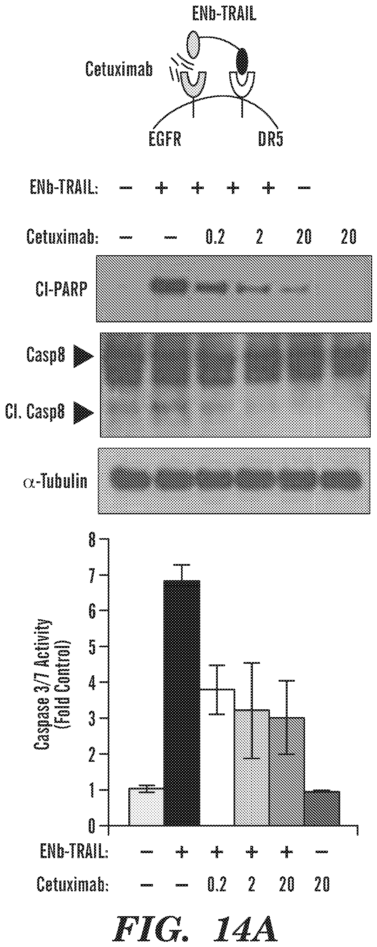

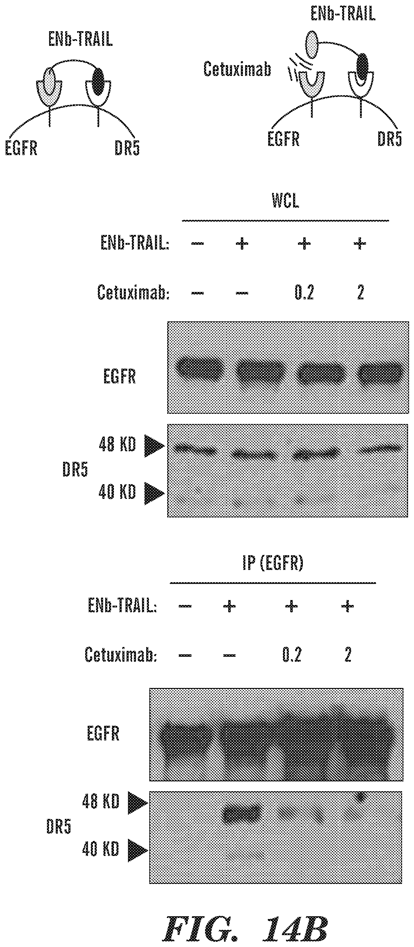

**FIGS. 14A-14B** demonstrate that cetuximab blocks ENb-TRAIL induced apoptosis by attenuating EGFR and DRS complex formation. **(****FIGS. 14A-14B****)** Glioblastoma (GBM) cells were pretreated with cetuximab for 30 min and then treated with ENb-TRAIL for 8h. **(****FIG. 14A****)** Western blot (upper panel) and caspase3/7 assay (lower panel) showing changes in apoptotic markers. **(****FIG. 14B****)** Coimmunoprecipitation and Western blot analysis showing changes in DRS pull down upon cetuximab treatment.

### DETAILED DESCRIPTION

Compositions and methods are provided herein that relate to the therapeutic treatment of multifocal brain metastases.

Among many other biological differences, one major difference between primary and metastatic brain tumors is their localization and distribution in the parenchyma. Although primary brain tumors mostly arise at a single site and progress from there, metastases occur in several distinct spots giving rise to multiple metastatic foci of different sizes (Modha et al., 2005). As described herein, an *in vivo* imageable breast-to-brain metastasis mouse model has been developed, and using real time *in vivo* imaging and subsequent composite fluorescence imaging, a widespread distribution of micro- and macro-metastasis at different stages of metastatic progression is shown. The ability of engineered adult stem cells, such as neural stem cells, to track metastatic deposits in this model was investigated and it was demonstrated that engineered stem cells either implanted or injected via circulation efficiently home to metastatic tumor deposits in the brain. Stem cells, such as neural stem cells, were engineered to express a tumor selective, potent and secretable variant of TRAIL, "STRAIL," and it was demonstrated that these cells significantly suppressed metastatic tumor growth and prolonged the survival of mice bearing metastatic breast tumors. Furthermore, the incorporation of a pro-drug converting enzyme, herpes simplex virus thymidine kinase, into therapeutic S-TRAIL secreting stem cells allowed their eradication post-tumor treatment. Accordingly, provided herein, in some aspects, are stem cells engineered to express metastatic tumor-specific biomolecules for the treatment of multifocal brain metastases.

Further, EGFR-specific nanobodies (ENb) have been engineered and shown to sterically hinder the binding of EGF to the receptor, thereby inhibiting EGFR and its downstream signaling ¹⁸. Based, in part, on the recent findings that metastatic breast cancer cells adopt a novel mechanism of evading death receptor (DR) mediated apoptosis to successfully colonize in the brain ¹⁹; a bimodal soluble TRAIL polypeptide, termed herein "ENb-TRAIL" was engineered comprising cDNA fusions encoding bivalent ENb and the soluble TRAIL ligand, which binds DR4/5. ENb-TRAIL led to tumor specific killing by suppressing tumor cell proliferation and subsequently induces caspase- mediated apoptosis in a broad spectrum of tumor ¹⁸. The data reveal that ENb-TRAIL can be expressed by neural stem cells and that ENb-TRAIL as a single molecule, engages both EGFR and death receptors simultaneously and is more effective than the combination of ENb and soluble TRAIL, as separate agents. In other words, synergistic effects on the treatment of brain metastases were observed using ENb-TRAIL as a single molecule. The delivery of the potent ENb-TRAIL fusion by expression in a stem cell permits the treatment of multifocal metastatic brain tumors via systemic administration of the engineered stem cells.

### Methods of Treating Metastatic Brain Tumors

Metastatic brain tumors are the most commonly observed intracranial tumors, frequently occurring in patients with metastatic cancers of the lung, breast, and skin (melanoma) (Eichler et al., 2011). Most patients have multiple metastatic lesions or foci at the time of diagnosis, making surgery an inadequate therapeutic option on its own. Parallel therapeutic approaches include stereotactic radiosurgery, whole brain radiotherapy and chemotherapy (Kocher et al., 2011).

Given the multistep and complex biological nature of brain metastasis, tumor models that recapitulate metastatic brain tumors are limited. There have been several studies that established tumor lines with metastatic potential (Bos et al., 2010) and specifically a clonal tumor population with brain-seeking properties has been described at a genomic level (Bos et al., 2009). A study reported a novel molecular mechanism for breast cancer cell infiltration into the brain by protection of cancer cells from death signals and allowing for efficient vascular co-option and colonization (Valiente et al., 2014). By expressing serpins, a family of protease inhibitors, metastatic tumor cells were able to adopt a unique defense mechanism and evade stroma-induced apoptosis. Therefore, activating the dormant, cell surface-mediated apoptotic mechanisms on tumor cells can be a favorable therapeutic approach for brain-metastatic tumors.

Accordingly, provided herein are therapeutic methods based on the results described herein, which demonstrate, in part, that stem cells engineered to express a tumor selective, potent and secretable soluble TRAIL polypeptide, either implanted or injected via circulation, efficiently home to metastatic tumor deposits in the brain, and suppress metastatic tumor growth and prolong the survival of mice bearing metastatic breast tumors.

In some aspects, provided herein are methods of treating multifocal metastases in the brain comprising administering to an individual in need thereof a neuronal stem cell engineered to express an sTRAIL polypeptide, whereby the growth and/or survival of multifocal metastases in the brain is/are inhibited.

As used herein, "metastatic brain cancer" or "secondary brain cancer" or "metastatic brain tumor" or "secondary brain tumor" occurs when cancer cells spread to the brain from a primary cancer elsewhere in the body, forming a tumor or tumors in the brain. Such brain cancers are about ten times more common than a cancer that starts or originates in the brain, which is known as "primary brain cancer." Cancer cells can break away from the primary tumor site and travel through blood and lymphatic vessels. Metastases most often appear in the brain at the junction of two types of brain tissue, referred to as gray matter and white matter. This junction is rich with blood vessels of very narrow diameter, and metastatic cells often lodge there.

Metastatic brain tumors are often multifocal in nature and are also referred to herein as "multifocal brain metastases." Although multifocal brain metastases can develop from almost any kind of cancer, those originating in the breast, lung, colon, and kidney, along with malignant melanoma, are the most likely to metastasize to the brain. About half of patients with brain metastases have more than one tumor in the brain.

In most patients with brain metastases, tumors appear in the cerebral cortex, the two large hemispheres of the brain where most high-level functions (such as consciousness, memory, language, and sensory perception) are governed. Fifteen percent of brain metastases develop in the cerebellum, where complex voluntary muscle movements are regulated and coordinated. Five percent of metastatic tumors develop in the brain stem, where functions such as visual coordination, swallowing, and balance are directed. In a small number of patients, brain metastases appear before the primary cancer is discovered in another part of the body. This is called a "metastasis of unknown origin." These tumors can develop when a patient's primary cancer, while still undetected or undetectable at its original site, sends out metastatic cells that travel to the brain and establish themselves there. In these patients, physicians can sometimes biopsy the tumor (depending on its location in the brain), identify the type of cells it is composed of, and determine its site of origin.

Symptoms of brain metastases are quite varied and depend on the location and size of the tumor or tumors. These symptoms can include, for example, headaches, seizures, speech problems, comprehension problems, impaired vision, weakness or numbness in parts of the body, and motor problems.

A "tumor" as used herein refers to an uncontrolled growth of cells which can interfere with the normal functioning of the bodily organs and systems. A "cancer" is an uncontrolled growth or proliferation of cells that has gained the ability for at least some of its cells to leave the primary site of growth and seed other tissues or organs, *i.e.,* the ability to metastasize, as the term is used herein. A patient subject that has a cancer or a tumor, *i.e.,* a cancer or tumor patient, is a subject having objectively measurable cancer (metastasized) or tumor cells present in the subject's body. Included in the definition of a tumor are benign and malignant tumors, as well as dormant tumors or micrometastases. Examples of cancer encompassed within the term include, but are not limited to, carcinoma, lymphoma, blastoma, sarcoma, and leukemia. More particular examples of such cancers include, but are not limited to, basal cell carcinoma, biliary tract cancer; bladder cancer; bone cancer; brain and CNS cancer; breast cancer; cancer of the peritoneum; cervical cancer; cholangiocarcinoma; choriocarcinoma; colon and rectum cancer; connective tissue cancer; cancer of the digestive system; endometrial cancer; esophageal cancer; eye cancer; cancer of the head and neck; gastric cancer (including gastrointestinal cancer); glioblastoma; hepatic carcinoma; hepatoma; intra-epithelial neoplasm; kidney or renal cancer; larynx cancer; leukemia; liver cancer; lung cancer *(e.g*., small-cell lung cancer, non-small cell lung cancer, adenocarcinoma of the lung, and squamous carcinoma of the lung); lymphoma including Hodgkin's and non-Hodgkin's lymphoma; melanoma; myeloma; neuroblastoma; oral cavity cancer *(e.g.,* lip, tongue, mouth, and pharynx); ovarian cancer; pancreatic cancer; prostate cancer; retinoblastoma; rhabdomyosarcoma; rectal cancer; cancer of the respiratory system; salivary gland carcinoma; sarcoma; skin cancer; squamous cell cancer; stomach cancer; teratocarcinoma; testicular cancer; thyroid cancer; uterine or endometrial cancer; cancer of the urinary system; vulval cancer; as well as other carcinomas and sarcomas; as well as B-cell lymphoma (including low grade/follicular non-Hodgkin's lymphoma (NHL); small lymphocytic (SL) NEIL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), tumors of primitive origins and Meigs' syndrome.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

Metastases are most often detected through the sole or combined use of magnetic resonance imaging (MRI) scans, computed tomography (CT) scans, blood and platelet counts, liver function studies, chest X-rays and bone scans in addition to the monitoring of specific symptoms.

In some embodiments, the methods further comprise administering to a subject having multifocal metastases in the brain one or more anti-cancer therapies or agents. The term "anti-cancer therapy" refers to a therapy useful in treating cancer. Examples of anti-cancer therapeutic agents include, but are not limited to, *e.g*., surgery, chemotherapeutic agents, immunotherapies, growth inhibitory agents, cytotoxic agents, agents used in radiation therapy, anti-angiogenesis agents, apoptotic agents, anti-tubulin agents, and other agents to treat cancer, such as anti-HER-2 antibodies *(e.g*., HERCEPTINO), anti-CD20 antibodies, an epidermal growth factor receptor (EGFR) antagonist *(e.g.,* a tyrosine kinase inhibitor), HER1/EGFR inhibitor *(e.g.,* erlotinib (TARCEVA^{®})), platelet derived growth factor inhibitors *(e.g.,* GLEEVEC^{™} (Imatinib Mesylate)), a COX-2 inhibitor (*e.g.,* celecoxib), interferons, cytokines, antagonists (*e.g*., neutralizing antibodies) that bind to one or more of the following targets PD1, PDL1, PDL2, TIM3 or any TIM family member, CEACAM1 or any CEACAM family member, ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also specifically contemplated for the methods described herein.

The term "cytotoxic agent" as used herein refers to a substance that inhibits or prevents the function of cells and/or causes destruction of cells. The term is intended to include radioactive isotopes *(e.g. At²¹¹* , *₁131* ₁125, y-90, _{Re}186_{, Re}188_{,} sₘ153, _{B}i212, P³² and radioactive isotopes of Lu), chemotherapeutic agents, and toxins such as small molecule toxins or enzymatically active toxins of bacterial, fungal, plant or animal origin, including active fragments and/or variants thereof

In some embodiments, the methods further comprise administering to a subject having multifocal metastases in the brain one or more immunotherapies. As used herein, the term "immunotherapy" refers to the treatment of disease via the stimulation, induction, subversion, mimicry, enhancement, augmentation or any other modulation of a subject's immune system to elicit or amplify adaptive or innate immunity (actively or passively) against cancerous or otherwise harmful proteins, cells or tissues. Immunotherapies *(i.e.,* immunotherapeutic agents) include cancer vaccines, immunomodulators, "antibody-based immunotherapies" or monoclonal antibodies *(e.g.*, humanized monoclonal antibodies), immunostimulants, cell-based therapies such as adoptive T-cell therapies or dendritic cell immunotherapies or dendritic cell vaccines, and viral therapies, whether designed to treat existing cancers or prevent the development of cancers or for use in the adjuvant setting to reduce likelihood of recurrence of cancer.

In some such embodiments, the immunotherapy is an adoptive T-cell therapy, which, as used herein, includes T-cell therapies in which T-cells are expanded *in vitro* using cell culture methods relying on the immunomodulatory action of interleukin-2 and returning these to the patient in large numbers intravenously in an activated state. Adoptive T-cell therapies can involve genetically engineering a subject's or patient's own T cells to produce recombinant receptors on their surface referred to as "chimeric antigen receptors" (CARs).

In some such embodiments, the immunotherapy involves dendritic cell vaccination. "Dendritic cell vaccination" refers to a form of immunotherapy designed to induce T cell-dependent immunity, such as cancer-specific T cell-dependent anti-tumor immunity, that can result in durable complete responses using DCs. Examples of "dendritic cell (DC) immunotherapies" or "dendritic cell vaccines," as used herein, include modified dendritic cells and any other antigen presenting cell, autologous or xeno, whether modified by multiple antigens, whole cancer cells, single antigens, by mRNA, phage display or any other modification, including but not restricted to ex vivo-generated, antigen-loaded dendritic cells (DCs) to induce antigen-specific T-cell immunity, *ex vivo* gene-loaded DCs to induce humoral immunity, *ex* vivo-generated, antigen-loaded DCs to induce tumor-specific immunity, *ex* vivo-generated immature DCs to induce tolerance, including, but not limited to, Provenge and others.

In some embodiments, the methods further comprise administering to a subject having multifocal metastases in the brain one or more chemotherapeutic agents.

As used herein, the terms "chemotherapy" or "chemotherapeutic agent" refer to any chemical agent with therapeutic usefulness in the treatment of diseases characterized by abnormal cell growth. Such diseases include tumors, neoplasms and cancer as well as diseases characterized by hyperplastic growth. Chemotherapeutic agents as used herein encompass both chemical and biological agents. These agents function to inhibit a cellular activity upon which the cancer cell depends for continued survival. Categories of chemotherapeutic agents include alkylating/alkaloid agents, antimetabolites, hormones or hormone analogs, and miscellaneous antineoplastic drugs. Most if not all of these agents are directly toxic to cancer cells and do not require immune stimulation. In one embodiment, a chemotherapeutic agent is an agent of use in treating neoplasms such as solid tumors. In one embodiment, a chemotherapeutic agent is a radioactive molecule. One of skill in the art can readily identify a chemotherapeutic agent of use *(e.g.* see Physicians' Cancer Chemotherapy Drug Manual 2014, Edward Chu, Vincent T. DeVita Jr., Jones & Bartlett Learning; Principles of Cancer Therapy, Chapter 85 in Harrison's Principles of Internal Medicine, 18th edition; Therapeutic Targeting of Cancer Cells: Era of Molecularly Targeted Agents and Cancer Pharmacology, Chs. 2829 in Abeloff s Clinical Oncology, 2013 Elsevier; Baltzer L, Berkery R (eds): Oncology Pocket Guide to Chemotherapy, 2nd ed. St. Louis, Mosby-Year Book, 1995; Fischer D S (ed): The Cancer Chemotherapy Handbook, 4th ed. St. Louis, Mosby-Year Book, 2003).

Non-limiting examples of chemotherapeutic agents can include alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics *(e.g.,* calicheamicin, especially calicheamicin gammall and calicheamicin omegall (see, *e.g.,* Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol-Myers Squibb Oncology, Princeton, N.J.), ABRAXANE^{®} Cremophor-free, albuminengineered nanoparticle formulation of paclitaxel (American Pharmaceutical Partners, Schaumberg, Ill.), and TAXOTERE^{®} doxetaxel (Rhone-Poulenc Rorer, Antony, France); chloranbucil; GEMZAR^{®} gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE; vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylomithine (DMFO); retinoids such as retinoic acid; capecitabine; combretastatin; leucovorin (LV); oxaliplatin, including the oxaliplatin treatment regimen (FOLFOX); lapatinib (TYKERB, inhibitors of PKC-alpha, Raf, H-Ras, EGFR *(e.g*., erlotinib (TARCEVA^{®})) and VEGF-A that reduce cell proliferation and pharmaceutically acceptable salts, acids or derivatives of any of the above.

In addition, the methods of treatment can further include the use of radiation or radiation therapy. By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

In some embodiments, surgical treatments are not performed in conjunction with the methods described herein.

In some embodiments, the methods of treatment can further include the use of surgical treatments.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder associated with a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation or at least slowing of progress or worsening of symptoms that would be expected in absence of treatment. Beneficial or desired clinical results include, but are not limited to, alleviation of one or more symptom(s), diminishment of extent of disease, stabilized *(i.e.,* not worsening) state of disease, delay or slowing of disease progression, amelioration or palliation of the disease state, and remission (whether partial or total). The term "treatment" of a disease also includes providing relief from the symptoms or side-effects of the disease (including palliative treatment).

For example, in some embodiments, the methods described herein comprise administering an effective amount of a soluble TRAIL polypeptide or cells engineered to express or secrete a soluble TRAIL polypeptide described herein to a subject in order to alleviate a symptom of a cancer. As used herein, "alleviating a symptom of a cancer" is ameliorating any condition or symptom associated with the cancer. As compared with an equivalent untreated control, such reduction or degree of prevention is at least 5%, at least 10%, at least 20%, at least 40%, at least 50%, at least 60%, at least 70%, at least 80%, at least 90%, at least 95%, or 100% as measured by any standard technique.

The term "effective amount" as used herein refers to the amount of, for example, a soluble TRAIL polypeptide or cells engineered to express or secrete a soluble TRAIL polypeptide needed to alleviate at least one or more symptom of the disease or disorder, and relates to a sufficient amount of pharmacological composition to provide the desired effect. The term "therapeutically effective amount" therefore refers to an amount of the soluble TRAIL polypeptide or cells engineered to express or secrete a soluble TRAIL polypeptide using the methods as disclosed herein, that is sufficient to effect a particular effect when administered to a typical subject. An effective amount as used herein would also include an amount sufficient to delay the development of a symptom of the disease, alter the course of a symptom disease (for example but not limited to, slow the progression of a symptom of the disease), or reverse a symptom of the disease. Thus, it is not possible to specify the exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, *e.g*., for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50, or the concentration which achieves a half-maximal inhibition of symptoms, as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

### Soluble TRAIL Polypeptides

Tumor necrosis factor (ligand) superfamily, member 10 (TRAIL) belongs to the TNF receptor ligand family, which also includes TNF and FASLG (Wiley et al., 1995; Pan al., 1997). It is well known that they are capable of inducing apoptosis in a number of cancer cells via binding to their cognate receptors and the initiation of death receptor mediated signaling (French and Tschopp, 1999). TRAIL is normally expressed on both normal and tumor cells as a non-covalent homotrimeric type-II transmembrane protein (memTRAIL). In addition, a naturally occurring soluble form of TRAIL (solTRAIL) can be generated by alternative mRNA splicing or proteolytic cleavage of the extracellular domain of memTRAIL. Such solTRAIL and retains tumor-selective pro-apoptotic activity. TRAIL utilizes an intricate receptor system comprising four distinct membrane receptors, designated TRAIL-R1 (also referred to as "DR4"), TRAIL-R2 (also referred to as "DRS"), TRAIL-R3 and TRAIL-R4. Of these receptors, only TRAIL-R1 and TRAIL-2 transmit an apoptotic signal. These two receptors belong to a subgroup of the TNF receptor family, the so-called death receptors (DRs), and contain the hallmark intracellular death domain (DD). This DD is critical for apoptotic signaling by death receptors (J. M. A. Kuijlen et al., Neuropathology and Applied Neurobiology, 2010 Vol. 36 (3), pp.168-182).

TRAIL activates the "extrinsic pathway" of apoptosis by binding to TRAIL-R1 and/or TRAIL-R2, whereupon the adaptor protein Fas-associated death domain and initiator caspase-8 are recruited to the DD of these receptors. Assembly of this "death-inducing signaling complex" (DISC) leads to the sequential activation of initiator and effector caspases, and ultimately results in apoptotic cell death. The extrinsic apoptosis pathway triggers apoptosis independently of p53 in response to pro-apoptotic ligands, such as TRAIL. TRAIL-R1 can induce apoptosis after binding non-cross-linked and cross-linked sTRAIL. TRAIL-R2 can only be activated by cross-linked sTRAIL. Death receptor binding leads to the recruitment of the adaptor FADD and initiator procaspase-8 and 10 to rapidly form the DISC. Procaspase-8 and 10 are cleaved into their activated configuration caspase-8 and 10. Caspase-8 and 10 in turn activate the effector caspase-3, 6 and 7, thus triggering apoptosis.

In certain cells, the execution of apoptosis by TRAIL further relies on an amplification loop via the "intrinsic mitochondrial pathway" of apoptosis. The mitochondrial pathway of apoptosis is a stress-activated pathway, *e.g*., upon radiation, and hinges on the depolarization of the mitochondria, leading to release of a variety of pro-apoptotic factors into the cytosol. Ultimately, this also triggers effector caspase activation and apoptotic cell death. This mitochondrial release of pro-apoptotic factors is tightly controlled by the Bcl-2 family of pro- and anti-apoptotic proteins. In the case of TRAIL receptor signaling, the Bcl-2 homology (BH3) only protein 'Bid' is cleaved into a truncated form (tBid) by active caspase-8. Truncated Bid subsequently activates the mitochondrial pathway.

TRAIL-R3 is a glycosylphosphatidylinositol-linked receptor that lacks an intracellular domain, whereas TRAIL-R4 only has a truncated and non-functional DD. The latter two receptors are thought, without wishing to be bound or limited by theory, to function as decoy receptors that modulate TRAIL sensitivity. Evidence suggests that TRAIL-R3 binds and sequesters TRAIL in lipid membrane microdomains. TRAIL-R4 appears to form heterotrimers with TRAIL-R2, whereby TRAIL-R2-mediated apoptotic signaling is disrupted. TRAIL also interacts with the soluble protein osteoprotegerin.

Diffuse expression of TRAIL has been detected on liver cells, bile ducts, convoluted tubules of the kidney, cardiomyocytes, lung epithelia, Leydig cells, normal odontogenic epithelium, megakaryocytic cells and erythroid cells. In contrast, weak or absent expression of TRAIL was observed in colon, glomeruli, Henle's loop, germ and Sertoli cells of the testis, endothelia in several organs, smooth muscle cells in lung, spleen and in follicular cells in the thyroid gland. TRAIL protein expression was demonstrated in glial cells of the cerebellum in one study. Vascular brain endothelium appears to be negative for TRAIL-R1 and weakly positive for TRAIL-R2. With regard to the decoy receptors, TRAIL-R4 and TRAIL-R3 have been detected on oligodendrocytes and neurons.

TRAIL-R1 and TRAIL-R2 are ubiquitously expressed on a variety of tumor types. Importantly for the compositions and methods involving TRAIL agents described herein, TRAIL-R1 and TRAIL-R2 are also expressed in the tumor tissue from astrocytoma grade II and glioblastoma patients. In a study on 62 primary GBM tumor specimens, TRAIL-R1 and TRAIL-R2 were expressed in 75% and 95% of the tumors, respectively. Of note, a statistically significant positive association was identified between agonistic TRAIL receptor expression and survival. Highly malignant tumors express a higher amount of TRAIL receptors in comparison with less malignant tumors or normal tissue. In general TRAIL-R2 is more frequently expressed on tumor cells than TRAIL-R1.

Accordingly, the term "Tumor necrosis factor-related apoptosis-inducing ligand" or "TRAIL" as used herein refers to the 281 amino acid polypeptide having the amino acid sequence of: MAMMEVQGGPSLGQTCVLIVIFTVLLQSLCVAVTYVYFTNELKQMQDKYSKSGIACFLKED DSYWDPNDEESMNSPCWQVKWQLRQLVRKMILRTSEETISTVQEKQQNISPLVRERGPQRV AAHITGTRGRSNTLSSPNSKNEKALGRKINSWESSRSGHSFLSNLHLRNGELVIHEKGFYYTYS QTYFRFQEEIKENTKNDKQMVQYIYKYTSYPDPILLMKSARNSCWSKDAEYGLYSIYQGGIF ELKENDRIFVSVTNEHLIDMDHEASFFGAFLVG (SEQ ID NO: 1), as described by, *e.g*., GenBank Accession No. NP 003801.1 , together with any naturally occurring allelic, splice variants, and processed forms thereof. Typically, TRAIL refers to human TRAIL. The term TRAIL, in some embodiments of the aspects described herein, is also used to refer to truncated forms or fragments of the TRAIL polypeptide, comprising, for example, specific TRAIL domains or residues thereof. Reference to any such forms of TRAIL can be identified in the application, *e.g*., by "TRAIL (39281)" or "amino acids 39-281 of SEQ ID NO: 1." The amino acid sequence of the human TRAIL molecule as presented in SEQ ID NO: 1 comprises an N-terminal cytoplasmic domain (amino acids 118), a transmembrane domain (amino acids 19-38), and an extracellular domain (amino acids 39-281). The extracellular domain comprises the TRAIL receptor-binding region. TRAIL also has a spacer region between the C-terminus of the transmembrane domain and a portion of the extracellular domain. This spacer region, located at the N-terminus of the extracellular domain, consists of amino acids 39 through 94 of SEQ ID NO: 1. Amino acids 138 through 153 of SEQ ID NO: 1 correspond to a loop between the 13 sheets of the folded (three dimensional) human TRAIL protein.

Accordingly, in some embodiments of the aspects described herein, a soluble TRAIL polypeptide comprises the extracellular domain of TRAIL, but lacks the transmembrane domain. In some embodiments of the aspects described herein, a soluble TRAIL polypeptide is a fusion protein comprising one or more domains of TRAIL (e.g., the extracellular domain) fused to a heterologous sequence. In some embodiments of the aspects described herein, a soluble TRAIL polypeptide further comprises a signal for secretion.

In some embodiments of the aspects described herein, a soluble TRAIL polypeptide comprises a therapeutic TRAIL module or therapeutic TRAIL domain or variant thereof having TRAIL apoptotic activity. As used herein, a "therapeutic TRAIL module," "therapeutic TRAIL domain," or "therapeutic TRAIL variant" refers to a polypeptide, or a nucleotide sequence encoding such a polypeptide, comprising an extracellular domain of human TRAIL, such as a human TRAIL of SEQ ID NO: 1, and maintaining TRAIL functional, i.e., apoptotic, activity. In some embodiments of the aspects described herein, an N-terminal secretion signal sequence is fused to the N-terminus of the extracellular domain of human TRAIL. In some embodiments of the aspects described herein, the therapeutic TRAIL module can further comprise an isoleucine zipper domain.

In some embodiments of the aspects described herein, the extracellular domain of human TRAIL comprises amino acids 39-281 of SEQ ID NO: 1. In some embodiments of the aspects described herein, the extracellular domain of human TRAIL comprises amino acids 95-281 of SEQ ID NO: 1. In some embodiments of the aspects described herein, the extracellular domain of human TRAIL comprises amino acids 114-281 of SEQ ID NO: 1. In some embodiments of the aspects described herein, the extracellular domain of human TRAIL comprises a sequence having at least 90% identity to amino acids 114-281 of SEQ ID NO: 1 and retains TRAIL apoptotic activity. In some embodiments of the aspects described herein, the extracellular domain of human TRAIL used in a soluble TRAIL polypeptide consists essentially of amino acids 114-281 of SEQ ID NO: 1. In some embodiments of the aspects described herein, the extracellular domain of human TRAIL used in a soluble TRAIL polypeptide consists of amino acids 114-281 of SEQ ID NO: 1.

Variants and derivatives of native TRAIL proteins for use in the soluble TRAIL polypeptides that retain a desired biological activity of TRAIL, such as "TRAIL apoptotic activity" are also within the scope of the compositions and methods described herein. In some embodiments, the biological or apoptotic activity of a therapeutic TRAIL module is essentially equivalent to the biological activity of a native TRAIL protein. In some such embodiments, biological activity of a native TRAIL protein is TRAIL apoptotic activity. In some embodiments, 100% of the apoptotic activity is retained by the soluble TRAIL polypeptide comprising a TRAIL fragment, variant or derivative. In some embodiments, at least 100% activity is retained (e.g., at least 95%, at least 90%, at least 85%, at least 80%, at least 75%, at least 70%, at least 65%, at least 60%, at least 55%, at least 50%, at least 45%, or at least 40%) as compared to the full length native TRAIL. Nonetheless, a "TRAIL fragment, derivative, or variant" that has less than 100% of the apoptotic activity of full-length native TRAIL must retain at least 40% of the apoptotic activity of the native, full-length TRAIL polypeptide. TRAIL fragments that retain the apoptotic activity of TRAIL are known in the art, and include, for example, biologically active domains and fragments disclosed in Wiley et al. (U.S. Patent Publication 20100323399).

One measurement of TRAIL apoptotic activity by a TRAIL variant or TRAIL domain is the ability to induce apoptotic death of Jurkat cells. Assay procedures for identifying biological activity of TRAIL variants by detecting apoptosis of target cells, such as Jurkat cells, are well known in the art. DNA laddering is among the characteristics of cell death via apoptosis, and is recognized as one of the observable phenomena that distinguish apoptotic cell death from necrotic cell death. Apoptotic cells can also be identified using markers specific for apoptotic cells, such as Annexin V, in combination with flow cytometric techniques, as known to one of skill in the art. Further examples of assay techniques suitable for detecting death or apoptosis of target cells include those described in WO2012/106281.

TRAIL variants can be obtained by mutations of native TRAIL nucleotide sequences, for example. A "TRAIL variant," as referred to herein, is a polypeptide substantially homologous to a native TRAIL, but which has an amino acid sequence different from that of native TRAIL because of one or a plurality of deletions, insertions or substitutions. "TRAIL encoding DNA sequences" encompass sequences that comprise one or more additions, deletions, or substitutions of nucleotides when compared to a native TRAIL DNA sequence, but that encode a TRAIL protein or fragment thereof that is essentially biologically equivalent to a native TRAIL protein, *i.e.* , has the same apoptosis inducing activity.

The variant amino acid or DNA sequence preferably is at least 70%, at least 75%, at least 80%, at least 85%, at least 90%, at least 91%, at least 92%, at least 93%, at least 94%, at least 95%, at least 96%, at least 97%, at least 98%, at least 99%, or more, identical to a native TRAIL sequence, and retains at least 40% of the apoptotic activity of the native, full-length TRAIL polypeptide. The degree of homology or percent identity between a native and a mutant sequence can be determined, for example, by comparing the two sequences using widely available computer programs commonly employed for this purpose on the world wide web.

Alterations of the native amino acid sequence can be accomplished by any of a number of known techniques known to one of skill in the art. Mutations can be introduced, for example, at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to provide an altered nucleotide sequence having particular codons altered according to the substitution, deletion, or insertion required. Techniques for making such alterations, among others, include those disclosed by Walder et al. (Gene 42:133, 1986); Bauer et al. (Gene 37:73, 1985); Craik (BioTechniques, January 1985, 12-19); Smith et al. (Genetic Engineering: Principles and Methods, Plenum Press, 1981); and U.S. Pat. Nos. 4,518,584 and 4,737,462.

TRAIL variants can, in some embodiments, comprise conservatively substituted sequences, meaning that one or more amino acid residues of a native TRAIL polypeptide are replaced by different residues, and that the conservatively substituted TRAIL polypeptide retains a desired biological activity, *i.e.,* apoptosis inducing activity or TRAIL apoptotic activity, that is essentially equivalent to that of the native TRAIL polypeptide or is at least 40% of the apoptotic activity of the native, full-length TRAIL polypeptide. Examples of conservative substitutions include substitution of amino acids that do not alter the secondary and/or tertiary structure of TRAIL.

In other embodiments, TRAIL variants can comprise substitution of amino acids that have not been evolutionarily conserved. Conserved amino acids located in the C-terminal portion of proteins in the TNF family, and believed to be important for biological activity, have been identified. These conserved sequences are discussed in Smith et al. (Cell, 73:1349, 1993, see page 1353 and FIG. 6); Suda et al. (Cell, 75:1169, 1993, see FIG. 7); Smith et al. (Cell, 76:959, 1994, see FIG. 3); and Goodwin et al. (Eur. J. Immunol., 23:2631, 1993, see FIG. 7 and pages 2638-39). Advantageously, in some embodiments, these conserved amino acids are not altered when generating conservatively substituted sequences. In some embodiments, if altered, amino acids found at equivalent positions in other members of the TNF family are substituted. Among the amino acids in the human TRAIL protein of SEQ ID NO:1 that are conserved are those at positions 124-125 (AH), 136 (L), 154 (W), 169 (L), 174 (L), 180 (G), 182 (Y), 187 (Q), 190 (F), 193 (Q), and 275-276 (FG) of SEQ ID NO:1. Another structural feature of TRAIL is a spacer region *(i.e.,* TRAIL (39-94)) between the C-terminus of the transmembrane region and the portion of the extracellular domain that is believed to be important for biological apoptotic activity. In some embodiments, when the desired biological activity of TRAIL domain is the ability to bind to a receptor on target cells and induce apoptosis of the target cells, substitution of amino acids occurs outside of the receptor-binding domain.

A given amino acid of a TRAIL domain used in a soluble TRAIL polypeptide described herein can, in some embodiments, be replaced by a residue having similar physiochemical characteristics, *e.g*., substituting one aliphatic residue for another (such as Ile, Val, Leu, or Ala for one another), or substitution of one polar residue for another (such as between Lys and Arg; Glu and Asp; or Gln and Asn). Other such conservative substitutions, *e.g*., substitutions of entire regions having similar hydrophobicity characteristics, are well known. TRAIL polypeptides comprising conservative amino acid substitutions can be tested in any one of the assays described herein to confirm that a desired degree of TRAIL apoptotic activity of a native TRAIL molecule is retained.

Amino acids can be grouped according to similarities in the properties of their side chains (in A. L. Lehninger, in Biochemistry, second ed., pp. 73-75, Worth Publishers, New York (1975)): (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp **(D),** Glu (E); (4) basic: Lys (K), Arg (R), His (H).

Alternatively, naturally occurring residues can be divided into groups based on common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; (6) aromatic: Trp, Tyr, Phe. Non-conservative substitutions will entail exchanging a member of one of these classes for another class.

Particularly preferred conservative substitutions for use in the TRAIL variants described herein are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp; and/or Phe into Val, into Ile or into Leu.

Any cysteine residue not involved in maintaining the proper conformation of the soluble TRAIL polypeptides also can be substituted, generally with serine, to improve the oxidative stability of the molecule and prevent aberrant crosslinking. Conversely, cysteine bond(s) can be added to the soluble TRAIL polypeptide to improve its stability or facilitate oligomerization.

The soluble TRAIL polypeptides described herein can further comprise, in some embodiments, a secretion signal sequence that permits a cell engineered to express a soluble TRAIL polypeptide to secrete it. As used herein, the terms "secretion signal sequence," "secretion sequence," "secretion signal peptide," or "signal sequence," refer to a sequence that is usually about 3-60 amino acids long and that directs the transport of a propeptide to the endoplasmic reticulum and through the secretory pathway during protein translation. As used herein, a signal sequence, which can also be known as a signal peptide, a leader sequence, a prepro sequence or a pre sequence, does not refer to a sequence that targets a protein to the nucleus or other organelles, such as mitochondria, chloroplasts and apicoplasts. In some embodiments of the soluble TRAIL polypeptides described herein, a "secretion signal sequence" comprises 5 to 15 amino acids with hydrophobic side chains that are recognized by a cytosolic protein, SRP (Signal Recognition Particle), which stops translation and aids in the transport of an mRNA-ribosome complex to a translocon in the membrane of the endoplasmic reticulum. In some embodimentsof the soluble TRAIL polypeptides described herein, the signal peptide comprises at least three regions: an amino-terminal polar region (N region), where frequently positive charged amino acid residues are observed, a central hydrophobic region (H region) of 7-8 amino acid residues and a carboxy-terminal region (C region) that includes the cleavage site. Commonly, the signal peptide is cleaved from the mature protein with cleavage occurring at this cleavage site.

In some embodiments, the secretory signal sequence is operably linked to the sequence encoding the TRAIL module of the soluble TRAIL polypeptides described herein, such that the two sequences are joined in the correct reading frame and positioned to direct the newly synthesized polypeptide into the secretory pathway of the host cell. Secretory signal sequences are commonly positioned 5' to the nucleotide sequence encoding the polypeptide of interest, although certain secretory signal sequences can be positioned elsewhere in the nucleotide sequence of interest (see, *e.*g., Welch et al., U.S. Pat. No. 5,037,743; Holland et al., U.S. Pat. No. 5,143,830).

In some embodiments of the aspects described herein, the secretory sequence comprises amino acids 1-81 of the Flt3L amino acid sequence having the sequence MTVLAPAWSP NSSLLLLLLL LSPCLRGTPD CYFSHSPISS NFKVKFRELT DHLLKDYPVT VAVNLQDEKH CKALWSLFLA QRWIEQLKTV AGSKMQTLLE DVNTEIHFVT SCTFQPLPEC LRFVQTNISH LLKDTCTQLL ALKPCIGKAC QNFSRCLEVQ CQPDSSTLLP PRSPIALEAT ELPEPRPRQL LLLLLLLLPL TLVLLAAAWG LRWQRARRRG ELHPGVPLPS HP **(SEQ ID NO: 2,** GenBank Accession P49772), or a fragment thereof In some embodiments of the aspects described herein, the signal peptide comprises amino acids 1-81 of SEQ ID NO: 2. In some embodiments of the aspects described herein, the secretory signal sequence comprises a sequence having at least 90% identity to amino acids 1-81 of SEQ ID NO: 2. In some embodiments of the aspects described herein, the secretory signal sequence consists essentially of amino acids 1-81 of SEQ ID NO: 2. In some embodiments of the aspects described herein, the secretory signal sequence consists of amino acids 1-81 of SEQ ID NO: 2.

While the secretory signal sequence can be derived from Flt3L, in other embodiments a suitable signal sequence can also be derived from another secreted protein or synthesized *de novo.* Other secretory signal sequences which can be substituted for the Flt3L signal sequence for expression in eukaryotic cells include, for example, naturally-occurring or modified versions of the human IL-17RC signal sequence, otPA pre-pro signal sequence, human growth hormone signal sequence, human CD33 signal sequence Ecdysteroid Glucosyltransferase (EGT) signal sequence, honey bee Melittin (Invitrogen Corporation; Carlsbad, Calif.), baculovirus gp67 (PharMingen: San Diego, Calif.) (US Pub. No. 20110014656). Additional secretory sequences include secreted alkaline phosphatase signal sequence, interleukin-1 signal sequence, CD-14 signal sequence and variants thereof (US Pub. No. 20100305002) as well as the following peptides and derivatives thereof: Sandfly Yellow related protein signal peptide, silkworm friboin LC signal peptide, snake PLA2, *Cyrpidina noctiluca* luciferase signal peptide, and pinemoth fibroin LC signal peptide (US Pub. No. 20100240097). Further signal sequences can be selected from databases of protein domains, such as SPdb, a signal peptide database described in Choo et al., BMC Bioinformatics 2005, 6:249, LOCATE, a mammalian protein localization database described in Sprenger et al. Nuc Acids Res, 2008, 36:D230D233, or identified using computer modeling by those skilled in the art (Ladunga, Curr Opin Biotech 2000, 1:13-18).

Selection of appropriate signal sequences and optimization or engineering of signal sequences is known to those skilled in the art (Stern et al., Trends in Cell & Molecular Biology 2007 2:1-17; Barash et al., Biochem Biophys Res Comm 2002, 294:835-842). In some embodiments, signal sequences can be used that comprise a protease cleavage site for a site-specific protease *(e.g*., Factor IX or Enterokinase). This cleavage site can be included between the pro sequence and the bioactive secreted peptide sequence, e.g., TRAIL domain, and the pro-peptide can be activated by the treatment of cells with the site-specific protease (US Pub. No. 20100305002).

The soluble TRAIL polypeptides described herein can, in some embodiments, further comprise a leucine zipper domain sequence. As used herein, "leucine zipper domains" refer to naturally occurring or synthetic peptides that promote oligomerization of the proteins in which they are found. The leucine zipper is a super-secondary structure that functions as a dimerization domain, and its presence generates adhesion forces in parallel alpha helices. A single leucine zipper comprises multiple leucine residues at approximately 7-residue intervals, which forms an amphipathic alpha helix with a hydrophobic region running along one side. The dimer formed by a zipper domain is stabilized by the heptan repeat, designated (abcdefg). according to the notation of McLachlan and Stewart (J. Mol. Biol. 98:293; 1975), in which residues a and d are generally hydrophobic residues, with d being a leucine, which line up on the same face of a helix. Oppositely-charged residues commonly occur at positions g and e. Thus, in a parallel coiled coil formed from two helical zipper domains, the "knobs" formed by the hydrophobic side chains of the first helix are packed into the "holes" formed between the side chains of the second helix. The residues at position d (often leucine) contribute large hydrophobic stabilization energies, and are important for oligomer formation (Krystek et al., Int. J. Peptide Res. 38:229, 1991). This hydrophobic region provides an area for dimerization, allowing the motifs to "zip" together. Furthermore, the hydrophobic leucine region is absolutely required for DNA binding. Leucine zippers were originally identified in several DNA-binding proteins (Landschulz et al., Science 240:1759, 1988), and have since been found in a variety of different proteins. Among the known leucine zippers are naturally occurring peptides and derivatives thereof that dimerize or trimerize.

Examples of zipper domains are those found in the yeast transcription factor GCN4 and a heat-stable DNA-binding protein found in rat liver (C/EBP; Landschulz et al., Science 243:1681, 1989). The nuclear transforming proteins, fos and jun, also exhibit zipper domains, as does the gene product of the proto-oncogene, c-myc (Landschulz et al., Science 240:1759, 1988). The fusogenic proteins of several different viruses, including paramyxovirus, coronavirus, measles virus and many retroviruses, also possess zipper domains (Buckland and Wild, Nature 338:547,1989; Britton, Nature 353:394, 1991; Delwart and Mosialos, AIDS Research and Human Retrovirtises 6:703, 1990). The zipper domains in these fusogenic viral proteins are near the transmembrane region of the protein. Oligomerization of fusogenic viral proteins is involved in fusion pore formation (Spruce et al, Proc. Natl. Acad. Sci. U.S.A. 88:3523, 1991). Zipper domains have also been reported to play a role in oligomerization of heat-shock transcription factors (Rabindran et al., Science 259:230, 1993).

Examples of leucine zipper domains suitable for producing soluble TRAIL polypeptides include, but are not limited to, those described in PCT application WO 94/10308; U.S. Pat. No. 5,716,805; the leucine zipper derived from lung surfactant protein D (SPD) described in Hoppe et al., 1994, FEBS Letters 344:191; and Fanslow et al., 1994, Semin. Immunol. 6:267-278. In some embodiments, leucine residues in a leucine zipper domain are replaced by isoleucine residues. Such peptides comprising isoleucine can also be referred to as isoleucine zippers, but are encompassed by the term "leucine zippers" as used herein.

In some embodiments of the aspects described herein, the soluble TRAIL polypeptides comprise a linker sequence inserted between any heterologous sequence and the TRAIL domain in order to preserve function of either portion of the generated fusion protein. Such linker sequences known in the art include a linker domain having 7 amino acids (EASGGPE; SEQ ID NO: 3), or a linker domain having 18 amino acids (GSTGGSGKPGSGEGSTGG; SEQ ID NO: 4). As used herein, a "linker module" refers to a peptide, or a nucleotide sequence encoding such a peptide, of at least 8 amino acids in length. In some embodiments of the aspects described herein, the linker module comprises at least 9 amino acids, at least 10 amino acids, at least 11 amino acids, at least 12 amino acids, at least 13 amino acids, at least 14 amino acids, at least 15 amino acids, at least 16 amino acids, at least 17 amino acids, at least 18 amino acids, at least 19 amino acids, at least 20 amino acids, at least 21 amino acids, at least 22 amino acids, at least 23 amino acids, at least 24 amino acids, at least 25 amino acids, at least 30 amino acids, at least 35 amino acids, at least 40 amino acids, at least 45 amino acids, at least 50 amino acids, at least 55 amino acids, at least 56 amino acids, at least 60 amino acids, or least 65 amino acids. In some embodiments of the aspects described herein, a linker module comprises a peptide of 18 amino acids in length. In some embodiments of the aspects described herein, a linker module comprises a peptide of at least 8 amino acids in length but less than or equal to 56 amino acids in length, *i.e.,* the length of the spacer sequence in the native TRAIL molecule of SEQ ID NO: 1. In some embodiments, the linker molecule comprises the spacer sequence of human TRAIL, *i.e.,* amino acids 39-94 of SEQ ID NO: 1, or a sequence having at least 80%, at least 85%, at least 90%, at least 95%, at least 99% identity to amino acids 39-94 of SEQ ID NO: 1.

In some embodiments of the methods and compositions described herein, a soluble TRAIL polypeptide comprises a heterologous sequence encoding another therapeutic, biologically active protein, such as interferons, cytokines, chemokines, or DNA coding for a prodrug converting enzyme, such as thymidine kinase. Any desired heterologous sequence can be added, including DNA that encodes selectable markers, or preferably genes coding for a therapeutic, biologically active protein, such as interferons, cytokines, chemokines, or more preferably DNA coding for a prodrug converting enzyme, including thymidine kinase (Martuza et al., Science, 252:854, 1991), cytosine deamindase (U.S. Pat. No. 5,358,866), cyp450 (U.S. Pat. No. 5,688,773), and others. In some embodiments, the heterologous sequence encodes a protein that inhibits tumor growth (e.g., a chemotherapeutic, growth regulatory agent) or modifies an immune response. An example of a chemotherapeutic agent is mitomicin C. In some embodiments, the heterologous sequence encodes a growth regulatory molecule (e.g., one that has been lost in tumorigenesis of the tumor). Examples of such molecules without limitation proteins from the caspase family such as Caspase-9 (P55211(CASP9 HUMAN); HGNC: 15111; Entrez Gene: 8422; Ensembl: ENSG000001329067; OMIM: 6022345; UniProtKB: P552113), Caspase-8 (Q14790 (CASP8 HUMAN); 9606 [NCBI]), Caspase-7 (P55210 (CASP7_HUMAN); 9606 [NCBI]), and Caspase-3 (HCGN: 1504; Ensembl:ENSG00000164305; HPRD:02799; MIM:600636; Vega:OTTHUMG00000133681), pro-apoptotic proteins such as Bax (HGNC: 9591; Entrez Gene: 5812; Ensembl: ENSG000000870887; OMIM: 6000405; UniProtKB: Q078123), Bid (HGNC: 10501; Entrez Gene: 6372; Ensembl: ENSG000000154757; OMIM: 6019975; UniProtKB: P559573), Bad (HGNC: 9361; Entrez Gene: 5722; Ensembl: ENSG000000023307; OMIM: 6031675; UniProtKB: Q92934), Bak (HGNC: 9491; Entrez Gene: 5782; Ensembl: ENSG000000301107; OMIM: 6005165; UniProtKB: Q166113), BCL2L11 (HGNC: 9941; Entrez Gene: 100182; Ensembl: ENSG000001530947; OMIM: 6038275; UniProtKB: 0435213), p53 (HGNC: 119981; Entrez Gene: 71572; Ensembl: ENSG000001415107; OMIM: 1911705; UniProtKB: P046373), PUMA (HGNC: 178681; Entrez Gene: 271132; Ensembl: ENSG000001053277; OMIM: 6058545; UniProtKB: Q96PG83; UniProtKB: Q9BXH13), Diablo/SMAC (HGNC: 215281; Entrez Gene: 566162; Ensembl: ENSG000001840477; OMIM: 6052195; UniProtKB: Q9NR283). In one embodiment, the nucleic acid encoes an immunomodulatory agent (e.g, immunostimulatory transgenes), including, without limitation, Flt-3 ligand, HMBG1, calreticulin, **GITR** ligand, interleukin-12, interleukin-15, interleukin-18, or CCL17.

As demonstrated herein, soluble TRAIL polypeptides can be engineered comprising cDNA fusions encoding a binding agent specific for a tumor-associated cell-surface protein, such as a growth factor receptor, and a potent cytotoxic variant of tumor necrosis factor apoptosis inducing ligand (TRAIL). Such bimodal, fusion soluble TRAIL polypeptides can engage both a tumor-associated cell-surface protein and TRAIL death receptors simultaneously and can be more effective and have synergistic effects relative to combinatorial therapies targeting a tumor-associated cell-surface protein and a TRAIL death receptor individually.

Accordingly, in some embodiments of these methods and all such methods described herein, the sTRAIL polypeptide comprises a fusion with a tumor-associated cell-surface protein binding agent. In other words, in some embodiments of the methods described herein, a soluble TRAIL polypeptide comprises a heterologous sequence targeting or capable of binding, for example, a second cancer receptor not bound by TRAIL.

As used herein, the terms "tumor-associated cell-surface protein" or "cancer cell receptor" or "tumor/cancer antigen" refer to a receptor polypeptide (e.g. a polypeptide that binds specifically to a molecule in the extracellular environment) that is present on the surface of a cancer cell and/or differentially expressed by cancer cells and can thereby be exploited in order to target cancer cells. A tumor-associated cell-surface protein can be a receptor displayed exclusively on cancer cells, a receptor displayed at a higher level on cancer cells than normal cells of the same or different tissue types, or a receptor displayed on both cancerous and normal cell types. In some embodiments, a tumor-associated cell-surface protein can be a receptor that, in cancer cells, has altered (e.g. higher or lower than normal) expression and/or activity. In some embodiments, a tumor-associated cell-surface protein can be a receptor that is implicated in the disease process of cancer. In some embodiments, a tumor-associated cell-surface protein can be a receptor that is involved in the control of cell death and/or apoptosis. Some examples of cancer antigens include the cancer-testis (CT) antigens BAGE, GAGE, MAGE-1 and MAGE-3, NY-ESO-1, SSX. These antigens are found in melanoma, lymphoma, lung, bladder, colon, and breast carcinomas (e.g., as described in Butterfield et al., J. Immunotherapy 2008; 31:294-309; Markowicz et al., J Clin Oncol 27:15s, 2009 (suppl; abstr 9039)). Cancer antigens normally found in melanocytes, epithelial tissues, prostate, and colon also include the differentiation antigens Gp100, Melan-A/Mart-1, Tyrosinase, PSA, CEA, and Mammaglobin-A. These antigens are found in melanoma, prostate cancer, and in colon and breast carcinomas. Some cancer antigens are shared antigens that are ubiquitously expressed at low levels but overexpressed in cancers. Examples of overexpressed cancer antigens include p53, HER-2/neu, livin, and survivin, found in esophagus, liver, pancreas, colon, breast, ovary, bladder, and prostate carcinomas. Other cancer antigens are unique, such as 0-catenin-m,13-Actin/4/m, Myosin/m, HSP70-2/m, and HLA-A2-R170J, which are associated with one or more of melanoma, non-small cell lung cancer, and renal cancer. Still other cancer antigens are the tumor-associated carbohydrate antigens that are normally found in epithelia tissues such as renal, intestinal, and colorectal tissues. These cancer antigens include GM2, GD2, GD3, MUC-1, sTn, and globo-H, which can be found in melanoma, neuroblastoma, colorectal, lung, breast, ovarian, and prostate cancers. Additional tumor antigens, peptide epitopes, and descriptions thereof are described in U.S. Pat. Nos. 7,906,620; 7,910,692; 8,097,242; 7,935,531; 8,012,468; 8,097,256; 8,003,773; Tartour et al., Immunol Lett 2000; 74(1): 1-3. In some embodiments, the intact cancer antigen is used, whereas in other embodiments, a peptide epitope of the cancer antigen (prepared either by proteolytic digestion or recombinantly) is used.

Non-limiting examples of tumor-associated cell-surface proteins useful for the treatment of multifocal brain metastases include, for example, EGFR, ER, PR, HER2, PDGFR, VEGFR, MET, c-MET, ALK, CD117, RET, DR4, DRS, and FasR.

Accordingly, in some embodiments of the methods described herein, the tumor-associated cell-surface protein is a growth factor receptor, such as EGFR, IGF-R or NGF-R.

In some such embodiments, the tumor-associated cell-surface protein is EGFR (epidermal growth factor receptor). As used herein, the term "EGFR" or "Epidermal Growth Factor Receptor" refers to a transmembrane receptor that binds to ligands including epidermal growth factor "EGF" and TGFa. Ligand recognition causes autophosphorylation of EGFR and activates the MAPK, Akt, and/or INK pathways leading to cellular proliferation. EGFR (HER1 in humans) is the cell-surface receptor for members of the epidermal growth factor family (EGF-family) of extracellular protein ligands. The EGFR is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). Mutations affecting EGFR expression or activity can result in cancer. EGFR overexpression has been associated with a number of cancers, including lung cancer, anal cancers, and glioblastoma multiforme. These somatic mutations involving EGFR lead to its constant activation, which produces uncontrolled cell division. In glioblastoma, a more or less specific mutation of EGFR, called EGFRvIII is often observed. Mutations, amplifications or misregulations of EGFR or family members are implicated in about 30% of all epithelial cancers. The ligand EGF can be used as the targeting agent for directing cytotoxic therapeutics primarily and specifically to cancers that are overexpressing EGFR or EGFRvIII.The sequences of EGFR are well known in the art, e.g. human EGFR (NCBI Gene ID:1956) (SEQ ID NO: 5 (mRNA). The amino acid sequence of human EGF is set forth below:






















 (SEQ
ID NO: 6- polypeptide).

EGFR is frequently overexpressed in triple-negative breast cancers (TNBCs) ¹⁰ and involved in malignant tumor growth ¹¹, epithelial-mesenchymal transition (EMT) ¹², migration and invasion of tumor cells ¹³. EGFR amplification or EGFR activating mutations have been found in a significantly larger fraction of breast to brain metastatic tumor tissue compared with samples from primary tumors with good prognosis, bone relapse, or other distant metastases ¹⁴ suggesting that EGFR is a potential therapeutic target in brain metastatic breast cancer. Recently completed randomized phase II clinical trial utilizing EGFR monoclonal antibody, Erbitux, combined with a chemotherapeutic agent, cisplatin was shown to have a significantly improved overall response rate and progression free survival rate in metastatic (liver, lung, bone and lymph nodes) TNBC patients ¹⁵. Although, this study offers promise for targeting breast tumors that metastasize to different organs, the insufficient delivery of drugs across the blood-brain barrier (BBB), in addition to the non-leaky vasculature of metastatic lesions in the brain is a major challenge in treating metastatic breast tumors in the brain. In recent years, antibody-based anti-cancer therapies that involve smaller antibody fragments such as Fabs, ScFvs and camelid (e.g. llama) derived heavy chain-only antibodies, nanobodies (Nbs) have been emerging ¹⁶'¹⁷.

In some embodiments of the methods described herein, the tumor-associated cell surface marker binding agent is an antibody or antigen-binding fragment thereof. In some such embodiments, the antibody or antigen-binding fragment thereof is a nanobody.

As used herein, the term "antibody agent" refers to a polypeptide comprising a monoclonal antibody or antigen-binding domain of a monoclonal antibody that includes at least one immunoglobulin variable domain or immunoglobulin variable domain sequence and which specifically binds a given antigen. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody agent" encompasses antigen-binding fragments of antibodies *(e.g.,* single chain antibodies, Fab and sFab fragments, F(ab')₂, Fd fragments, Fv fragments, scFv, and single domain antibodies (dAb) (de Wildt et al., Eur J. Immunol. 1996; 26(3):629-39)) as well as complete antibodies. In particular embodiments, an antibody can have the structural features of IgA, IgG, IgE, IgD, IgM (as well as subtypes thereof). Antibodies can be from any source, including primate (human and non-human primate) and primatized antibodies.

The VH and VL regions can be further subdivided into regions of hypervariability, termed "complementarity determining regions" ("CDR"), interspersed with regions that are more conserved, termed "framework regions" ("FR"). The extent of the framework region and CDRs has been precisely defined (see, Kabat, E. A., et al. (1991) Sequences of Proteins of Immunological Interest, Fifth Edition, U.S. Department of Health and Human Services, NIH Publication No. 913242, and Chothia, C. et al. (1987) J. Mol. Biol. 196:901-917; Kabat definitions are used herein). Each VH and VL is typically composed of three CDRs and four FRs, arranged from amino-terminus to carboxyterminus in the following order: FR1, CDR1, FR2, CDR2, FR3, CDR3, and FR4.

One or more regions of an antibody can be human or effectively human. For example, one or more of the variable regions can be human or effectively human. For example, one or more of the CDRs can be human, e.g., HC (heavy chain) CDR1, HC CDR2, HC CDR3, LC (light chain) CDR1, LC CDR2, and LC CDR3. One or more of the framework regions can be human, e.g., FR1, FR2, FR3, and FR4 of the HC or LC. For example, at least 70, 75, 80, 85, 90, 91, 92, 93, 94, 95, 96, 97, 98, 99, or 100% of an immunoglobulin variable domain, the constant region, the constant domains (CH1, CH2, CH3, CL1), or the entire antibody can be human or effectively human. Fully human monoclonal antibodies also can be prepared by immunizing mice transgenic for large portions of human immunoglobulin heavy and light chain loci. Following immunization of these mice *(e.g.,* XENOMOUSETM (Abgenix), HUMAB-MOUSETm (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

The terms "antigen-binding fragment" and "antigen-binding domain" are used herein to refer to one or more fragments of a full length antibody that retain the ability to specifically bind to a target of interest. Examples of binding fragments encompassed within the term "antigen-binding fragment" of a full length antibody include (i) a Fab fragment, a monovalent fragment consisting of the VL, VH, CL and CH1 domains; (ii) a F(ab')₂ fragment, a bivalent fragment including two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment (Ward et al., (1989) Nature 341:544-546), which consists of a VH or VL domain; and (vi) an isolated complementarity determining region (CDR) that retains specific antigen-binding functionality. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See *e.g.,* U.S. Pat. Nos. 5,260,203, 4,946,778, and 4,881,175; Bird et al. (1988) Science 242:423-426; and Huston et al. (1988) Proc. Natl. Acad. Sci. USA 85:5879-5883.

Antibody fragments can be obtained using any appropriate technique including conventional techniques known to those of skill in the art. The term "monospecific antibody" refers to an antibody that displays a single binding specificity and affinity for a particular target, *e.g.,* epitope. This term includes a "monoclonal antibody" or "monoclonal antibody composition," which as used herein refer to a preparation of antibodies or fragments thereof of single molecular composition, irrespective of how the antibody was generated.

Antibodies and antibody agents can be raised against a polypeptide or portion of a polypeptide by methods known to those skilled in the art. Antibodies are readily raised in animals such as rabbits or mice by immunization with the gene product, or a fragment thereof *(e.g.,* PSA or PSMA). Immunized mice are particularly useful for providing sources of B cells for the manufacture of hybridomas, which in turn are cultured to produce large quantities of monoclonal antibodies. While both polyclonal and monoclonal antibodies can be used in the methods described herein, it is preferred that a monoclonal antibody is used where conditions require increased specificity for a particular protein.

Phage display can also be particularly effective in identifying antibody agents useful for the methods and assays described herein. For example, in order to identify aptamers for use in the methods described herein, briefly, one prepares a phage library (using *e.g.,* m13, fd, or lambda phage), displaying inserts from 4 to about 80 amino acid residues using conventional procedures. The inserts can represent, for example, a completely degenerate or biased array. One then can select phagebearing inserts which bind to a target polypeptide or fragments thereof. This process can be repeated through several cycles of reselection of phage that bind to the target molecule. Repeated rounds lead to enrichment of phage bearing particular sequences. DNA sequence analysis can be conducted to identify the sequences of the expressed polypeptides. The minimal linear portion of the sequence that binds to the target polypeptide can be determined. One can repeat the procedure using a biased library containing inserts containing part, or all, of the minimal linear portion plus one or more additional degenerate residues upstream or downstream thereof. Yeast two-hybrid screening methods also may be used to identify polypeptides that bind to the target polypeptide or fragment thereof. Thus, target polypeptides or fragments thereof can be used to screen peptide libraries, including phage display libraries, to identify and select peptide binding partners of the target polypeptide. Phage display can also be used to identify antibodies or antibody domains specific for a target, see, e.g. Barbas et al. PNAS 1991 88:7978-82; Burton et al. 1991 PNAS 88:10134-7; Yang et al. 1995 J Mol Biol 254:392-403; Barbas et al. PNAS 1994 91:3809-13; Barbas et al. PNAS 1992 89:4457-61.

In some embodiments, an antibody agent can be a nanobody agent. As used herein, the term "nanobody" refers to an antibody comprising the small single variable domain (WM) of antibodies obtained from camelids and dromedaries. Antibody proteins obtained from members of the camel and dromedary (Camelus baclrianus and Calelus dromaderius) family including new world members such as llama species (Lama paccos, Lama glama and Lama vicugna) have been characterized with respect to size, structural complexity and antigenicity for human subjects. Certain IgG antibodies from this family of mammals as found in nature lack light chains, and are thus structurally distinct from the typical four chain quaternary structure having two heavy and two light chains, for antibodies from other animals. See PCT/EP93/ 02214 (WO 94/04678 published 3 Mar. 1994).

A region of the camelid antibody which is the small single variable domain identified as VHEI can be obtained by genetic engineering to yield a small protein having high affinity for a target, resulting in a low molecular weight antibody-derived protein known as a "camelid nanobody". See U.S. Pat. No. 5,759,808 issued Jun. 2, 1998; see also Stijlemans, B. et al., 2004 J Biol Chem 279: 1256-1261; Dumoulin, M. et al., 2003 Nature 424: 783-788; Pleschberger, M. et al. 2003 Bioconjugate Chem 14: 440-448; Cortez-Retamozo, V. et al. 2002 Int J Cancer 89: 456-62; and Lauwereys, M. et al. 1998 EMBO J. 17: 3512-3520. Engineered libraries of camelid antibodies and antibody fragments are commercially available, for example, from Ablynx, Ghent, Belgium. As with other antibodies of non-human origin, an amino acid sequence of a camelid antibody can be altered recombinantly to obtain a sequence that more closely resembles a human sequence, i.e., the nanobody can be "humanized". Thus the natural low antigenicity of camelid antibodies to humans can be further reduced.

The camelid nanobody has a molecular weight approximately one-tenth that of a human IgG molecule and the protein has a physical diameter of only a few nanometers. One consequence of the small size is the ability of camelid nanobodies to bind to antigenic sites that are functionally invisible to larger antibody proteins, i.e., camelid nanobodies are useful as agents that detect antigens that are otherwise cryptic using classical immunological techniques, and as possible therapeutic agents. Thus yet another consequence of small size is that a camelid nanobody can inhibit as a result of binding to a specific site in a groove or narrow cleft of a target protein, and hence can serve in a capacity that more closely resembles the function of a classical low molecular weight drug than that of a classical antibody. The low molecular weight and compact size further result in camelid nanobodies being extremely thermostable, stable to extreme pH and to proteolytic digestion, and poorly antigenic. Another consequence is that camelid nanobodies readily move from the circulatory system into tissues, and even cross the blood-brain barrier and can treat disorders that affect nervous tissue. Nanobodies can further facilitate drug transport across the blood brain barrier. See U.S. patent application 20040161738 published Aug. 19, 2004. These features combined with the low antigenicity to humans indicate great therapeutic potential.

Accordingly, in some embodiments, the soluble TRAIL polypeptide comprising a heterologous sequence that binds to EGFR comprises an antibody agent. In some embodiments, the first portion of a soluble TRAIL polypeptide is an antibody agent which specifically binds to and inhibits EGFR and the second portion is a TRAIL domain and/or variant. In some embodiments, the first portion of a soluble TRAIL polypeptide is a nanobody agent which specifically binds to and inhibits EGFR and the second portion is a TRAIL domain and/or variant. In some embodiments, the first portion of a soluble TRAIL polypeptide is an antibody agent which specifically binds to and inhibits EGFR and the second portion comprises amino acids 114-281 of SEQ ID NO: 1. In some embodiments, the first portion of a soluble TRAIL polypeptide is a nanobody agent which specifically binds to and inhibits EGFR and the second portion comprises amino acids 114-281 of SEQ ID NO: 1.

Examples of antibody agents which bind to and inhibit EGFR are known in the art and commercially available, e.g. an inhibitory monoclonal antibody is available as Cat No. 05-101 from Millipore; Billerica, MA and anti-EGFR nanobodies have been previously described in the art, see e.g. Roovers RC, et al. (2007) Cancer Immunol Immunother 56:303-317 and Roovers RC, et al. Int J Cancer.

An exemplary nucleotide sequence encoding a nanobody agent that specifically binds to and inhibits EGFR is:

TCAGATCCGCTAGCATGACAGTGCTGGCGCCAGCCTGGAGCCCAACAACCTATNTCCTCCTGCTGCTGCTGCT
GAGCTCGGGACTCAGTGGGGAATTCCAGGTAAAGCTGGAGGAGTCTGGGGGAGGTCGGTGCAGACTGGGGG
CTCTCTGAGACTCACCTGTGCAGCCTCTGGACGCACTTCAAGGAGCTATGGCATGGGATGGTTCCGCCAGGCT
CCAGGGAAGGAGCGTGAGTTTGTATCAGGTATTAGTTGGAGGGGTGATAGTACAGGCTATGCAGACTCCGTG
AAGGGCCGATTCACCATCTCCAGAGACAACGCCAAGAACACGGTGGATCTGCAAATGAACAGCCTGAAACCT
GAGGACACGGCCATTTATTATTGTGCGGCGGCCGCAGGTAGTGCCTGGTACGGAACATTGTATGAATACGACT
ACTGGGGCCAGGGGACCCAGGTCACCGTCTCCTCAGGCGGTGGAGGCAGCGGTGGCGGGGGATCCGAGGTGC
AGCTGGTGGAGTCTGGGGGAGGATTGGTGCAGGCTGGGGGCTCTCTGAGACTCTCCTGTGCAGCCTCTGGACG
CACCTTCAGTAGCTATGCCATGGGCTGGTTCCGCCAGGCTCCAGGGAAGGAGCGTGAGTTTGTAGTAGCTATT
AACTGGAGTAGTGGTAGCACATACTATGCAGACTCCGTGAAGGGCCGATTCACCATCTCCAGAGACAACGCC
AAGAACACGATGTATCTGCAGATGAACAGCCTGAAACCTGAGGACACGGCCGTTTATTACTGTGCAGCAGGG

TATCAGATTAATAGTGGTAATTACAACTTTAAAGACTATGAGTATGACTACTGGGGCCAGGGGACCCAGGTCA
CCGTCTCCTCA (SEQ ID NO: 7)

In some embodiments, a portion of a soluble TRAIL polypeptide can specifically bind a receptor by virtue of being a ligand or ligand mimetic of the receptor. As used herein, the term "specific binding" refers to a chemical interaction between two molecules, compounds, cells and/or particles wherein the first entity binds to the second, target entity with greater specificity and affinity than it binds to a third entity which is a non-target. In some embodiments, specific binding can refer to an affinity of the first entity for the second target entity which is at least 10 times, at least 50 times, at least 100 times, at least 500 times, at least 1000 times or greater than the affinity for the third nontarget entity. In some embodiments, "selectively binds" or "specifically binds" can refer to the ability of a polypeptide domain described herein to bind to a target, such as a molecule present on the cell-surface, with a K_{D} of 10⁻⁵ M (10000 nM) or less, e.g., 10⁻⁶ M or less, 10⁻⁷ M or less, 10⁻⁸ M or less, 10⁻⁹ M or less, 10⁻¹⁰ M or less, 10⁻¹¹M or less, or 10⁻¹² M or less. Specific binding can be influenced by, for example, the affinity and avidity of the polypeptide agent and the concentration of polypeptide agent. The person of ordinary skill in the art can determine appropriate conditions under which the polypeptide agents described herein selectively bind the targets using any suitable methods, such as titration of a polypeptide agent in a suitable cell binding assay.

Also, as demonstrated herein, the incorporation of a pro-drug converting enzyme, such as herpes simplex virus thymidine kinase (HSV-TK), into therapeutic soluble TRAIL polypeptidesecreting stem cells allowed their eradication post-tumor treatment. Accordingly, in some embodiments of the methods described herein, a stem cell engineered to express a soluble TRAIL polypeptide comprises a heterologous sequence encoding a prodrug-converting enzyme, such as HSV thymidine kinase, carboxyl esterase. In such embodiments where the stem cell engineered to express soluble TRAIL polypeptide comprises a heterologous sequence encoding a prodrug converting enzyme, such as herpes simplex virus thymidine kinase, the method further comprises, after treatment for multifocal brain metastases has been effected or completed, administration of a prodrug, such as gancyclovir, which is converted to its active/cytotoxic form by the prodrug converting enzyme, thereby eradicating the stem cells encoding the soluble TRAIL polypeptide.

As used herein, a "prodrug" is a masked form of an active drug that has been designed to be activated after an enzymatic or chemical reaction once it has been administered into the body.

As used herein, a "prodrug modifying gene" or "prodrug modifying element" or gene encoding a "prodrug converting enzyme," or variations thereof refer to a gene that encodes a polypeptide that converts a prodrug to an active, e.g., cytotoxic compound. One non-limiting example of such a prodrug converting enzyme is herpes simplex 1 thymidine kinase gene (HSV-TK), which converts ganciclovir to a toxic nucleotide analog. Non-limiting, exemplary prodrug converting enzymes with their prodrug partners include, but are not limited to, herpes simplex virus thymidine kinase/gancyclovir, varicella zoster thymidine kinase/gancyclovir, cytosine deaminase/5-fluorouracil, purine nucleoside phosphorylase/6-methylpurine deoxyriboside, beta lactamase/cephalosporindoxorubicin, carboxypeptidase G2/4-[(2-chloroethyl)(2-mesuloxyethypaminolbenzoyl-L-glutamic acid, cytochrome P450/acetominophen, horseradish peroxidase/indole-3-acetic acid, nitroreductase/CB 1954, rabbit carboxylesterase/7-ethy1-10-[4-(1-piperidino)-1-piperidino] carbonyloxycam- potothecin, mushroom tyrosinase/bis-(2-chloroe thyl)amino-4-hydroxyphenylaminomethanone 28, beta galactosidase/1-chloromethyl-5-hydroxy-1,2-dihyro-3H-benz[e]indole, beta glucuronidase/epirubicinglucoronide, thymidine phosphorylase/5'-deoxy-5-fluorouridine, deoxycytidine kinase/cytosine arabinoside, beta-lactamase and linamerase/linamarin. Coding sequences for the various prodrug converting enzymes are known in the art.

### Constructs and Cellular Engineering

Gene therapy or transgene compositions are also contemplated for use with the methods described herein. Such methods allow clinicians to introduce a nucleic acid sequence encoding a soluble TRAIL polypeptide or component thereof of interest directly into a patient *(in vivo* gene therapy) or into cells isolated from a patient or a donor *(ex vivo* gene therapy). Therapeutic soluble TRAIL polypeptides produced by transduced cells after gene therapy can be maintained at a relatively constant level in, for example, the brain of a subject, as compared to a protein that is administered directly. Such sustained production of soluble TRAIL polypeptides is particularly appropriate in the treatment methods described herein. Expression can be transient (on the order of hours to weeks) or sustained (weeks to months or longer), depending upon the specific construct used and the target tissue or cell type. These transgenes can be introduced as a linear construct, a circular plasmid, or a viral vector, which can be an integrating or non-integrating vector. The transgene can also be constructed to permit it to be inherited as an extrachromosomal plasmid (Gassmann, et al., Proc. Natl. Acad. Sci. USA (1995) 92:1292).

Further, regulatable genetic constructs using small molecule inducers have been developed that can be included in vectors to be used in some embodiments of the aspects described herein. (Rivera et al. (1996) Nat. Med. 2:1028-32; No et al. (1996) Proc. Natl. Acad. Sci. USA, 93:3346-51; Gossen and Bujard (1992) Proc. Natl. Acad. Sci. USA 89:5547-51; the GeneSwitch^{®} system (Valentis, Inc., Burlingame, Calif.)). These systems are based on the use of engineered transcription factors, the activity of which is controlled by a small molecule drug, and a transgene, the expression of which is driven by the regulated transcription factor (Rivera et al. (1996) Nat. Med. 2:1028-32; Pollock et al. (2000) Proc. Natl. Acad. Sci. USA 97:13221-26; U.S. Pat. Nos. 6,043,082 and 6,649,595; Rivera et al. (1999) Proc. Natl. Acad. Sci. USA 96:8657-62).

In some of the aspects described herein, a nucleic acid sequence encoding a soluble TRAIL polypeptide, or any module or portion thereof, is operably linked to a vector. In general, as used herein, the term "vector" refers to any genetic element, such as a plasmid, phage, transposon, cosmid, chromosome, virus, virion, etc., that is capable of replication when associated with the proper control elements and that can transfer gene sequences to cells. Thus, the term includes cloning and expression vehicles, as well as viral vectors. By "recombinant vector" is meant a vector that includes a heterologous nucleic acid sequence, or "transgene" that is capable of expression *in vivo.* It should be understood that the vectors described herein can, in some embodiments, be combined with other suitable compositions and therapies. Vectors useful for the delivery of a sequence encoding soluble TRAIL polypeptides or a portion thereof can include one or more regulatory elements *(e.g*., promoter, enhancer, etc.) sufficient for expression of the soluble TRAIL polypeptides in the desired target cell or tissue. The regulatory elements can be chosen to provide either constitutive or regulated/inducible expression.

In some of the aspects described herein, oncolytic viruses are used to encode a soluble TRAIL polypeptide. Numerous oncolytic viruses are known in the art and are described, for example, in Kirn et al. (1999, In: Gene Therapy of Cancer, Academic Press, San Diego, Calif., pp. 235-248), any of which is envisioned for use herein. By way of example, appropriate oncolytic viruses include type 1 herpes simplex viruses, type 2 herpes simplex viruses, vesicular stomatitis viruses, oncolytic adenovirus (U.S. Patent No. 8,216,819), Newcastle disease viruses, vaccinia viruses, and mutant strains of these viruses. In some embodiments, the oncolytic virus is replication-selective or replication-competent. In some embodiments, the oncolytic virus is replication incompetent.

The oncolytic viruses useful in the methods described herein are, in some embodiments, replication-selective. It is understood that an oncolytic virus may be made replication-selective if replication of the virus is placed under the control of a regulator of gene expression such as, for example, the enhancer/promoter region derived from the 5'-flank of the albumin gene (e.g. see Miyatake et al., 1997, J. Virol. 71:5124-5132). By way of example, the main transcriptional unit of an HSV may be placed under transcriptional control of the tumor growth factor-beta (TGF-(3) promoter by operably linking HSV genes to the TGF-0 promoter. It is known that certain tumor cells overexpress TGF-0, relative to non-tumor cells of the same type. Thus, an oncolytic virus wherein replication is subject to transcriptional control of the TGF-0 promoter is replication-selective, in that it is more capable of replicating in the certain tumor cells than in non-tumor cells of the same type. Similar replication-selective oncolytic viruses may be made using any regulator of gene expression which is known to selectively cause overexpression in an affected cell. The replication-selective oncolytic virus may, for example, be an HSV-1 mutant in which a gene encoding ICP34.5 is mutated or deleted.

An oncolytic virus can further comprise other modifications in its genome. For example, it can comprise additional DNA inserted into the UL44 gene. This insertion can produce functional inactivation of the UL44 gene and the resulting lytic phenotype, or it may be inserted into an already inactivated gene, or substituted for a deleted gene.

The oncolytic virus can also have incorporated therein one or more promoters that impart to the virus an enhanced level of tumor cell specificity. In this way, the oncolytic virus may be targeted to specific tumor types using tumor cell-specific promoters. The term "tumor cell-specific promoter" or "tumor cell-specific transcriptional regulatory sequence" or "tumor-specific promoter" or "tumor-specific transcriptional regulatory sequence" indicates a transcriptional regulatory sequence, promoter and/or enhancer that is present at a higher level in the target tumor cell than in a normal cell. For example, the oncolytic virus may be under the control of an exogenously added regulator such as tetracycline (U.S. Patent No. 8,2366,941).

In some embodiments, the oncolytic virus (e.g., oHSV) vector is engineered to place at least one viral protein necessary for viral replication under the control of a tumor-specific promoter. Or, alternatively a gene (a viral gene or exogenous gene) that encodes a cytotoxic agent can be put under the control of a tumor-specific promoter. By cytotoxic agent as used here is meant any protein that causes cell death. For example, such would include ricin toxin, diphtheria toxin, or truncated versions thereof. Also, included would be genes that encode prodrugs, cytokines, or chemokines. Such viral vectors may utilize promoters from genes that are highly expressed in the targeted tumor such as the epidermal growth factor receptor promoter (EGFr) or the basic fibroblast growth factor (bFGF) promoter, or other tumor associated promoters or enhancer elements.

One exemplary oncolytic virus for use in the methods described herein is oncolytic herpes simplex virus (oHSV). The oHSV can comprise one or more exogenous nucleic acids encoding for one or more of the polypeptides described herein. Methods of generating an oHSV comprising such an exogenous nucleic acid are known in the art. The specific position of insertion of the nucleic acid into the oHSV genome can be determined by the skilled practitioner.

Oncolytic herpes simplex viruses (oHSV) are known in the art and are described, for example, in Kim et al. (1999, In: Gene Therapy of Cancer, Academic Press, San Diego, Calif, pp. 235-248), and include type 1 herpes simplex viruses and type 2 herpes simplex viruses. In one embodiment, the oHSV used in the methods, compositions, and kits disclosed herein is replication-selective or replication-competent such as one of the examples described herein. In some embodiments, the oHSV is replication-incompetent.

Herpes simplex 1 type viruses are among the preferred viruses, for example the variant of HSV-1 viruses that do not express functional ICP34.5, and thus exhibit significantly less neurotoxicity than their wild type counterparts. Such variants include without limitation oHSV-R3616, one of the HSV-1 viruses described in Coukos et al., Gene Ther. Mol. Biol. 3:79-89 (1998), and Varghese and Rabkin, Cancer Gene Therapy 9:967-978 (2002). Other exemplary HSV-1 viruses include 1716, R3616, and R4009. Other replication selective HSV-1 virus strains that can be used include, e.g., R474 (wherein genes encoding proteins ICP34.5 and ICP47 are deleted), G207 (genes encoding ICP34.5 and ribonucleotide reductase are deleted), NV1020 (genes encoding UL24, UL56 and the internal repeat are deleted), NV1023 (genes encoding UL24, UL56, ICP47 and the internal repeat are deleted), 3616-UB (genes encoding ICP34.5 and uracil DNA glycosylase are deleted), G92A (in which the albumin promoter drives transcription of the essential ICP4 gene), hrR3 (the gene encoding ribonucleotide reductase is deleted), and R7041 (Us3 gene is deleted) and HSV strains that do not express functional ICP34.5.

oHSV for use in the methods described herein is not limited to one of the HSV-1 mutant strains described herein. Any replication-selective strain of a herpes simplex virus may be used. In addition to the HSV-1 mutant strains described herein, other HSV-1 mutant strains that are replication selective have been described in the art. Furthermore, HSV-2, mutant strains such as, by way of example, HSV-2 strains 2701 (RL gene deleted), Delta RR (ICP10PK gene is deleted), and FusOn-H2 (ICP10 PK gene deleted) can also be used in the methods and compositions described herein.

Non-laboratory strains of HSV can also be isolated and adapted for use in the Methods described herein (U.S. Patent No. 7,063,835). Furthermore, HSV-2 mutant strains such as, by way of example, HSV-2 strains HSV-2701, HSV-2616, and HSV-2604 may be used in the methods described herein.

In some embodiments, the oHSV is G474. G474 is a third generation virus, which contains 1) a mutation of ICP6, which targets viral deletion to tumor cells, 2) a deletion of y34.5, which encodes ICP34.5 and blocks eIF2a phosphorylation and is the major viral determinant of neuropathogenicity, and 3) an additional deletion of the ICP47 gene and US11 promoter, so that the late gene US 11 is now expressed as an immediate-early gene and able to suppress the growth inhibited properties of y34.5 mutants. Deletion of ICP47 also abrogates HSV-1 inhibition of the transporter associated with antigen presentation and MHC class I downregulation (Todo et al., Proc. Natl. Acad. Sci. USA, 98:6396-6401(2001)).

A nucleic acid sequence encoding a soluble TRAIL polypeptide or portion thereof, can, in some embodiments, be delivered using non-viral, plasmid-based nucleic acid delivery systems, as described in U.S. Pat. Nos. 6,413,942, 6,214,804, 5,580,859, 5,589,466, 5,763,270 and 5,693,622. Such plasmids comprise the sequence encoding the soluble TRAIL polypeptide or portion thereof, operably linked to control elements that direct the expression of the soluble TRAIL polypeptide or portion thereof in a target cell, and are well known to those of ordinary skill in the art.

In some embodiments, plasmid vectors comprising nucleic acid sequence(s) encoding a soluble TRAIL polypeptide or portion thereof can be packaged in liposomes prior to delivery to a subject or to cells, as described in U.S. Pat. Nos. 5,580,859, 5,549,127, 5,264,618, 5,703,055. For a review of the use of liposomes as carriers for delivery of nucleic acids, see, Hug and Sleight (1991) Biochim. Biophys. Acta. 1097:1-17; Straubinger et al. (1983) in Methods of Enzymology Vol. 101, pp. 512-27; de Lima et al. (2003) Current Medicinal Chemistry, Volume 10(14): 1221-31. The DNA can also be delivered in cochleate lipid compositions similar to those described by Papahadjopoulos et al. (1975) Biochem. Biophys. Acta. 394:483-491. See also U.S. Pat. Nos. 4,663,161 and 4,871,488.

Biolistic delivery systems employing particulate carriers such as gold and tungsten can also be used to deliver nucleic acid sequence encoding a soluble TRAIL polypeptide or portion thereof. See, *e.g.,* U.S. Pat. Nos. 4,945,050, 5,036,006, 5,100,792, 5,179,022, 5,371,015, and 5,478,744.

A wide variety of methods can be used to deliver the vectors comprising nucleic acid sequence(s) encoding a soluble TRAIL polypeptide or portion thereof Such methods include DEAE dextran-mediated transfection, calcium phosphate precipitation, polylysine- or polyornithine-mediated transfection, or precipitation using other insoluble inorganic salts, such as strontium phosphate, aluminum silicates including bentonite and kaolin, chromic oxide, magnesium silicate, talc, and the like. Other useful methods of transfection include electroporation, sonoporation, protoplast fusion, peptoid delivery, or microinjection. See, *e.g*., Sambrook et al (1989) Molecular Cloning, A Laboratory Manual, Cold Spring Harbor Laboratories, New York, for a discussion of techniques for transforming cells of interest; and Felgner, P. L. (1990) Advanced Drug Delivery Reviews 5:163-87, for a review of delivery systems useful for gene transfer. Exemplary methods of delivering DNA using electroporation are described in U.S. Pat. Nos. 6,132,419; 6,451,002, 6,418,341, 6,233,483, U.S. Patent Publication No. 2002/0146831, and International Publication No. WO/0045823.

In other embodiments of the aspects described herein, plasmid vectors comprising nucleic acid sequence(s) encoding a soluble TRAIL polypeptide or portion thereof can also be introduced directly into the CNS by intrathecal (IT) injection, as described herein in greater detail with regard to protein administration. Plasmid DNA can be complexed with cationic agents such as polyethyleneimine (PEI) or Lipofectamine 2000 to facilitate uptake.

Retroviruses, such as lentiviruses, provide another platform for delivery of nucleic acid sequences encoding a soluble TRAIL polypeptide or portion thereof of interest. A selected nucleic acid sequence can be inserted into a vector and packaged in retroviral particles using techniques known in the art. These retroviral vectors contain the components necessary for the correct packaging of the viral genome and integration into the host cell DNA. The recombinant virus can then be isolated and delivered to cells of the subject either *in vivo* or *ex vivo.* The nucleic acid sequences encoding a soluble TRAIL polypeptide or portion thereof are cloned into one or more vectors, which facilitates delivery of the nucleic acid into a patient. Retroviral systems are described in, for example, U.S. Pat. No. 5,219,740; Miller and Rosman (1989) BioTechniques 7:980-90; Miller, A. D. (1990) Human Gene Therapy 1:5-14; Scarpa et al. (1991) Virology 180:849-52; Miller et al., Meth. Enzymol. 217:581-599 (1993); Burns et al. (1993) Proc. Natl. Acad. Sci. USA 90:8033-37; Boris-Lawrie and Temin (1993) Curr. Opin. Genet. Develop. 3:102-09. Greater detail about retroviral vectors can be found, for example, in Boesen et al., Biotherapy 6:291-302 (1994), which describes the use of a retroviral vector to deliver the *mdrl* gene to hematopoietic stem cells in order to make the stem cells more resistant to chemotherapy. Other references illustrating the use of retroviral vectors in gene therapy include: Clowes et al., J. Clin. Invest. 93:644-651 (1994); Kiem et al., Blood 83:1467-1473 (1994); Salmons and Gunzberg, Human Gene Therapy 4:129-141 (1993); and Grossman and Wilson, Curr. Opin. in Genetics and Devel. 3:110-114 (1993.

In some embodiments of the aspects described herein, a lentiviral system is used to deliver a nucleic acid sequence encoding a soluble TRAIL polypeptide or portion thereof. Lentiviral vectors contemplated for use include, for example, the HIV based vectors described in U.S. Patent Nos. 6,143,520; 5,665,557; and 5,981,276.

In some embodiments, a nucleotide sequence encoding a soluble TRAIL polypeptide or portion thereof is inserted into an adenovirus-based expression vector. Unlike retroviruses, which integrate into the host genome, adenoviruses persist extrachromosomally thus minimizing the risks associated with insertional mutagenesis (Haj-Ahmad and Graham (1986) J. Virol. 57:267-74; Bett et al. (1993) J. Virol. 67:5911-21; Mittereder et al. (1994) Human Gene Therapy 5:717-29; Seth et al. (1994) J. Virol. 68:933-40; Barr et al. (1994) Gene Therapy 1:51-58; Berkner, K. L. (1988) BioTechniques 6:616-29; and Rich et al. (1993) Human Gene Therapy 4:461-76). Adenoviral vectors have several advantages in gene therapy. They infect a wide variety of cells, have a broad host-range, exhibit high efficiencies of infectivity, direct expression of heterologous sequences at high levels, and achieve long-term expression of those sequences *in vivo.* The virus is fully infective as a cell-free virion so injection of producer cell lines is not necessary. With regard to safety, adenovirus is not associated with severe human pathology, and the recombinant vectors derived from the virus can be rendered replication defective by deletions in the early-region 1 ("El") of the viral genome. Adenovirus can also be produced in large quantities with relative ease. For all these reasons vectors derived from human adenoviruses, in which at least the El region has been deleted and replaced by a gene of interest, have been used extensively for gene therapy experiments in the pre-clinical and clinical phase.

Adenoviral vectors for use with the methods described herein can be derived from any of the various adenoviral serotypes, including, without limitation, any of the over 40 serotype strains of adenovirus, such as serotypes 2, 5, 12, 40, and 41. The adenoviral vectors used herein are replication-deficient and contain the sequence of interest under the control of a suitable promoter, such as any of the promoters discussed below with reference to adeno-associated virus. For example, U.S. Pat. No. 6,048,551, describes replication-deficient adenoviral vectors that include a human gene under the control of the Rous Sarcoma Virus (RSV) promoter. Other recombinant adenoviruses of various serotypes, and comprising different promoter systems, can be created by those skilled in the art. See, e.g., U.S. Pat. No. 6,306,652.

Other useful adenovirus-based vectors for delivery of nucleic acid sequence encoding a soluble TRAIL polypeptide or portion thereof include, but are not limited to: "minimal" adenovirus vectors as described in U.S. Pat. No. 6,306,652, which retain at least a portion of the viral genome required for encapsidation (the encapsidation signal), as well as at least one copy of at least a functional part or a derivative of the ITR; and the "gutless" (helper-dependent) adenovirus in which the vast majority of the viral genome has been removed and which produce essentially no viral proteins, thus allowing gene therapy to persist for over a year after a single administration (Wu et al. (2001) Anesthes. 94:1119-32; Parks (2000) Clin. Genet. 58:1-11; Tsai et al. (2000) Curr. Opin. Mol. Ther. 2:515-23).

In some embodiments of the compositions and methods described herein, a nucleotide sequence encoding a soluble TRAIL polypeptide or portion thereof is inserted into an adeno-associated virus-based expression vector. AAV is a parvovirus which belongs to the genus *Dependovirus* and has several features not found in other viruses. AAV can infect a wide range of host cells, including non-dividing cells. AAV can infect cells from different species. AAV has not been associated with any human or animal disease and does not appear to alter the biological properties of the host cell upon integration. Indeed, it is estimated that 80-85% of the human population has been exposed to the virus. Finally, AAV is stable at a wide range of physical and chemical conditions, facilitating production, storage and transportation.

AAV is a helper-dependent virus; that is, it requires co-infection with a helper virus *(e.g*., adenovirus, herpesvirus or vaccinia) in order to form AAV virions in the wild. In the absence of co-infection with a helper virus, AAV establishes a latent state in which the viral genome inserts into a host cell chromosome, but infectious virions are not produced. Subsequent infection by a helper virus rescues the integrated genome, allowing it to replicate and package its genome into infectious AAV virions. While AAV can infect cells from different species, the helper virus must be of the same species as the host cell. Thus, for example, human AAV will replicate in canine cells co-infected with a canine adenovirus.

Adeno-associated virus (AAV) has been used with success in gene therapy. AAV has been engineered to deliver genes of interest by deleting the internal nonrepeating portion of the AAV genome *(i.e.,* the rep and cap genes) and inserting a heterologous sequence (in this case, the sequence encoding the multimodal TRAIL agent) between the ITRs. The heterologous sequence is typically functionally linked to a heterologous promoter (constitutive, cell-specific, or inducible) capable of driving expression in the patient's target cells under appropriate conditions.

Recombinant AAV virions comprising a nucleic acid sequence encoding a multimodal TRAIL agent of interest can be produced using a variety of art-recognized techniques, as described in U.S. Pat. Nos. 5,139,941; 5,622,856; 5,139,941; 6,001,650; and 6,004,797. Vectors and cell lines necessary for preparing helper virus-free rAAV stocks are commercially available as the AAV Helper-Free System (Catalog No. 240071) (Stratagene, La Jolla, Calif.).

Additional viral vectors useful for delivering nucleic acid molecules encoding a soluble TRAIL polypeptide or portion thereof of interest include those derived from the pox family of viruses, including vaccinia virus and avian poxvirus. Alternatively, avipoxviruses, such as the fowlpox and canarypox viruses, can be used to deliver the genes. The use of avipox vectors in human and other mammalian species is advantageous with regard to safety because members of the avipox genus can only productively replicate in susceptible avian species. Methods for producing recombinant avipoxviruses are known in the art and employ genetic recombination, see, *e.g.,* WO 91/12882; WO 89/03429; and WO 92/03545.

Molecular conjugate vectors, such as the adenovirus chimeric vectors, can also be used for delivery of sequence encoding soluble TRAIL polypeptide or portion thereof (Michael et al. (1993) J. Biol. Chem. 268:6866-69 and Wagner et al. (1992) Proc. Natl. Acad. Sci. USA 89:6099-6103). Members of the *Alphavirus* genus, for example the Sindbis and Semliki Forest viruses, can also be used as viral vectors for delivering a nucleic acid sequence encoding a soluble TRAIL polypeptide or portion thereof of interest (See, *e.g.,* Dubensky et al. (1996) J. Virol. 70:508-19; WO 95/07995; WO 96/17072).

Essentially any cell type can be engineered with a sequence encoding the soluble TRAIL polypeptide or portion thereof, including soluble TRAIL fusion polypeptides, as described herein, for use in cellular therapies. Thus, differentiated somatic cells and stem cells, as well as cells of a cell line, can be engineered to express, using any method known to one of skill in the art, a desired soluble TRAIL polypeptide or portion thereof, including soluble TRAIL fusion polypeptides. In some embodiments of the aspects described herein, a cell can be transduced with a delivery vector comprising a nucleic acid sequence encoding a soluble TRAIL polypeptide or portion thereof, including a soluble TRAIL fusion polypeptide. In other embodiments of the compositions and methods described herein, a cell can be transfected with a nucleic acid sequence encoding a soluble TRAIL polypeptide or portion thereof, including a soluble TRAIL fusion polypeptide. Provided herein are exemplary stem cells, somatic cells, and cell line sources useful with the methods and compositions described herein. However, the description herein is not meant to be limiting and any cell known or used in the art can be genetically modified or engineered to express and secrete a soluble TRAIL polypeptide or portion thereof, including a soluble TRAIL fusion polypeptide. In some embodiments, the cells to be engineered can be from an autologous, *i.e.,* from the same subject, or from one or more heterologous sources.

In some embodiments of the methods described herein, the cell type that is engineered to express a soluble TRAIL polypeptide or portion thereof is a stem cell. Stem cells are undifferentiated cells defined by their ability at the single cell level to both self-renew and differentiate to produce progeny cells, including self-renewing progenitors, non-renewing progenitors, and terminally differentiated cells. Some stem cells, depending on their level of differentiation, are also characterized by their ability to differentiate *in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts. (See, *e.g.,* Potten et al., Development 110: 1001 (1990); U.S. Pat. Nos. 5,750,376, 5,851,832, 5,753,506, 5,589,376, 5,824,489, 5,654,183, 5,693,482, 5,672,499, and 5,849,553).

The stem cells for use with the compositions and methods comprising multimodal TRAIL agents described herein can be naturally occurring stem cells or "induced" stem cells, such as "induced pluripotent stem cells" (iPS cells) generated using any method or composition known to one of skill in the art. Stem cells can be obtained or generated from any mammalian species, *e.g*. human, primate, equine, bovine, porcine, canine, feline, rodent, *e.g*. mice, rats, hamster, etc. In some embodiments of the aspects described herein, a stem cell is a human stem cell.

Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages, but all within a particular tissue, organ, or physiological system (for example, hematopoietic stem cells (HSC) can produce progeny that include HSC (self-renewal), blood cell restricted oligopotent progenitors and the cell types and elements *(e.g*., platelets) that are normal components of the blood); (4) oligopotent, meaning able to give rise to a more restricted subset of cell lineages than multipotent stem cells; and (5) unipotent, meaning able to give rise to a single cell lineage *(e.g*., spermatogenic stem cells).

Stem cells of interest for use in the compositions and methods described herein include embryonic cells of various types, exemplified by human embryonic stem (hES) cells, for reference only described by Thomson et al. (1998) Science 282:1145; embryonic stem cells from other primates, such as Rhesus stem cells (Thomson et al. (1995) Proc. Natl. Acad. Sci USA 92:7844); marmoset stem cells (Thomson et al. (1996) Biol. Reprod. 55:254); and human embryonic germ (hEG) cells (Shambloft et al., Proc. Natl. Acad. Sci. USA 95:13726, 1998). Cells derived from embryonic sources can include embryonic stem cells or stem cell lines obtained from a stem cell bank or other recognized depository institution.

In some embodiments of the aspects described herein, a cell engineered to express or secrete a soluble TRAIL polypeptide or portion thereof is an adult or somatic stem cell. Adult stem cells are generally limited to differentiating into different cell types of their tissue of origin. However, for reference only, if the starting stem cells are derived from the inner cell mass of the embryo, they can generate many cell types of the body derived from all three embryonic cell types: endoderm, mesoderm and ectoderm. Stem cells with this property are said to be "pluripotent." Embryonic stem cells are one kind of pluripotent stem cell. Thus, pluripotent embryonic stem cells can be differentiated into many specific cell types. For reference only, since the embryo is a potential source of all types of precursor cells, it is possible to differentiate, for example, engineered embryonic stem cells into other lineages by providing the appropriate signals, such as the expression of proteins, using any method known to one of skill in the art, to embryonic stem cells.

Somatic or adult stem cells have major advantages, for example, as using somatic stem cells allows a patient's own cells to be expanded in culture and then re-introduced into the patient. Natural somatic stem cells have been isolated from a wide variety of adult tissues including blood, bone marrow, brain, olfactory epithelium, skin, pancreas, skeletal muscle, and cardiac muscle. Each of these somatic stem cells can be characterized based on gene expression, factor responsiveness, and morphology in culture. Exemplary naturally occurring somatic stem cells include, but are not limited to, neural stem cells, neural crest stem cells, mesenchymal stem cells, hematopoietic stem cells, and pancreatic stem cells. In addition, iPS cells generated from a patient provide a source of cells that can be engineered to express a soluble TRAIL polypeptide or portion thereof, expanded, and reintroduced to the patient, before or after stimulation to differentiate to a desired lineage or phenotype, such as a neural or neuronal stem cell.

In some embodiments of the aspects described herein, a somatic stem cell engineered to express a soluble TRAIL polypeptide or portion thereof is a neuronal or neural stem cell. In some embodiments of the aspects described herein, a somatic stem cell engineered to express a soluble TRAIL polypeptide or portion thereof is a mesenchymal stem cell. In some embodiments of the aspects described herein, a somatic stem cell engineered to express a soluble TRAIL polypeptide or portion thereof is an iPS cell differentiated into a neuronal stem cell. In some embodiments of the aspects described herein, a somatic stem cell engineered to express a soluble TRAIL polypeptide or portion thereof is an iPS cell differentiated into a mesenchymal stem cell.

Cord blood cells are used as a source of transplantable stem and progenitor cells and as a source of marrow repopulating cells for the treatment of malignant diseases *(e.g*., acute lymphoid leukemia, acute myeloid leukemia, chronic myeloid leukemia, myelodysplastic syndrome, and neuroblastoma) and non-malignant diseases such as Fanconi's anemia and aplastic anemia (Kohli-Kumar et al., 1993 Br. J. Haematol. 85:419-422; Wagner et al., 1992 Blood 79;1874-1881; Lu et al., 1996 Crit. Rev. Oncol. Hematol 22:61-78; Lu et al., 1995 Cell Transplantation 4:493-503). Accordingly, in some aspects, cells to be engineered to secrete or express a multimodal TRAIL agent can also be derived from human umbilical cord blood cells (HUCBC), which are recognized as a rich source of hematopoietic and mesenchymal stem cells (Broxmeyer et al., 1992 Proc. Natl. Acad. Sci. USA 89:4109-4113). One advantage of HUCBC for use with the methods and compositions described herein is the immature immunity of these cells, which is very similar to fetal cells, and thus significantly reduces the risk for rejection by the host (Taylor & Bryson, 1985 J. Immunol. 134:1493-1497).

In some embodiments of the aspects described herein, iPS cells are engineered to express or secrete the soluble TRAIL polypeptide or portion thereof described herein. In some embodiments of the aspects described herein, iPS cells are engineered to express or secrete the soluble TRAIL polypeptide or portion thereof prior to being differentiated into another desired cell type. In some embodiments of the aspects described herein, iPS cells are engineered to express or secrete the soluble TRAIL polypeptide or portion thereof after differentiation into another desired cell type. This can be accomplished, for example, by placing the soluble TRAIL polypeptide expressing construct under control of a promoter only active in the more differentiated (stem) cell phenotype.

Studies have shown that "neural stem cells" or "neuronal stem cells" (NSCs) or neural precursor cells injected into the parenchyma of the brain or intracranially migrate towards injured or pathological central nervous system (CNS) sites. This chemotropic property of NSCs has been utilized for cell-based therapies to treat diverse neurological diseases as described herein and in T. Bagci-Onder et al., Cancer Research 2011, 71:154-163; Hingtgen S. et al., Stem Cells 2010, 28(4):832-41; Hingtgen S. et al., Mol Cancer Ther. 2008, 7(11): 3575-85; Brustle 0. et al., 6 Current Opinion in Neurobiology. 688 (1996); Flax J. D., et al., 16 Nature Biotechnology. 1033. (1998); Kim S.U., 24. Neuropathology. 159 (2004); Lindvall 0 et al., 10 (suppl) Nature Medicine. S42 (2004); Goldman S., 7. Nature Biotechnology. 862 (2005); Muller F. et al., 7 Nature Reviews Neuroscience. 75 (2006); Lee, J. P., et al. 13 Nature Medicine 439 (2007), and Kim S. U. et al., 87 Journal of Neuroscience Research 2183 (2009.

It is shown herein that systemic or intra-arterial administration of engineered neuronal stem cells permits the cells to track to multifocal metastatic lesions for delivery of therapeutic sTRAIL polypeptides. The stem cells cross the blood-brain barrier and become established at multifocal tumor sites and inhibit tumor growth and viability.

Accordingly, in some embodiments of the compositions and methods described herein, a pharmaceutically acceptable composition comprising a neural stem cell and a soluble TRAIL polypeptide or portion thereof can be administered to a subject. In some such embodiments, the neural stem cell is genetically engineered to express or secrete a soluble TRAIL polypeptide or portion thereof. Because NSCs can be engineered to package and release replication-defective retroviral particles or replication-conditional herpes virus vectors which, in turn, can serve as vectors for the transfer of sequences to CNS cells, neural progenitor/stem cells can serve to magnify the efficacy of viral-mediated gene delivery to large regions in the brain. Additional vectors that can be used in the embodiments described herein include herpes simplex virus vectors, SV 40 vectors, polyoma virus vectors, papilloma virus vectors, picomovirus vectors, vaccinia virus vectors, and a helper-dependent or gutless adenovirus. In one embodiment, the vector can be a lentivirus. Methods for preparing genetically engineered neural stem cells and compositions thereof for therapeutic treatment have been described in U.S. Patent Nos.: U.S.7393526 and U.S.7655224.

In various embodiments of the compositions and methods described herein, the neural stem cells that can be used include, but are not limited to, human neural stem cells, mouse neural stem cells HSN-1 cells, fetal pig cells and neural crest cells, bone marrow derived neural stem cells, and hNT cells. HSN-1 cells can be prepared, for example, as described in, *e.g.,* Ronnett et al. (Science 248, 603-605, 1990). The preparation of neural crest cells is described in U.S. Pat. No. 5,654,183. hNT cells can be prepared as described in, e.g, Konubu et al. (Cell Transplant 7, 549-558, 1998). In some embodiments of the compositions and methods described herein, the neural stem cells that can be used are neural stem cells derived or differentiated from a precursor stem cell, such as a human embryonic stem cell or an induced pluripotent stem (iPS) cell. Such neural stem cells can be generated from or differentiated from human embryonic stem cells, using, for example, compositions and methods described in Nature Biotechnology 27, 275 - 280 (2009), "Highly efficient neural conversion of human ES and iPS cells by dual inhibition of SMAD signaling,". Such neural stem cells can be generated from or differentiated from iPS cells, using, for example, the compositions and methods described in US Patent Publication US 2010/0021437 A1, "NEURAL STEM CELLS DERIVED FROM INDUCED PLURIPOTENT STEM CELLS,".

Accordingly, as used herein, "neural stem cells" or "neuronal stem cells" refer to a subset of multipotent cells which have partially differentiated along a neural cell pathway and express some neural markers including, for example, nestin. Neural stem cells can differentiate into neurons or glial cells (e.g., astrocytes and oligodendrocytes). Thus, "neural stem cells derived or differentiated from iPS cells" refers to cells that are multipotent but have partially differentiated along a neural cell pathway (i.e., express some neural cell markers), and themselves are the result of *in vitro* or *in vivo* differentiation iPS cells.

Neural selection factors that can be used to differentiate pluripotent stem cells, such as embryonic stem cells or iPS cells into neural stem cells, include, for example, sonic hedgehog (SHH), fibroblast growth factor-2 (FGF-2), and fibroblast growth factor-8 (FGF-8), which can be used alone or in pairwise combination, or all three factors may be used together. In some embodiments, iPS cells are cultured in the presence of at least SHH and FGF-8. In other embodiments, FGF-2 is omitted. Preferably, the neural stem cells derived from iPS cells express nestin. In some embodiments, the pluripotent stem cells are cultured in the presence of the one or more neural selection factors for 4, 5, 6, 7, 8, 9, 10, 12, 14, 16, 18, 20 days or more. Preferably, the population of neural stem cells is characterized in that at least 50%, at least 75%, at least 85%, at least 90%, at least 95%, or at least 99% of the cells of the population express nestin. Preferably, the nestin-expressing cells further express at least one of En-1, Pitx3, and Nurr-1. In other embodiments, the population of neural stem cells has been depleted of at least 50%, 75%, 85%, 95%, or 99% of the cells expressing surface markers of immature embryonic stem cells including, for example, SSEA-1, SSEA-3, SSEA-4, Tra-1-81, and Tra-1-60. Preferably, the population of neural stem cells contains less than 10%, less than 5%, less than 2.5%, less than 1%, or less than 0.1% of cells that express the selected marker (e.g., SSEA-4).

In some embodiments, the cells engineered to express or secrete the soluble TRAIL polypeptides or portions thereof described herein described herein comprise cells of a cell line.

Various aspects and embodiments of the methods described herein involve administration of an effective amount of a cell or a population of cells, engineered to express soluble TRAIL polypeptides or portions thereof as described herein, to an individual or subject in need of a cellular therapy. The cell or population of cells being administered can be an autologous population, or be derived from one or more heterologous sources. The cell can be, for example, a stem cell, such as a lineage-restricted progenitor cell, multipotent cell, or an oligopotent cell, or a fully or partially differentiated progeny of a stem cell.

A variety of approaches for administering cells to subjects are known to those of skill in the art. An advantage provided by the use of neural stem cells to deliver therapeutic proteins is that the cells need not be administered directly to the brain- that is, intracranial administration and administration directly to the parenchyma of the brain are not required. The ability to administer systemically, e.g., by intraarterial injection, permits a less invasive approach and facilitates delivery throughout the brain, rather than at just one or several focal points of injection to the brain parenchyma. This approach permits delivery of therapeutic cells via the brain's circulation is much the same way that the multifocal metastatic tumor cells arrived in the brain. Such methods can include systemic injection, for example, injection directly into the carotid artery. In some embodiments of the methods described herein, administration does not include or comprise implantation of cells directly into a tumor target site in a subject, such as a surgical site. Cells can be inserted into a delivery device
which facilitates introduction by injection or implantation into the subject. Such delivery devices can include tubes, e.g., catheters, for injecting cells and fluids into the body of a recipient subject. In some embodiments, the tubes additionally have a needle, e.g., through which the cells can be introduced into the subject at a desired location. The cells can be prepared for delivery in a variety of different forms. Cells can be mixed with a pharmaceutically acceptable carrier or diluent in which the cells remain viable.

Pharmaceutically acceptable carriers and diluents include saline, aqueous buffer solutions, solvents and/or dispersion media. The use of such carriers and diluents is well known in the art. The solution is preferably sterile and fluid. Preferably, prior to the introduction of cells as described herein, the solution is stable under the conditions of manufacture and storage and preserved against the contaminating action of microorganisms such as bacteria and fungi through the use of agents such as parabens, chlorobutanol, phenol, ascorbic acid, thimerosal, and the like.

Direct injection techniques for cell administration can also be used to stimulate transmigration of cells through the entire vasculature, or to a particular organ, such as for example the brain. This includes non-specific targeting of the vasculature. One can target any organ by selecting a specific injection site, *e.g.,* the carotid artery for targeting the brain. Accordingly, in some embodiments of the methods described herein, stem cells, such as neural stem cells, encoding a soluble TRAIL polypeptide are administered via direct injection into the carotid artery.

Alternatively, the injection can be performed systemically into any vessel in the body. Thus, in some embodiments of the methods described herein, stem cells, such as neural stem cells, encoding a soluble TRAIL polypeptide are administered systemically. In some embodiments of the methods described herein, stem cells, such as neural stem cells, encoding a soluble TRAIL polypeptide are not administered intravenously.

It is further contemplated that, in some embodiments of these aspects, cells engineered to express the soluble TRAIL polypeptides described herein, can not only be administered to a subject in need as cells in suspension, but also as cells populating a matrix, scaffold, or other support, to enhance retention of cells and delivery of the soluble TRAIL polypeptides at a site. Encapsulation of stem cells has shown to permit enhanced delivery of engineered stem cells, as described in, for example, Compte M. et al., Stem Cells 2009, 27(3):753-760.

In some embodiments, a "support" refers to any suitable carrier material to which cells, such as engineered neural stem cells expressing a soluble TRAIL polypeptide described herein, are able to attach themselves or adhere, and can be used in order to form a corresponding cell composite, e.g. an artificial tissue. In some embodiments, a matrix or carrier material, respectively, is present already in a three-dimensional form desired for later application.

In some such embodiments, a matrix or a scaffold comprises a "biocompatible substrate" that can be used as a material that is suitable for implantation into a subject onto which a cell population can be deposited. A biocompatible substrate does not cause toxic or injurious effects once implanted in the subject. The biocompatible substrate can provide the supportive framework that allows cells to attach to it, and grow on it. Cultured populations of cells can then be grown on the biocompatible substrate, which provides the appropriate interstitial distances required for cell-cell interaction.

A matrix, structure, or scaffold can be used to aid in further controlling and directing a cell or population of cells expressing or secreting a soluble TRAIL polypeptide described herein. A matrix or scaffold can be designed or selected to provide environmental cues to control and direct the migration of cells to a site of injury or disease. A structure or scaffold can be engineered from a nanometer to micrometer to millimeter to macroscopic length, and can further comprise or be based on factors such as, but not limited to, material mechanical properties, material solubility, spatial patterning of bioactive compounds, spatial patterning of topological features, soluble bioactive compounds, mechanical perturbation (cyclical or static strain, stress, shear, etc...), electrical stimulation, and thermal perturbation.

A scaffold can be in any desired geometric conformation, for example, a flat sheet, a spiral, a cone, a v-like structure and the like. A scaffold can be shaped into, *e.g.,* a heart valve, vessel (tubular), planar construct or any other suitable shape. Such scaffold constructs are known in the art (see, *e.g*., WO02/035992, U.S. Pat. Nos. 6,479,064, 6,461,628).

Biopolymer structures can be generated by providing a transitional polymer on a substrate; depositing a biopolymer on the transitional polymer; shaping the biopolymer into a structure having a selected pattern on the transitional polymer (poly(N- Isopropylacrylamide); and releasing the biopolymer from the transitional polymer with the biopolymer' s structure and integrity intact. A biopolymer can be selected from a natural or synthetic extracellular matrix (ECM) protein, growth factor, lipid, fatty acid, steroid, sugar and other biologically active carbohydrates, a biologically derived homopolymer, nucleic acids, hormone, enzyme, pharmaceutical composition, cell surface ligand and receptor, cytoskeletal filament, motor protein, silks, polyprotein (e.g., poly(lysine)) or any combination thereof.

Biopolymers useful in the generation of the matrices and scaffolds for the embodiments directed to cellular therapies using soluble TRAIL polypeptides described herein include, but are not limited to, a) extracellular matrix proteins to direct cell adhesion and function (e.g., collagen, fibronectin, laminin, etc.); (b) growth factors to direct cell function specific to cell type (e.g., nerve growth factor, bone morphogenic proteins, vascular endothelial growth factor, etc.); (c) lipids, fatty acids and steroids (e.g., glycerides, non-glycerides, saturated and unsaturated fatty acids, cholesterol, corticosteroids, sex steroids, etc.);(d) sugars and other biologically active carbohydrates *(e.g.*, monosaccharides, oligosaccharides, sucrose, glucose, glycogen, etc.); (e) combinations of carbohydrates, lipids and/or proteins, such as proteoglycans (protein cores with attached side chains of chondroitin sulfate, dermatan sulfate, heparin, heparan sulfate, and/or keratan sulfate); glycoproteins *[e.g*., selectins, immunoglobulins, hormones such as human chorionic gonadotropin, Alpha-fetoprotein and Erythropoietin (EPO), etc.]; proteolipids *(e.g.*, N-myristoylated, palmitoylated and prenylated proteins); and glycolipids *(e.g.*, glycoglycerolipids, glycosphingolipids, glycophosphatidylinositols, etc.); (f) biologically derived homopolymers, such as polylactic and polyglycolic acids and poly-L-lysine; (g) nucleic acids *(e.g.,* DNA, RNA, etc.); (h) hormones *(e.g.,* anabolic steroids, sex hormones, insulin, angiotensin, etc.); (i) enzymes (types: oxidoreductases, transferases, hydrolases, lyases, isomerases, ligases; examples: trypsin, collegenases, matrix metallproteinases, etc.); (j) pharmaceuticals *(e.g*., beta blockers, vasodilators, vasoconstrictors, pain relievers, gene therapy, viral vectors, anti-inflammatories, etc.); (k) cell surface ligands and receptors *(e.g*., integrins, selectins, cadherins, etc.); (1) cytoskeletal filaments and/or motor proteins *(e.g.,* intermediate filaments, microtubules, actin filaments, dynein, kinesin, myosin, etc.), or any combination thereof. For example, a biopolymer can be selected from the group consisting of fibronectin, vitronectin, laminin, collagen, fibrinogen, silk or silk fibroin.

It is contemplated that in addition to the administration of cells in suspension via, e.g., intraarterial injection, in some embodiments of the methods described herein, cells can be engineered to express or secrete a soluble TRAIL polypeptide that are encapsulated in an extracellular matrix comprising a thiol-modified hyaluronic acid and a thiol-reactive cross-linker, such as, for example, polyethylene glycol diacrylate, can also be administered.

In one method of cell encapsulation, the isolated cells are mixed with sodium alginate and extruded into calcium chloride so as to form gel beads or droplets. The gel beads are incubated with a high molecular weight (e.g., MW 60-500 kDa) concentration (0.03-0.1% w/v) polyamino acid (e.g., poly-L-lysine) to form a membrane. The interior of the formed capsule is re-liquified using sodium citrate. This creates a single membrane around the cells that is highly permeable to relatively large molecules (MW .about.200-400 kDa), but retains the cells inside. The capsules are incubated in physiologically compatible carrier for several hours in order that the entrapped sodium alginate diffuses out and the capsules expand to an equilibrium state. The resulting alginate-depleted capsules is reacted with a low molecular weight polyamino acid which reduces the membrane permeability (MW cut-off 40-80 kDa).

Other exemplary materials suitable for use in matrices and scaffolds include, but are not limited to, PEG diacylate, hyaluronic acid, polylactic acid (PLA), poly-L-lactic acid (PLLA), poly-D-lactic acid (PDLA), polyglycolide, polyglycolic acid (PGA), polylactide-co-glycolide (PLGA), polydioxanone, polygluconate, polylactic acid-polyethylene oxide copolymers, modified cellulose, collagen, polyhydroxybutyrate, polyhydroxpriopionic acid, polyphosphoester, poly(alpha-hydroxy acid), polycaprolactone, polycarbonates, polyamides, polyanhydrides, polyamino acids, polyorthoesters, polyacetals, polycyanoacrylates, degradable urethanes, aliphatic polyester polyacrylates, polymethacrylate, acyl substituted cellulose acetates, non-degradable polyurethanes, polystyrenes, polyvinyl chloride, polyvinyl fluoride, polyvinyl imidazole, chlorosulphonated polyolifins, polyethylene oxide, polyvinyl alcohol, Teflon, nylon silicon, and shape memory materials, such as poly(styrene-block-butadiene), polynorbornene, hydrogels, metallic alloys, and oligo(-caprolactone)diol as switching segment/oligo(p-dioxyanone)diol as physical crosslink. Other suitable polymers can be obtained by reference to The Polymer Handbook, 3rd edition (Wiley, N.Y., 1989).

In some embodiments, the stem cells useful for the methods described herein can further be encapsulated in a synthetic extracellular matrix (sECM) or biodegradable hydrogel. Encapsulation of cells as the term is used herein refers to immobilization of cells within biocompatible, semipermeable membranes. Encapsulation of stem cells is described in Encapsulated Stem Cells for Cancer Therapy (Khalid Shah, Biomatter. Jan 1, 2013; 3(1): e24278). The encapsulation of cells allows the delivery of molecules of interest for a longer period of time as cells can release the molecules at the tumor site, especially if they hone to a tumor site as has been shown. Due to their ability to provide a physiologic environment that promotes cell survival and prevent immune response while permitting easy in vivo transplantation and cell retention, biodegradable hydrogels and synthetic extracellular matrix (sECM) to encapsulate stem cells have been utilized (Burdick et al. Adv Mater. 2011;23H41-56; Allison et al., Hyaluronan: a powerful tissue engineering tool. Tissue Eng. 2006;12:2131-40). A variety of biomaterials such as alginate, hyaluronic acid, agarose and other polymers have been used for encapsulation.

Hyaluronic acid (HA) is a non-sulfated, linear polysaccharide with the repeating disaccharide, 0-1,4-D-glucuronic acid-r3-1,3-N-acetyl-Dglucosamine. HA is ubiquitous and highly hydrated polyanion and an essential component of the extracellular matrix (ECM); its structural and biological properties mediate cellular signaling, wound repair, morphogenesis and matrix organization (Prestwich, J Control Release. 2011;155:193-9). Although HA and its derivatives have been clinically used in the past, it has become recognized as an important building block for the creation of new biomaterials for use in cell therapy (Prestwich, J Cell Biochem. 2007;101:1370-83; Prestwich , Acc Chem Res. 2008;41:139-48; Shu et al., Biomaterials. 2003;24:3825-34) . Chemical modification of HA alters its material and biological properties (Shu et al., Biomacromolecules. 2002;3:1304-11), and targets three functional groups: the glucuronic acid carboxylic acid, the primary and secondary hydroxyl groups, and the N-acetyl group (following deamidation). The chemical, mechanical, and biological criteria for clinical and preclinical biomaterials are design constraints that must be incorporated into the biomaterial design (Prestwich, J Cell Biochem. 2007;101:1370-83; Vanderhooft et al., Biomacromolecules. 2007;8:2883-9). HA-based synthetic extracellular matrices (sECMs) have been developed for use in drug evaluation and regenerative medicine (Vanderhooft et al., Macromol Biosci. 2009;9:20-8). These sECMs were based on modification of the carboxylate groups of glycosaminoglycans (GAGs) and proteins such as gelatin using hydrazides containing disulfides (Pan et al., J Neurosci Res. 2009;87:3207-20; Sayyar et al., J Tissue Eng. 2012;3:2041731412462018 ). More importantly, in vivo injectable cell suspensions in the sECM macromonomers can be crosslinked with cytocompatible bifunctional polyethylene glycol (PEG) derived crosslinkers (Park et al., Gene Ther. 2002;9:613-24). The mechanical properties and rates of biodegradation could be altered by several varying parameters (Teng et al., Proc Natl Acad Sci USA. 2002;99:3024-9): (1) molecular weight of starting HA employed; (2) percentage of thiol modification; (3) concentrations of thiolated HA and thiolated gelatin; (4) molecular weight of the crosslinker polyethylene glycol diacrylate (PEGDA); and (5) ratio of thiols to acrylates. Living hydrogels allow control of gel composition and mechanics, and permit incorporation of cells and a wide variety of small molecules, large molecules, nanoparticles, and microparticles (Shu et al., Biomacromolecules. 2002;3:1304-11).

In some embodiments, additional bioactive substances can be added to a biopolymer matrix or scaffold comprising the cells engineered to express soluble TRAIL polypeptides described herein. The amounts of such optionally added bioactive substances can vary widely with optimum levels being readily determined in a specific case by routine experimentation.

The soluble TRAIL polypeptides or cells engineered to express soluble TRAIL polypeptides described herein can be administered directly as a pharmaceutical composition to a subject in need thereof. As used herein, the terms "administering," and "introducing" are used interchangeably and refer to the placement of cells engineered to express soluble TRAIL polypeptides into a subject by a method or route which results in at least partial localization of such agents at a desired site, such as localization to a plurality of metastatic foci in the brain.

In some embodiments of the methods described herein, the cells engineered to express soluble TRAIL polypeptides are administered to a subject in need thereof by any mode of administration that delivers the agent systemically or to a desired surface or target, and can include, but is not limited to, injection, infusion, and instillation. "Injection" includes, without limitation, intravenous, intraarterial, intrathecal, intraventricular, subarachnoid, intraspinal, and intracerebro spinal injection and infusion. In some embodiments, the soluble TRAIL polypeptides or cells engineered to express soluble TRAIL polypeptides for use in the methods described herein are not administered by intravenous infusion or injection. In some embodiments, the soluble TRAIL polypeptides or cells engineered to express soluble TRAIL polypeptides for use in the methods described herein are administered by intraarterial administration.

The phrases "parenteral administration" and "administered parenterally" as used herein, refer to modes of administration other than enteral and topical administration, usually by injection. The phrases "systemic administration," "administered systemically", "peripheral administration" and "administered peripherally" as used herein refer to the administration of soluble TRAIL polypeptides or cells engineered to express soluble TRAIL polypeptides other than directly into a target site, tissue, or organ, such as a metastatic tumor site, such that it enters the subject's circulatory system.

For the clinical use of the methods described herein, administration of the soluble TRAIL polypeptides or cells engineered to express soluble TRAIL polypeptides can include formulation into pharmaceutical compositions or pharmaceutical formulations for parenteral administration, e.g., intraarterial or intravenous, or other mode of administration. In some embodiments of the methods, the soluble TRAIL polypeptides or cells engineered to express soluble TRAIL polypeptides described herein can be administered along with any pharmaceutically acceptable carrier compound, material, or composition which results in an effective treatment in the subject. Thus, a pharmaceutical formulation for use in the methods described herein can comprise a soluble TRAIL polypeptide or cells engineered to express soluble TRAIL polypeptides as described herein in combination with one or more pharmaceutically acceptable ingredients.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent, media, encapsulating material, manufacturing aid (e.g., lubricant, talc magnesium, calcium or zinc stearate, or steric acid), or solvent encapsulating material, involved in maintaining the stability, solubility, or activity of, a cell engineered to express soluble TRAIL polypeptide. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) excipients, such as cocoa butter and suppository waxes; (8) oils, such as peanut oil, cottonseed oil, safflower oil, sesame oil, olive oil, corn oil and soybean oil; (9) glycols, such as propylene glycol; (10) polyols, such as glycerin, sorbitol, mannitol and polyethylene glycol (PEG); (11) esters, such as ethyl oleate and ethyl laurate; (12) agar; (13) buffering agents, such as magnesium hydroxide and aluminum hydroxide; (14) alginic acid; (15) pyrogen-free water; (16) isotonic saline; (17) Ringer's solution; (19) pH buffered solutions; (20) polyesters, polycarbonates and/or polyanhydrides; (21) bulking agents, such as polypeptides and amino acids (22) serum components, such as serum albumin, HDL and LDL; (23) C2-C12 alcohols, such as ethanol; and (24) other non-toxic compatible substances employed in pharmaceutical formulations. Release agents, coating agents, preservatives, and antioxidants can also be present in the formulation. The terms such as "excipient", "carrier", "pharmaceutically acceptable carrier" or the like are used interchangeably herein.

Some further embodiments of the formulations and modes of direct administration of the soluble TRAIL polypeptides or cells engineered to express soluble TRAIL polypeptides that can be used in the methods described herein are illustrated below.

Since administration of parenteral dosage forms typically bypasses the patient's natural defenses against contaminants, parenteral dosage forms or carriers for engineered cells are preferably sterile or capable of being sterilized prior to administration to a patient.

Suitable vehicles that can be used to provide parenteral dosage forms as described herein are well known to those skilled in the art. Examples include, without limitation: sterile water; water for injection USP; saline solution; glucose solution; aqueous vehicles such as but not limited to, sodium chloride injection, Ringer's injection, dextrose injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

It is understood that the foregoing description and the following examples are illustrative only and are not to be taken as limitations upon the scope of the invention. Various changes and modifications to the disclosed embodiments, which will be apparent to those of skill in the art, may be made without departing from the scope of the present disclosure.

### EXAMPLES

Characterizing clinically relevant brain metastasis models and assessing the therapeutic efficacy in such models are fundamental for the development of novel therapies for metastatic brain cancers. As described herein, an *in vivo* imageable breast-to-brain metastasis mouse model has been developed. Using real time *in vivo* imaging and subsequent composite fluorescence imaging, a widespread distribution of micro- and macro-metastasis at different stages of metastatic progression is shown. It is also shown that extravasation of tumor cells and the close association of tumor cells with blood vessels in the brain mimics the multi-foci metastases observed in the clinics. The ability of engineered adult stem cells to track metastatic deposits in this model was explored, and it is shown that engineered stem cells either implanted or injected via circulation efficiently home to metastatic tumor deposits in the brain. It was tested whether TNF receptor superfamily member 10A/10B apoptosisinducing ligand (TRAIL) based pro apoptotic therapies that induce death receptor signaling within the metastatic tumor cells are favorable therapeutic approaches. Stem cells were engineered to express a tumor selective, potent and secretable variant of a TRAIL, S-TRAIL, and it is shown herein that these cells significantly suppressed metastatic tumor growth and prolonged the survival of mice bearing metastatic breast tumors. Furthermore, the incorporation of pro-drug converting enzyme, herpes simplex virus thymidine kinase, into therapeutic S-TRAIL secreting stem cells allowed their eradication post-tumor treatment. These studies are the first of their kind that provide insight into targeting brain metastasis with stem-cell mediated delivery of pro-apoptotic ligands and directly translate to clinical applications.

Metastatic brain tumors are the most commonly observed intracranial tumors, frequently occurring in patients with metastatic cancers, particularly from those of the lung, breast, and skin (melanoma) (Eichler et al., 2011). The incidence of brain metastasis has been estimated to be 35% of metastatic breast cancer patients (Lockman et al., 2010), which is often manifested with neurological symptoms and diagnosed with clinical imaging modalities (Steeg et al., 2011). Most patients have multiple metastatic lesions at the time of diagnosis, making surgery an inadequate therapeutic option on its own. Parallel therapeutic approaches include stereotactic radiosurgery, whole brain radiotherapy and chemotherapy (Kocher et al., 2011). However, the blood-brain barrier, which prevents the brain permeability of many systemic therapies, and the negative side effects of radiotherapy pose challenges for the success of existing therapies and cause failure to improve overall patient survival (Lockman et al., 2010). Therefore, there is a considerable need for the development of novel tumor-specific therapies that aim to overcome the challenges of delivery and specificity issues.

Given the multistep and complex biological nature of brain metastasis, tumor models that recapitulate metastatic brain tumors are limited. There have been several studies that established tumor lines with metastatic potential (Bos et al., 2010) and specifically a clonal tumor population with brainseeking properties has been described at a genomic level (Bos et al., 2009). A recent study reported a novel molecular mechanism for breast cancer cell infiltration into the brain by protection of cancer cells from death signals and allowing for efficient vascular co-option and colonization (Valiente et al., 2014). By expressing serpins, a family of protease inhibitors, metastatic tumor cells were able to adopt a unique defense mechanism and evade stroma-induced apoptosis. An approach that activates the dormant, cell surface-mediated apoptotic mechanisms on tumor cells can be a favorable therapeutic approach for brain-metastatic tumors.

Tumor necrosis factor (ligand) superfamily, member 10 TRAIL belongs to the TNF receptor ligand family, which also includes TNF and FASLG (Wiley et al., 1995; Pan al., 1997). It is well known that they are capable of inducing apoptosis in a number of cancer cells via binding to their cognate receptors and the initiation of death receptor mediated signaling (French and Tschopp, 1999). Among them, TRAIL is a promising candidate for cancer therapies due to its capability of specifically targeting tumor cells while sparing normal cells (Stuckey and Shah, 2013). Therefore, it is of great interest to evaluate the strategy of targeting death receptor mediated apoptosis in metastatic tumors. However, in the case of brain neoplasms and metastases, local delivery of sufficient TRAIL ligand may be challenged by the presence of the blood--brain barrier.

Among many other biological differences, one major difference between the primary and metastatic brain tumors is their localization and distribution in the parenchyma. Although primary brain tumors mostly arise at a single site and progress from there, metastases occur in several distinct spots giving rise to multiple metastatic foci of different sizes (Modha et al., 2005). With this being a major impediment for therapeutic success, tumor-seeking stem-cell-based therapies were evaluated for treating multiple metastatic tumor lesions in the brain. In this study, the nature of the metastatic breast cancer model was first assessed in detail, which was established through several rounds of brain selection of breast carcinoma cells (Bos et al., 2009). Using non-invasive bioluminescent and fluorescent imaging, the temporal and spatial distribution of the metastases in the brain were then assessed. The metastatropic properties of engineered NSCs and the therapeutic potential of TRAILsecreting engineered neural stem cells (NSC-S-TRAIL) in this model were further explored. The studies described herein establish that NSCs engineered to express metastatic tumor-specific biomolecules can target brain metastasis and deliver effective treatment.

### Materials and methods

### Cell lines

MDA231-Br2Ma cells were cultured in Dulbecco's modified Eagle medium (DMEM) supplemented with 10% fetal bovine serum (FBS) and 1% penicillin-streptomycin as described (Bos et al., 2009). Primary mouse NSCs were obtained from Stem Cell Technologies and cultured in NEUROCULT mouse neural stem cell basal medium (Stem Cell Technologies) supplemented with NEUROCULT proliferation supplements, 2 mg/ml of heparin (Sigma), 20 ng/ml of human EGF (R&D Systems), and 20 ng/ml of human FGF2 (PeproTech) and penicillin/streptomycin as described (Bagci-Onder et al., 2011). Immortalized mouse NSCs, generated from embryonic cerebellum, were grown in DMEM supplemented with 10% FBS, 5% normal horse serum, sodium bicarbonate and sodium pyruvate as described (Tang et al., 2003). Human NSCs and human MSCs were used and grown as previously described (Kim et al., 2006; Shah et al., 2008; Sasportas et al., 2009).

### Engineered viral vectors, viral packaging and transduction of cells

Lentiviral construct, Pico2-Fluc.mCherry, was packaged as described (Bagci-Onder et al., 2011). Pico2- Rluc.mCherry was constructed by replacing firefly luciferase (Flue) with Renilla luciferase (Rluc). MDA231-BrM2a cells were transduced at a multiplicity of infection (MOI) of 2 in medium containing protamine sulphate (10 ng/ml). Retroviral vectors, MIGRI-TRAIL, MIGRI-TK-GF1 (thymidine kinase-GFP Flue) or MIGRI-GF1 (GFP-Fluc) vectors are described elsewhere (Martinez-Quintanilla et al., 2013). NSCs and MSCs were transduced at multiplicity of infection of 5 using protamine sulphate. All cells were visualized by fluorescence microscopy for green fluorescent protein (GFP) or mCherry expression to confirm transduction. To prepare the conditioned medium, 200 000 mouse NSCs or NSC-S-TRAIL cells were seeded per well of a 6-well plate and cultured for 48 h before supernatant collection. ELISA was used to quantify secreted TRAIL in the conditioned medium from engineered NSCs as described previously (Bagci-Onder et al., 2013).

### Cell viability assays

The effect of secreted TRAIL on cell viability was measured using CELLTITER-GLO^{®} (Promega) 24 h post-treatment as described (Bagci-Onder et al., 2013). All experiments were performed in triplicates. In co-culture experiments, the Flue activity was used as an indicator of cell viability. Accordingly, MBr-FmC cells and mouse NSCs [NSC-GFP, NSC-S-TRAIL, NSC-TK (thymidine kinase), NSC-TR-TK (TRAIL-thymidine kinase)] were seeded at 1:1 ratio in 24-well (0.5 x 105/well; Costar). MBr-FmC cells were assessed for their viability using Flue activity 48 h after coculturing. In the other experiments, MBr-FmC were co-cultured with different ratios of engineered human NSCs or human MSCs and 48 h later Flue activity was measured to indicate the viability of tumor cells.

### Western blotting analysis

Following treatment with secreted TRAIL (24 h), MDA231-Br2Ma cells were lysed with NP40 buffer supplemented with protease (Roche) and phosphatase inhibitors (Sigma). For immunoblotting, 30 lag of harvested proteins from each lysate were resolved on 10% SDS-PAGE and immunoblotted with antibodies against caspase 8, caspase 9 (Cell Signaling), cleaved poly ADPribose polymerase (PARP) (Cell Signaling) or alpha tubulin (Sigma) and detected by chemiluminescence incubation with horseradish peroxidase-conjugated secondary antibodies.

### Creation of a brain metastasis mouse model

Athymic nude female mice (6-8 weeks of age, Charles River Laboratories) were anaesthetized with ketamine-xylazine and an incision was made to expose the right carotid artery. Using 8-0 suture, both common and internal carotid arteries were temporarily ligated and a catheter connected to a 1 ml syringe was inserted into the external carotid artery to inject tumor cells. Two hundred thousand cells suspended in 100 [11 phosphate-buffered saline (PBS) were slowly injected through the catheter. The external carotid artery was then permanently ligated under the dissecting scope (Olympus, SZX10) using fine surgical tools and blood circulation was restored by releasing both common and internal carotid blood flow. Mice were imaged for tumor cell presence a few days later and then periodically for tumor progression by bioluminescence. In a few experiments to assess tumor growth, tumor cells were injected directly into the brain parenchyma using stereotactic injection as described (Shah *et al.,* 2008). The spatial distribution of brain metastases was examined by fluorescence microscopy on the brains of mice sacrificed at different time periods and as described below.

### Assessment of therapeutic potential of neural stem cells in tumor-bearing mice

To test both the metastatic tumor tracking (metastatropic) ability and efficacy of modified NSCs, NSCs (either expressing GFP, secreted TRAIL or Flue) were injected into metastatic tumor-bearing mice by two different routes: (i) NSCs were stereotactically implanted into mice with established brain metastases into the brain parenchyma at a single site 80 [200 000/5 ul PBS; from bregma, anteroposterior: -2 mm, mediolateral: 1.5 mm ventral (from dura): 2 mm] (Shah *et al.,* 2008); or (ii) NSCs were injected via the left external carotid artery with microsurgical technique as described above. In both cases, 200 000 NSCs were injected and NSC distribution was assessed by immunofluorescence on brain sections obtained after injection, as described below. The efficacy of NSC application was assessed by measuring tumor growth using bioluminescence imaging. Mice were periodically sacrificed to examine histology, as described below

### Assessment ofNSC-TR-TK in mouse brains

Two hundred thousand NSC-TR-TK-GF1 cells were either intracranially or intracarotidly injected in nude mice. Seven days after intracranial injection, or 3 days after intracarotid injection, daily PBS or ganciclovir (75 mg/kg body weight) were administrated via intraperitoneal (i.p.) injection for 2 weeks. NSC fate was assessed by bioluminescence imaging at various time points. To assess the eradication of therapeutic NSC post-tumor treatment, MBr-RmC cells (50 000 cells/ mouse) were intracranially injected in nude mice and tumor-bearing mice were injected intratumorally with NSC-TR-TK-GF1 (50 000 cells/mouse) in the mouse brain. Daily PBS or ganciclovir were administrated via intraperitoneal injection after 4 days of NSC injection and the fate of NSCs was assessed by bioluminescence imaging at different time points.

### Histology

Mice were sacrificed and brains were dissected and processed for immunohistochemistry as described (Shah *et al.,* 2008). Ten-micrometer sections were assessed for mCherry and GFP expression representing tumor cells and NSCs, respectively. To assess the temporal and spatial distribution of tumor and stem cells, coronal sections were obtained along the anterior-posterior axis at 200 um intervals. The composite images of each section were generated by stitching different individual images using Adobe Photoshop tools. The number of metastatic lesions was defined by measuring the number of mCherry-positive foci using ImageJ tools (NIH). Accordingly, each composite image was subjected to ImageJ particle analysis and the number of mCherry-positive particles per section was plotted. Caspase 3 staining was performed as described (Sasportas et al., 2009) using cleaved caspase-3 antibody (Cell Signaling). The number of caspase 3-positive puncta per tumor foci area was counted in multiple sections using ImageJ measurement tools. The average of these measurements per multiple sections was plotted. Endothelial cell staining was performed using anti-CD31 (Abcam). Whole-section images were acquired using a x 2 objective with a Olympus dissecting scope (SZX10) and processed using Adobe Photoshop. Higher magnification images were acquired with Olympus Digital Imaging Software (CellSens). Detailed section analysis was performed using confocal microscopy (LSM Pascal, Zeiss). Image quantification for caspase staining was performed using ImageJ and Adobe Photoshop measure tools.

### Statistical analysis

Data were analyzed by Student t-test when comparing two groups and ANOVA when comparing more than two groups. Data were plotted as mean ± SEM and differences were considered significant at P < 0.05. Survival curves were compared using the log-rank test. Analyses were done using GraphPad Prism 5.01.

### Results

### Characterization of brain metastasis in vivo

To establish an in vivo breast-to-brain metastatic model that can recapitulate the steps of metastatic progression and be imaged non-invasively, we engineered MDA231-BrM2a cells, which were previously isolated by several rounds of brain colonization from breast cancer (Bos et al., 2009), to express Flue and mCherry using a bicistronic lentiviral vector (referred to as MBr-FmC; FIG. 7A). Assessment of tumor growth after intracranial implantation of MBr-FmC confirmed their survival in the brain microenvironment (FIG. 7B and FIG. 7C). To mimic a majority of the steps of metastatic colonization and blood vessel interactions, we injected MBr-FmC cells via the intracarotid artery in mice. Non-invasive bioluminescence imaging on tumor-bearing mice revealed exclusive brain metastases and the progression of tumor over 45 days, indicating tumor colonization throughout the brain (FIG. 1A and 1B). Assessment of mCherry fluorescence on coronal sections along the anterior-posterior axis 15, 25 and 45 days posttumor cell injection, revealed distinct tumor foci that were detectable as early as 15 days post-tumor cell injection (FIG. 1C). The tumor cells distributed along all representative sections, and the number and size of metastases increased over time throughout the same coordinates correlating with increased bioluminescence. Quantification of the histology revealed a widespread distribution of micro- and macrometastases in the initial stages of metastatic progression (Day 15). The size of these metastatic lesions markedly increased at Day 25 and reached a maximally tolerated size at Day 45 post-MBr-FmC inoculation (FIG. 1D). Larger nodules developed hemorrhagic lesions, and astrogliosis was observed in the periphery of the tumors (FIGS. 7D-7F). Furthermore, CD31 immunohistochemistry highlighting the brain vasculature revealed the full extravasation of tumor cells and the close association of tumor cells with blood vessels. Specifically, the tumor cells displayed distinct associations with vessels, such as vascular mimicry, neovascularization, subpial growth, vascular co-option (FIG. 1E), which constitute the hallmarks of brain metastasis. These results reveal that the intracarotid injection of breast to brain metastatic tumor cells result in the formation of a clinically-relevant mouse model that resembles the multi-foci metastases observed in the clinics.

### Stem cells migrate to metastatic tumor foci

We first tested two NSC lines for their ability to survive in vivo. A primary NSC line growing as neurospheres (referred to as primary NSCs) and an immortalized NSC line growing as monolayers were transduced to express GF1 and implanted intracranially. Bioluminescence imaging showed that there was a direct correlation between NSC number and the Flue bioluminescence imaging signal within the ranges tested (FIG. 8A and 8B). In vivo survival studies revealed that immortalized NSCs survived markedly better than primary NSCs (FIG. 8C). To test the migratory potential of NSC in metastatic brain tumors, we administered NSCs engineered to express GFP (NSC-GFP) intraparenchymally into metastatic brain tumor-bearing mice generated after intracarotid artery injection of MBr-FmC tumor cells (FIG. 2A). Intraparenchymal administration of NSCs resulted in detectable GFP-positive NSCs in the periphery of, or within the metastatic tumor foci throughout the brain (FIG. 2B and 2C). Histological examination of mCherry-labelled tumor cells and GFP-labelled NSCs in the brain sections revealed that NSCs were selectively located in the tumor-rich regions [FIG. 2B (i, iii, iv, v and vii)], but not in the tumor-free locations throughout the brain [FIG. 2B (ii and vi)]. Specifically, the migration of NSCs towards the metastatic deposits was observed for the parenchymal micro and macrometastases [FIG. 2C(i)], leptomeningeal metastases [FIG. 2C(ii)], perivascular metastases [FIG. 2C(iii)], as well as cerebellar metastases [FIG. 2C(iv)], attesting to the highly tumoritropic migration ability of NSC towards metastatic brain tumors. Furthermore, CD31 immunohistochemistry analysis showed that migrating NSCs were present in or around tumor foci but not randomly distributed in the brain parenchyma or along blood vessels on their own (FIG. 9). These results show that NSCs migrate toward metastatic foci in the brain, and this tumoritropic migration of NSC is unlikely guided by brain endothelium.

### NSCs secreting TRAIL home to metastatic foci and suppress brain metastasis growth in vivo

Based on the ability of NSCs to track metastastic deposits, NSCs were engineered to express a potent and secretable variant of tumor specific therapeutic agent, TRAIL (NSC-S-TRAIL). NSC-S-TRAIL secreted TRAIL (400 ng/ml) in the conditioned medium resulting in a significantly decreased MBr-FmC cell viability compared to the control NSC-GFP medium (FIG. 10A). Cocultures of MBr-FmC and NSC-S-TRAIL were also established and showed a time-dependent decrease in MBr-FmC cell viability upon NSC-S-TRAIL treatment (FIG. 3A). Western blot analysis on the lysates of MBr-FmC treated with NSC-S-TRAIL revealed significant changes in initiator and effector caspases (caspases 8 and 3) and cleaved PARP as compared to the control treatment (FIG. 3B). To verify the in vivo potential of the therapeutic NSCs on metastatic tumor growth, MBr-FmC cells and NSCs were admixed at 1: 1 ratio and implanted intraparenchymally. Bioluminescence imaging analysis showed a significant decrease in the MBr-FmC cell growth exposed to NSC-S-TRAIL, as opposed to the control NSC-GFP-exposed tumor cells that kept growing over time (FIG. 10B). Next, the therapeutic potential of stereotactically implanted NSC-S-TRAIL was tested in the brain metastasis model. A marked suppression of metastatic tumor growth was observed upon NSC-S-TRAIL treatment as compared to NSC-GFP treatment (FIG. 3C). The whole brain sections collected at Day 40 revealed the growth and distribution of mCherry labelled tumor cells surrounded and intercalated by GFP-labelled control NSC. In contrast, the NSC-S10 TRAIL treated tumors were less abundant, confirming the homing and therapeutic efficacy of NSC-S-TRAIL in the metastasis model (FIG. 3D and 3E). A significantly higher cleaved caspase 3 expression on the brain sections confirmed NSC-S-TRAIL-mediated tumor cell apoptosis (FIG. 3F). These results show that NSCsecreted secreted TRAIL induce caspase 3-mediated apoptosis in metastatic tumor cells both *in vitro* and *in vivo.*

Next, the survival and distribution of NSCs were tested in a clinically relevant strategy wherein we administered NSCs or NSC-S-TRAIL via circulation (by intracarotid artery injection on the contralateral site of tumor inoculation) in the breast-to-brain metastasis model. To image tumor cells and NSC simultaneously, lines MBr-RmC (MDA231-BrM2a expressing Rluc and mCherry) and NSC-GF1 (NSC expressing both Flue and GFP) were created. Dual Rluc and Flue bioluminescence imaging confirmed the presence of both metastatic tumors and NSCs in the brain (FIG. 4A and 4B). Furthermore, representative fluorescent images revealed that intracarotid injection of NSCs resulted in detectable GFP-positive NSCs in the periphery of, or within the metastatic tumor foci in the brain (FIG. 4C). Next, the therapeutic efficacy of NSC-S-TRAIL administered via circulation in the brain metastasis model was tested (FIG. 4D). Bioluminescence imaging showed a significant suppression of metastatic tumor growth in the brain of NSC-S-TRAIL treated group compared with NSC treated group (FIG. 4E). A significant survival benefit of the metastatic tumor-bearing mice was observed in the NSC-S-TRAIL-treated group compared to the NSC group or control group (FIG. 4F). Taken together, these results show that NSCs engineered to secrete TRAIL demonstrate efficacy in breast-to-brain metastasis by homing to metastatic deposits and inducing tumor-specific apoptosis in the brain.

### Elimination of therapeutic stem cells by HSV-TK

To translate NSC-S-TRAIL therapy for brain metastasis into clinics, it is critical to follow the long-term fate of NSCs post-tumor treatment and ultimately, selectively eradicate therapeutic NSCs to avoid continuous access of therapeutic agent to normal brain tissue and to circumvent any malignant transformation of NSCs. To address this, NSC-S-TRAIL expressing cells were engineered to further express herpes simplex virus thymidine kinase (HSV-TK) and GFP-Fluc (GF1) (NSC-TR-TK-GF1) and tested for efficacy of thymidine kinase suicide therapy in vitro. As revealed by both Flue bioluminescence imaging and GFP immunohistochemistry, administration of ganciclovir eliminated modified NSC in vitro (FIG. 11A). It was next confirmed that NSCs retain their capacity to reduce tumor cell viability, without themselves being killed (FIG. 11B). Further, the efficacy of ganciclovir to eradicate NSCs in non-tumor bearing brain was tested. As revealed by Flue bioluminescence imaging, NSCs were substantially eradicated by thymidine kinase suicide therapy, either implanted via the intracranial (FIG. 5A) or intracarotid route (FIG. 5B). Next, the eradication of therapeutic NSCs post-tumor treatment in vivo was tested. NSC-TR-TK-GF1 expressing cells were intracranially injected into MBr-RmC tumor-bearing brains. Flue bioluminescence imaging revealed the eradication of therapeutic NSC-TR-TK-GF1 after ganciclovir administration compared to control group treated with PBS (FIG. 5C). Fluorescent images of brain sections collected from tumor-bearing mice (before/after ganciclovir treatment) confirmed the elimination of MBr-RmC tumor cells by NSC-TR-TK-GF1 administration (FIG. 11C). These results reveal that therapeutic stem cells can be eradicated both in vitro and in vivo post-tumor treatment.

### Human NSCs and MSCs expressing secreted TRAIL and HSV-TK have anti-tumor effects

To make the therapeutic strategy more clinically relevant, human NSCs and human MSCs were used and the efficacy of the TRAIL/thymidine kinase system on metastatic tumor cells was tested. Human NSCs and MSCs were engineered to express secreted TRAIL (hNSC-TR/hMSC-TR) or co-express secreted TRAIL and HSV-TK (hNSC-TR-TK/hMSC-TR-TK), and established cocultures of MBr-FmC with different proportions of modified human NSCs and MSCs. Flue bioluminescence imaging data showed a dose-dependent decrease of MBr-FmC cell viability within the different proportions of therapeutic human NSCs and MSCs tested (FIGS. 6A and 6C). Furthermore, the administration of ganciclovir successfully eradicated hNSC-TR-TK/hMSC-TR-TK in vitro (FIGS. 6B and 6D). These results reveal that human MSCs and NSCs, like their mouse counterparts, have the same efficacy of killing breast-to-brain metastatic tumor cells and can be eliminated post ganciclovir treatment.

### Discussion

In this study, an imageable model of clinically-relevant breast-to-brain metastasis that displays various features of brain metastasis was first developed and extensively characterized. The ability of engineered NSCs to home to metastatic brain tumors in this model was also established. The data show that therapeutic NSC-S-TRAIL significantly suppresses metastatic tumor growth and prolongs the survival of mice bearing metastatic breast tumors in the brain. The experiments demonstrated that the incorporation of pro-drug converting enzyme, HSV-TK into therapeutic NSC-S-TRAIL allows the eradication of NSCs post-tumor treatment.

Based on case series from the 1960s and 1970s, the incidence of clinically evident brain metastasis among women with stage IV breast cancer is estimated to be 10-16%. These figures underestimate the true incidence, given that brain metastases are found in 30% of patients at autopsy (Lin et al., 2004). Although a number of previous studies have made significant advances in our understanding of the biology of brain metastasis, it is still of utmost importance to use the most suitable model that mimics the clinical settings. Most of these studies have used intracranial injection of tumor cells directly to the brain parenchyma (Eichler et al., 2011), which does not mimic the actual clinical settings. There are multiple steps to metastatic colonization of the brain, namely, initial arrest of tumor cells at the brain capillaries, extravasation, continuation of perivascular position, vessel co-option, micrometastatic growth, and macrometastatic growth. To mimic a majority of the steps of metastatic colonization and blood vessel interactions, an imageable model of breast-to-brain metastasis was created by injecting tumors cells via the intracarotid artery in mice. Histological examination of tumor cell-endothelial cell interactions on the mice brains inoculated with MBr cells, displayed all these features. As the wide localization of multiple lesions is a major impediment in the treatment of such metastasis, it is crucial to use models that display this feature to develop effective therapies.

One of the major causes of therapeutic failure in metastatic brain cancer is the insufficient delivery of drugs across the blood-brain barrier (Eichler et al., 2011). As most metastatic lesions in the brain bear non-leaky vasculature, the success of systemic therapies is only marginal. Therefore, on-site delivery of tumor-specific therapeutics to the metastatic lesions would offer great potential for these tumors.

The cell-of-origin of metastatic brain tumors resides in a distant organ, as opposed to being in the brain, as for primary brain tumors; thus, the biology of metastatic tumors is markedly different than that of primary brain tumors. To our knowledge, the work discussed herein is the first study of its kind to demonstrate a novel stem cell-based approach for established multifocal brain metastasis. It is demonstrated herein that intracarotid injection of tumor cells displays all the features of metastasis and that NSCs, either engrafted directly to brain or administered via circulation, have immense ability to track metastatic tumor lesions. Therefore, 'arming' NSCs with anti-tumor biomolecules offers a viable approach to eradicate these multifocal lesions.

Previous work showed that the infiltrating metastatic breast cancer cells that survived within the brain were shielded from death receptor-mediated apoptosis signaling (Valiente et al., 2014). Activation or reactivation of death receptor signaling provides a target for treating breast-to-brain metastasis. In the studies described herein, it was found that the expression level of TNFRSF10B (previously known as **DR5)** was extremely high in the metastatic MBr cells and TRAIL specifically killed the tumor cells instead of the normal cells. TRAIL can therefore be used as an anti-tumor biomolecule to induce metastatic cell apoptosis via death receptor-mediated signaling.
The data provided herein demonstrated that NSC-delivered secreted TRAIL induces apoptosis in the brain-metastatic breast cancer cell line MBr *in vitro* and *in vivo.* Additionally, it was shown that direct implantation of NSCs at a single region in the brain parenchyma was sufficient to eradicate many metastatic lesions spread throughout the brain in the described model. When administered through the blood flow, NSC-S-TRAIL extravasated at tumor-rich sites and eradicated tumor lesions. This also conferred a significant survival advantage to mice, further revealing the in vivo efficacy of NSC-delivered secreted TRAIL for treating metastasis in the brain. This approach could be further improved by engineering NSCs to be more permeable through the brain endothelium by introducing the markers identified for transendothelial migration, such as, ST6GALNAC5 (Bos et al., 2009). All in all, with either route of administration, NSC-S-TRAIL displayed significant efficacy in metastatic lesions. Administration via the circulation provided strong results against multifocal metastases and has the advantage of being less invasive than intracranial and/or intraparenchymal administration. The ability of NSCs administered outside the brain to effectively track or migrate from the circulation to metastatic tumor foci that are on the other side of the blood-brain barrier is remarkable.

To avoid possible de novo tumor formation and ensure safety of the use of stem cells, it would be best to remove residual stem cells after they have carried the therapeutics to the tumors. To eliminate therapeutic NSCs from the brain, NSCs were engineered with a suicide gene system where the administration of pro-drug ganciclovir was sufficient to remove the HSV-TK expressing NSCs from the brain after treatment. Tumor elimination and eradication of therapeutic cells are expected to result in a burden of dead cells in the tumor microenvironment. This might possibly activate gliosis and microglial activation contributing to the clearance of cellular debris, as reported previously for brain injury models (Kim et al., 2015). Although the long-term consequences of NSC-TK based therapies remain to be explored in detail, the studies described herein provide clear proof of principle for efficacy in a clinically relevant model of multifocal breast to brain metastasis.

### References

Bagci-Onder T, Agarwal A, Flusberg D, Wanningen S, Sorger P, Shah K. Real-time imaging of the dynamics of death receptors and therapeutics that overcome TRAIL resistance in tumors. Oncogene 70 2013; 32: 2818-27.

Bagci-Onder T, Wakimoto H, Anderegg M, Cameron C, Shah K. A dual PI3K/mTOR inhibitor, PI-103, cooperates with stem cell delivered TRAIL in experimental glioma models. Cancer Res 2011; 71: 154-63.75

Bos PD, Nguyen DX, Massague J. Modeling metastasis in the mouse. Cur Opin Pharmacol 2010; 10: 571-7.

Bos PD, Zhang XH, Nadal C, Shu W, Gomis RR, Nguyen DX, et al. Genes that mediate breast cancer metastasis to the brain. Nature 2009; 459: 1005-9.80

Corsten MF, Shah K. Therapeutic stem-cells for cancer treatment: hopes and hurdles in tactical warfare. Lancet Oncol 2008; 9: 376-84.

Eichler AF, Chung E, Kodack DP, Loeffler JS, Fukumura D, Jain RK. The biology of brain metastases-translation to new therapies. Nat Rev 2011; 8: 344-56.

French LE, Tschopp J. The TRAIL to selective tumor death. Nat Med 1999; 5: 146-7.

Kaneko Y, Tajiri N, Staples M, Reyes S, Lozano D, Sanberg PR, et al. Bone marrowderived stem cell therapy for metastatic brain cancers. Cell Transplant 2015; 24: 625-30.

Kauer TM, Figueiredo JL, Hingtgen S, Shah K. Encapsulated therapeutic stem cells implanted in the tumor resection cavity induce cell death in gliomas. Nat Neurosci 2012; 15: 197-204.

Kienast Y, von Baumgarten L, Fuhrmann M, Klinkert WE, Goldbrunner R, Herms J, et al. Real-time imaging reveals the single steps of brain metastasis formation. Nat Med 2010; 16: 11622.

Kim JY, Kim N, Yenari MA. Mechanisms and potential therapeutic applications of microglial activation after brain injury. CNS Neurosci Ther 2015; 21: 309-19.

Kim SK, Kim SU, Park IH, Bang JH, Aboody KS, Wang KC, et al. Human neural stem cells target experimental intracranial medulloblastoma and deliver a therapeutic gene leading to tumor regression. Clin Cancer Res 2006; 12: 5550-6.

Kocher M, Soffietti R, Abacioglu U, Villa S, Fauchon F, Baumert BG, et al. Adjuvant whole-brain radiotherapy versus observation after radiosurgery or surgical resection of one to three cerebral metastases: results of the EORTC 22952-26001 study. J Clin Oncol 2011; 29: 134-41.

Lin NU, Bellon JR, Winer EP. CNS metastases in breast cancer. J Clin Oncol 2004; 22: 3608-17.

Lockman PR, Mittapalli RK, Taskar KS, Rudraraju V, Gril B, Bohn KA, et al. Heterogeneous blood-tumor barrier permeability determines drug efficacy in experimental brain metastases of breast cancer. Clin Cancer Res 2010; 16: 5664-78.

Martinez-Quintanilla J, Bhere D, Heidari P, He D, Mahmood U, Shah K. Therapeutic efficacy and fate of bimodal engineered stem cells in malignant brain tumors. Stem Cells 2013; 31: 1706-14. Modha A, Shepard SR, Gutin PH. Surgery of brain metastases-is there still a place for it? J Neuro Oncol 2005; 75: 21-9.

Pan G, Ni J, Wei YF, Yu G, Gentz R, Dixit VM. An antagonist decoy receptor and a death domain-containing receptor for TRAIL. Science 10 1997; 277: 815-8.

Park KI, Ourednik J, Ourednik V, Taylor RM, Aboody KS, Auguste IU, et al. Global gene and cell replacement strategies via stem cells. Gene Ther 2002; 9: 613-24.

Sasportas LS, Kasmieh R, Wakimoto H, Hingtgen S, van de Water JA, Mohapatra G, et al. Assessment of therapeutic efficacy and fate of engineered human mesenchymal stem cells for cancer therapy. Proc Natl Acad Sci USA 2009; 106: 4822-7.

Schmidt NO, Przylecki W, Yang W, Ziu M, Teng Y, Kim SU, et al. Brain tumor tropism of transplanted human neural stem cells is induced by vascular endothelial growth factor. Neoplasia 2005; 7: 623-9.

Seol HJ, Jin J, Seong DH, Joo KM, Kang W, Yang H, et al. Genetically engineered human neural stem cells with rabbit carboxyl esterase can target brain metastasis from breast cancer. Cancer Lett 2011; 311: 152-9.

Shah K, Bureau E, Kim DE, Yang K, Tang Y, Weissleder R, et al. Glioma therapy and real-time imaging of neural precursor cell migration and tumor regression. Ann Neurol 2005; 57: 3441.

Shah K, Hingtgen S, Kasmieh R, Figueiredo JL, Garcia-Garcia E, Martinez-Serrano A, et al. Bimodal viral vectors and in vivo imaging reveal the fate of human neural stem cells in experimental glioma model. J Neurosci 2008; 28: 4406-13.

Shah K, Hsich G, Breakefield XO. Neural precursor cells and their role in neuro-oncology. Dev Neurosci 2004; 26: 118-30.

Steeg PS, Camphausen KA, Smith QR. Brain metastases as preventive and therapeutic targets. Nat Rev Cancer 2011; 11: 35 352-63.

Stuckey DW, Shah K. TRAIL on trial: preclinical advances in cancer therapy. Trends Mol Med 2013; 19: 685-94.

Stuckey DW, Shah K. Stem cell-based therapies for cancer treatment: separating hope from hype. Nat Rev Cancer 2014; 14: 40 683-91.

Tang Y, Shah K, Messerli SM, Snyder E, Breakefield X, Weissleder R. In vivo tracking of neural progenitor cell migration to glioblastomas. Hum Gene Ther 2003; 14: 1247-54.

Valiente M, Obenauf AC, Jin X, Chen Q, Zhang XH, Lee DJ, et al. Serpins promote cancer cell survival and vascular co-option in brain metastasis. Cell 2014; 156: 1002-16.

Wiezorek J, Holland P, Graves J. Death receptor agonists as a targeted therapy for cancer. Clin Cancer Res 2010; 16: 1701-8.

Wiley SR, Schooley K, Smolak PJ, Din WS, Huang CP, NichollJK, et al. Identification and characterization of a new member of the TNF family that induces apoptosis. Immunity 1995; 3: 673-82.

Yip S, Shah K. Stem-cell based therapies for brain tumors. Curr Opin Mol Ther 2008; 10: 334-42.

Zhao D, Najbauer J, Garcia E, Metz MZ, Gutova M, Glackin CA, et al. Neural stem cell tropism to glioma: critical role of tumor hypoxia. Mol Cancer Res 2008; 6: 1819-29.

### EXAMPLE 2

Metastatic brain tumors are the most commonly observed intracranial tumors ^{1-4.} Patients with advanced breast cancer have a high propensity to metastasize to the brain ^{5,6} with EGFR positive and triple-negative breast cancer (TNBC) subtypes showing the highest incidence of brain metastases ⁷. Most patients have multiple metastatic lesions at the time of diagnosis making surgery an inadequate therapeutic option on its own'. Furthermore, the tight blood brain barrier (BBB) preventing the brain permeability of systemic therapies and the non-leaky vasculature of most metastatic lesions in the brain pose challenges for the success of existing therapies and result in failure to improve overall patient survival'. Therefore to effectively treat multiple highly aggressive breast metastatic foci in the brain, there is an urgent need to develop tumor specific agents that simultaneously target aberrant signaling pathways in TNBC and utilize delivery vehicles which specifically seek metastatic foci in the brain.

EGFR is frequently overexpressed in TNBC ¹⁰ and involved in malignant tumor growth ₁₁. epithelial-mesenchymal transition (EMT) ¹² migration and invasion of tumor cells ¹³. EGFR amplification or EGFR activating mutations have been found in a significantly larger fraction of breast to brain metastatic tumor tissue compared with samples from primary tumors with good prognosis, bone relapse, or other distant metastases ¹⁴ suggesting that EGFR is a potential therapeutic target in brain metastatic breast cancer. Recently completed randomized phase II clinical trial utilizing EGFR monoclonal antibody, Erbitux combined with a chemotherapeutic agent, cisplatin was shown to have a significantly improved overall response rate and progression free survival rate in metastatic (liver, lung, bone and lymph nodes) TNBC patients ^{1s}. Although, this study offers promise for targeting breast tumors that metastasize to different organs, the insufficient delivery of drugs across BBB in addition to the non-leaky vasculature of metastatic lesions in the brain is a major challenge in treating metastatic breast tumors in the brain. In recent years, antibody-based anticancer therapies that involve smaller antibody fragments such as Fabs, ScFvs and camelid (e.g. llama) derived heavy chainonly antibodies, nanobodies (Nbs) have been emerging _{16,17}.

As demonstrated herein, we have engineered EGFR-specific nanobodies (ENb) and shown that they sterically hinder the binding of EGF to the receptor, thereby inhibiting EGFR and its downstream signaling ¹⁸. Based on the recent findings that metastatic breast cancer cells adopt a novel mechanism of evading death receptor (DR) mediated apoptosis to successfully colonize in the brain ¹⁹; we have engineered a bimodal protein ENb-TRAIL, consisting of cDNA fusions encoding bivalent ENb and a potent cytotoxic variant of tumor necrosis factor apoptosis inducing ligand (TRAIL) which binds DR4/5. ENb-TRAIL lead to tumor specific killing by suppressing tumor cell proliferation and subsequently induces caspase- mediated apoptosis in a broad spectrum of tumor ¹⁸. Furthermore, our published and preliminary data reveal that ENb-TRAIL can be expressed by neural stem cells and ENb-TRAIL as a single molecule, engages both EGFR and death receptors simultaneously and is more effective than the combination of ENb and soluble TRAIL.

In light of the central role that selective expression and over-expression of tumor specific cell surface receptors such as EGFR and DR4/5 play in tumor therapy, screening of TNBC that recapitulate the pathological characteristics of breast to brain metastasis for the expression of these receptors is important. In our recent studies, we chose established TNBC lines and primary brain seeking patient derived TNBC lines (BMET02 and BMET05: isolated from the brain metastatic sites of patients with advanced breast cancer) and screened them for the expression of EGFR and DR4/5 by Western blotting (FIG. 12A). Our data reveal that most of the TNBC lines overexpress EGFR and express varying levels of DR4 and DRS and BMET02 and BMET05 express EGFR and high levels of DR4/5. Furthermore, we show that brain seeking BMET05 can be readily engineered to express FmC and forms metastatic tumors in mouse brains (FIG. 12B). Immunohistochemistry on brain sections from tumor bearing mice revealed that BMET05 cells retain the expression of EGFR and DR4/5 in metastatic tumor foci in the brain (FIGS. 12C-12H). These new data are exciting as they provide validity to further develop tumor models from brain seeking patient derived TNBC lines and test EGFR and DR4/5 targeted therapies proposed in this application.

We have been prospectively collecting CNS metastases tissue samples. Genomic analysis on paraffin-embedded sample pairs of brain metastasis and primary tumors from patients revealed mutations in clinically relevant genes like EGFR and P53 in the brain metastases ²⁰. Our recently obtained data on the expression analysis of EGFR and DRS on paraffin-embedded sample pairs of brain metastasis and primary tumors revealed a significantly higher EGFR and DRS expression in brain metastatic sections as compared to their primary counterparts (FIG. 13).

In an ongoing study, we have evaluated the role of EGFR in ENb-TRAIL induction of apoptosis. We used EGFR specific monoclonal antibody Cetuximab to block ENb-TRAIL binding to EGFR. As both ENb and Cetuximab are known to target the extracellular domain III of EGFR ²¹⁻²³, therefore pre-treating cells with cetuximab should reduce the amount of EGFR exposed to ENb-TRAIL and hinder ENb-TRAIL binding to EGFR. Pre-treatment with Cetuximab significantly reduced ENb-TRAIL-induced apoptosis in ENb and TRAIL non-responsive brain tumor cells (LN229) (FIG. 14A). Furthermore, co-immunoprecipitation assays revealed that EGFR and DRS formed complex in the presence of ENb-TRAIL and Cetuximab pretreatment significantly reduced the amount of EGFR-ENb-TRAIL-DRS complex (FIG. 14B). These results reveal that ENb-binding to EGFR is critical for EGFR-ENb-TRAIL-DRS complex mediated induction of apoptosis.

### References

**1.** Eichler, A.F., et al. The biology of brain metastases-translation to new therapies. Nat Rev Clin Oncol 8, 344-356 (2011).
2. Kodack, D.P., Askoxylakis, V., Ferraro, G.B., Fukumura, D. & Jain, R.K. Emerging strategies for treating brain metastases from breast cancer. Cancer Cell 27, 163-175 (2015).
3. Lim, E. & Lin, N.U. Updates on the management of breast cancer brain metastases. Oncology (Williston Park) 28, 572-578 (2014).
4. Lin, X. & DeAngelis, L.M. Treatment of Brain Metastases. J Clin Oncol 33, 3475-3484 (2015).
5. Bai, B., Yuan, Z.Y., Liu, D.G., Teng, X.Y. & Wang, S.S. Clinical features and survival analysis of different subtypes of patients with breast cancer brain metastases. Chinese journal of cancer 29, 413-419 (2010).
6. Nam, B.H., et al. Breast cancer subtypes and survival in patients with brain metastases. Breast cancer research : BCR 10, R20 (2008).
7. Preusser, M., Berghoff, A.S., Schadendorf, D., Lin, N.U. & Stupp, R. Brain metastasis: opportunity for drug development? Current opinion in neurology 25, 786-794 (2012).
8. Kocher, M., et al. Adjuvant whole-brain radiotherapy versus observation after radiosurgery or surgical resection of one to three cerebral metastases: results of the EORTC 22952-26001 study. J Clin Oncol 29, 134-141 (2011).
9. Lockman, P.R., et al. Heterogeneous blood-tumor barrier permeability determines drug efficacy in experimental brain metastases of breast cancer. Clin Cancer Res 16, 5664-5678 (2010).
10. Masuda, H., et al. Role of epidermal growth factor receptor in breast cancer. Breast cancer research and treatment 136, 331-345 (2012).
11. Zhang, X.T., et al. A positive feedback loop of ER-alpha36/EGFR promotes malignant growth of ER-negative breast cancer cells. Oncogene 30, 770-780 (2011).
12. Thiery, J.P. Epithelial-mesenchymal transitions in tumor progression. Nat Rev Cancer 2, 442-454 (2002).
13. Zhang, D., et al. Epidermal growth factor receptor tyrosine kinase inhibitor reverses mesenchymal to epithelial phenotype and inhibits metastasis in inflammatory breast cancer. Clin Cancer Res 15, 6639-6648 (2009).
14. Hohensee, I., et al. Frequent genetic alterations in EGFR- and HER2-driven pathways in breast cancer brain metastases. The American journal of pathology 183, 83-95 (2013).
15. Baselga, J., et al. Randomized phase II study of the anti-epidermal growth factor receptor monoclonal antibody cetuximab with cisplatin versus cisplatin alone in patients with metastatic triple-negative breast cancer. J Clin Oncol 31, 2586-2592 (2013).
16. Maussang, D., et al. Llama-derived single variable domains (nanobodies) directed against chemokine receptor CXCR7 reduce head and neck cancer cell growth in vivo. J Biol Chem 288, 29562-29572 (2013).
17. Oliveira, S., Heukers, R., Somkom, J., Kok, R.J. & van Bergen En Henegouwen, P.M. Targeting tumors with nanobodies for cancer imaging and therapy. J Control Release 172, 607-617 (2013).
18. van de Water, J.A., et al. Therapeutic stem cells expressing variants of EGFR-specific nanobodies have antitumor effects. Proc Natl Acad Sci U S A 109, 16642-16647 (2012).
19. Valiente, M., et al. Serpins promote cancer cell survival and vascular co-option in brain metastasis. Cell 156, 1002-1016 (2014).
20. Brastianos, P.K., et al. Genomic Characterization of Brain Metastases Reveals Branched Evolution and Potential Therapeutic Targets. Cancer discovery (2015).
21. Li, S., et al. Structural basis for inhibition of the epidermal growth factor receptor by cetuximab. Cancer Cell 7, 301-311 (2005).
22. Roovers, R.C., et al. A biparatopic anti-EGFR nanobody efficiently inhibits solid tumor growth. Int J Cancer 129, 2013-2024 (2011).
23. Schmitz, K.R., Bagchi, A., Roovers, R.C., van Bergen en Henegouwen, P.M. & Ferguson, K.M. Structural evaluation of EGFR inhibition mechanisms for nanobodiesNHH domains. Structure 21, 1214-1224 (2013).

## Claims

1. Stem cells engineered to express a binding agent specific for a tumor-associated cell-surface protein for use in the treatment of multifocal metastases in the brain, wherein the stem cells comprise mesenchymal stem cells (MSCs) or neuronal stem cells (NSCs) and the binding agent comprises a soluble TRAIL (sTRAIL) polypeptide , and wherein the treatment comprises administering the engineered stem cells to an individual in need thereof, via intracarotid artery injection, whereby the growth and/or survival of multifocal metastases in the brain is/are inhibited.

2. The stem cells for the use of claim 1, wherein the tumor-associated cell-surface protein is death receptor 4 (DR4) or death receptor 5 (DR5)

3. The stem cells for the use of claim 1, wherein the sTRAIL polypeptide comprises a fusion with a second tumor-associated cell-surface protein binding agent.

4. The stem cells for the use of claim 3, wherein the second tumor-associated cell-surface protein is a growth factor receptor.

5. The stem cells for the use of claim 4, wherein the growth factor receptor is selected from EGFR, IGF-R and NGF-R.

6. The stem cells for the use of claim 3, wherein the second tumor-associated cell surface marker binding agent is an antibody or antigen-binding fragment thereof, or a nanobody.

7. The stem cells for the use of claim 1, wherein the neuronal stem cells are derived from induced pluripotent stem (iPS) cells.

8. The stem cells for the use any one of claims 1-7, wherein the stem cells are further engineered to express a HSV thymidine kinase.

9. The stem cells for the use of claim 8, wherein the treatment further comprises, after treatment for multifocal brain metastases has been effected, administering gancyclovir, thereby eradicating the stem cells.

## Patentansprüche

1. Gentechnisch veränderte Stammzellen, um ein für ein tumorassoziiertes Zelloberflächenprotein spezifisches Bindungsmittel zu exprimieren, zur Verwendung bei der Behandlung von multifokalen Metastasen im Gehirn, wobei die Stammzellen mesenchymale Stammzellen (MSCs) oder neuronale Stammzellen (NSCs) umfassen und die Bindungsmittel ein lösliches TRAIL-Polypeptid (sTRAIL-Polypeptid) umfassen, und wobei die Behandlung das Verabreichen der gentechnisch veränderten Stammzellen an ein Individuum, das diese benötigt, über intrakarotide Arterieninjektion umfasst, wodurch das Wachstum und/oder Überleben von multifokalen Metastasen im Gehirn gehemmt wird/werden.

2. Stammzellen zur Verwendung nach Anspruch 1, wobei das tumorassoziierte Zelloberflächenprotein der Todesrezeptor 4 (DR4) oder Todesrezeptor 5 (DR5) ist.

3. Stammzellen zur Verwendung nach Anspruch 1, wobei das sTRAIL-Polypeptid eine Fusion mit einem zweiten tumorassoziierten Zelloberflächenprotein-Bindungsmittel umfasst.

4. Stammzellen zur Verwendung nach Anspruch 3, wobei das zweite tumorassoziierte Zelloberflächenprotein ein Wachstumsfaktorrezeptor ist.

5. Stammzellen zur Verwendung nach Anspruch 4, wobei der Wachstumsfaktorrezeptor ausgewählt ist aus EGFR, IGF-R und NGF-R.

6. Stammzellen zur Verwendung nach Anspruch 3, wobei das zweite tumorassoziierte Zelloberflächenmarker-Bindungsmittel ein Antikörper oder ein Antigenbindungsfragment davon oder ein Nanobody ist.

7. Stammzellen zur Verwendung nach Anspruch 1, wobei die neuronalen Stammzellen von induzierten pluripotenten Stammzellen (iPS) stammen.

8. Stammzellen zur Verwendung nach einem der Ansprüche 1 bis 7, wobei die Stammzellen weiter manipuliert sind, um eine HSV-Thymidinkinase zu exprimieren.

9. Stammzellen zur Verwendung nach Anspruch 8, wobei die Behandlung, nachdem eine Behandlung für multifokale Hirnmetastasen durchgeführt wurde, weiter das Verabreichen von Gancyclovir umfasst, wodurch die Stammzellen beseitigt werden.

## Revendications

1. Cellules souches modifiées pour exprimer un agent de liaison spécifique d'une protéine de surface cellulaire associée à une tumeur, destinées à être utilisées dans le traitement de métastases multifocales dans le cerveau, dans lesquelles les cellules souches comprennent des cellules souches mésenchymateuses (CSM) ou des cellules souches neuronales (CSN) et l'agent de liaison comprend un polypeptide TRAIL soluble (sTRAIL), et dans lesquelles le traitement comprend l'administration des cellules souches modifiées à un individu qui en a besoin, par injection dans l'artère intracarotidienne, selon lesquelles la croissance et/ou la survie des métastases multifocales dans le cerveau est/sont inhibées.

2. Cellules souches pour l'utilisation selon la revendication 1, dans lesquelles la protéine de surface cellulaire associée à la tumeur est le récepteur de mort 4 (DR4) ou le récepteur de mort 5 (DR5).

3. Cellules souches pour l'utilisation selon la revendication 1, dans lesquelles le polypeptide sTRAIL comprend une fusion avec un second agent de liaison à une protéine de surface cellulaire associée à une tumeur.

4. Cellules souches pour l'utilisation selon la revendication 3, dans lesquelles la seconde protéine de surface cellulaire associée à la tumeur est un récepteur de facteur de croissance.

5. Cellules souches pour l'utilisation selon la revendication 4, dans lesquelles le récepteur de facteur de croissance est choisi parmi EGFR, IGF-R et NGF-R.

6. Cellules souches pour l'utilisation selon la revendication 3, dans lesquelles le second agent de liaison du marqueur de surface cellulaire associé à la tumeur est un anticorps ou un fragment de liaison à l'antigène de celui-ci, ou un nanocorps.

7. Cellules souches pour l'utilisation selon la revendication 1, dans lesquelles les cellules souches neuronales sont dérivées de cellules souches pluripotentes induites (iPS).

8. Cellules souches pour l'utilisation selon l'une quelconque des revendications 1-7, dans lesquelles les cellules souches sont en outre modifiées pour exprimer une thymidine kinase de HSV.

9. Cellules souches pour l'utilisation selon la revendication 8, dans lesquelles le traitement comprend en outre, après que le traitement des métastases cérébrales multifocales a été effectué, l'administration de gancyclovir, éradiquant ainsi les cellules souches.
